(19) Europäisches Patentamt
European Patent Office
Office européen des brevets

(11) EP 4 506 345 A1

(12) **EUROPEAN PATENT APPLICATION**
published in accordance with Art. 153(4) EPC

(43) Date of publication:
**12.02.2025 Bulletin 2025/07**

(21) Application number: **23784296.8**

(22) Date of filing: **06.04.2023**

(51) International Patent Classification (IPC):
***C07D 409/10*** (2006.01) ***C07D 409/14*** (2006.01)
***C07D 401/14*** (2006.01) ***A61K 31/4439*** (2006.01)
***A61K 31/506*** (2006.01) ***A61P 9/00*** (2006.01)
***A61P 11/00*** (2006.01) ***A61P 13/12*** (2006.01)

(52) Cooperative Patent Classification (CPC):
**A61K 31/4178; A61K 31/4439; A61K 31/506; A61P 1/00; A61P 1/04; A61P 1/08; A61P 1/10; A61P 1/12; A61P 1/14; A61P 1/16; A61P 3/04; A61P 3/10; A61P 9/00; A61P 9/04; A61P 9/10;**
(Cont.)

(86) International application number:
**PCT/CN2023/086425**

(87) International publication number:
**WO 2023/193733 (12.10.2023 Gazette 2023/41)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **06.04.2022 CN 202210359646**
**22.06.2022 CN 202210715417**
**19.10.2022 CN 202211281172**
**29.03.2023 CN 202310325262**

(71) Applicant: **Wuhan Humanwell Innovative Drug Research and Development Center Limited Company**
**Wuhan, Hubei 430075 (CN)**

(72) Inventors:
- **ZHANG, Xuejun**
**Wuhan, Hubei 430075 (CN)**
- **ZANG, Yang**
**Wuhan, Hubei 430075 (CN)**
- **DING, Xiaohua**
**Wuhan, Hubei 430075 (CN)**
- **GAO, Zhenxing**
**Wuhan, Hubei 430075 (CN)**
- **QIAN, Lina**
**Wuhan, Hubei 430075 (CN)**
- **LI, Qun**
**Wuhan, Hubei 430075 (CN)**
- **YANG, Hui**
**Wuhan, Hubei 430075 (CN)**
- **LI, Lie**
**Wuhan, Hubei 430075 (CN)**
- **YANG, Jun**
**Wuhan, Hubei 430075 (CN)**

(74) Representative: **Dehns Germany Partnerschaft mbB**
**Theresienstraße 6-8**
**80333 München (DE)**

(54) **AT2R AGONIST**

(57) The present disclosure relates to the field of medicine. Specifically, provided in the present disclosure are a compound represented by formula I, and a tautomer, a stereoisomer, a hydrate, a solvate, and a pharmaceutically acceptable salt or a prodrug thereof. The compound can be used as an AT2R agonist,



Processed by Luminess, 75001 PARIS (FR)

(Cont. next page)

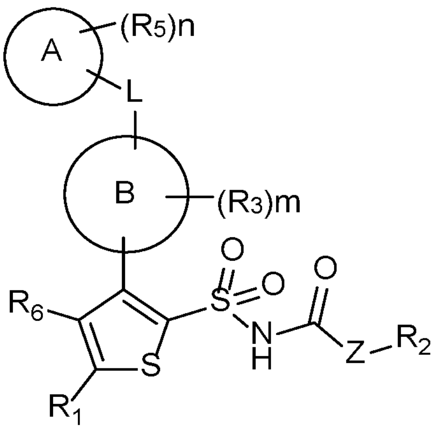

(I).

(52) Cooperative Patent Classification (CPC): (Cont.)
**A61P 9/12; A61P 11/00; A61P 11/06; A61P 13/08; A61P 13/12; A61P 15/00; A61P 17/06; A61P 25/28; A61P 27/02; A61P 29/00; A61P 35/00; A61P 37/08; C07D 401/14; C07D 409/10; C07D 409/14**

## Description

### PRIORITY APPLICATION INFORMATION

The present disclosure claims priority and benefits of Chinese Patent Application No. 202210359646.3 filed with the China National Intellectual Property Administration on April 6, 2022, Chinese Patent Application No. 202210715417.0 filed with the China National Intellectual Property Administration on June 22, 2022, Chinese Patent Application No. 202211281172.1 filed with the China National Intellectual Property Administration on October 19, 2022, and Chinese Patent Application No. 202310325262.4 filed with the China National Intellectual Property Administration on March 29, 2023. The disclosures of the aforementioned applications are hereby incorporated by reference in their entireties.

### FIELD

The present disclosure relates to the field of medicine, and more particularly, to an angiotensin II (Ang II) type 2 receptor (AT2R) agonist.

### BACKGROUND

Idiopathic pulmonary fibrosis (IPF) refers to an abnormal repair of damaged alveolar epithelial cells, which causes the proliferation of pulmonary fibroblasts and their transformation into myofibroblasts, excessive secretion of extracellular matrix, collagen deposition, changes in alveolar structure, and ultimately the formation of fibrosis. The pathogenesis of IPF has not yet been fully elucidated. Based on current studies, it is believed that IPF is closely related to oxidative stress, inflammatory response, and a regulation of renin-angiotensin-aldosterone system (RAAS) by body fluids. Currently, it is believed that the RAAS system plays an important role in the process of pulmonary fibrosis. Angiotensin converting enzyme (ACE) can hydrolyze angiotensin I (Ang I) into angiotensin II (Ang II), which plays an important role in the occurrence and development of various inflammations.

In humans, two major types of Ang II receptors have been identified, i.e., Ang II type 1 receptors (AT1R) and Ang II type 2 receptors (AT2R). Ang II has exhibited functions of regulating blood pressure, and fluid and electrolyte homeostasis in many organs, including kidney, adrenal gland, heart, blood vessel, brain, gastrointestinal tract, and reproductive organ. The effects of Ang II are regulated by the balance of expression of two G protein-coupled receptors (GPCRs), AT1R and AT2R. AT1R is expressed throughout the life cycle and is primarily responsible for regulating blood pressure. The blockers of AT1R are widely used as antihypertensive drugs in clinical practice. The most of the physiological effects of Ang II are controlled by AT1R. AT2R is mainly expressed in embryonic tissues and is related to blood pressure regulation, nerve growth, pain control, and myocardial regeneration. Drugs targeting AT2R can improve cardiovascular function, relieve neuropathic pain, etc. However, AT2R expression is significantly increased in pathological conditions, such as vascular injury, wound healing, and heart failure.

Several studies in adult subjects appear to confirm the fact that AT2R activation has an effect opposite to that of AT1R modulation in the regulation of responses following Ang II stimulation.

It has been verified that AT2R involves in the inhibition of apoptosis and cell proliferation. Recently, it has been suggested that AT2R agonists may be useful for the treatment and/or prevention of digestive tract diseases, for example, dyspepsia and irritable bowel syndrome, and multi-organ failure (see International Patent Application WO 99/43339).

There remains a need for effective and/or selective AT2R agonists that is expected to be used in useful in the above-mentioned diseases.

### SUMMARY

The present disclosure is intended to solve one of the technical problems in the related art at least to some extent. To this end, an object of the present disclosure is to provide a heterocyclic compound as an AT2R agonist and use thereof. The heterocyclic compound has a structure as shown in a first aspect of the present disclosure, and the heterocyclic compound may be used to prepare a medicament, pharmaceutical composition, or formulation for treating and/or preventing a disease or condition associated with AT2R; or to treat and/or prevent a disease or condition associated with AT2R.

The first aspect of the present disclosure provides a compound represented by Formula I, or a tautomer, stereoisomer, hydrate, solvate, pharmaceutically acceptable salt, or prodrug thereof:

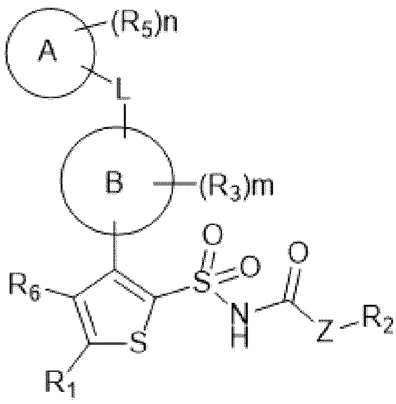

Formula I,

wherein:

ring B is selected from a benzene ring or a 5- to 6-membered heteroaromatic ring, wherein:

when ring B is the benzene ring, when $R_6$ is hydrogen, and when m is selected from 1, 2, 3, or 4, L is unsubstituted $C_1$ to $C_6$ alkyl or $C_1$ to $C_6$ alkyl substituted with one or more substituents, each of the one or more substituents being independently selected from halogen, $C_1$ to $C_6$ alkyl, halogenated $C_1$ to $C_6$ alkyl, or oxo (=O), and when a plurality of substituents are present, the plurality of substituents are the same or different; or

when ring B is the benzene ring, when $R_6$ is hydrogen, and when m is selected from 0, L is $C_1$ to $C_6$ alkyl substituted with one or more substituents, each of the one or more substituents being independently selected from halogen, $C_1$ to $C_6$ alkyl, halogenated $C_1$ to $C_6$ alkyl, or oxo (=O), and when a plurality of substituents are present, the plurality of substituents are the same or different; or

when ring B is the benzene ring, and when $R_6$ is selected from halogen, hydroxyl, cyano, amino, $C_1$ to $C_6$ alkyl, $C_3$ to $C_7$ cycloalkyl, $C_1$ to $C_6$ alkoxy, or -O-$C_3$-$C_7$ cycloalkyl, m is selected from 0, 1, 2, 3, or 4, and L is unsubstituted $C_1$ to $C_6$ alkyl or $C_1$ to $C_6$ alkyl substituted with one or more substituents, each of the one or more substituents being independently selected from halogen, $C_1$ to $C_6$ alkyl, halogenated $C_1$ to $C_6$ alkyl, or oxo (=O), wherein $R_6$ is independently substituted with 0, 1, 2, 3, or 4 substituents selected from halogen, hydroxyl, amino, cyano, $C_1$ to $C_6$ alkyl, or $C_3$ to $C_7$ cycloalkyl, when a plurality of substituents are present, the plurality of substituents are the same or different; or

when ring B is the 5- to 6-membered heteroaromatic ring, m is selected from 0, 1, 2, 3, or 4, L is unsubstituted $C_1$ to $C_6$ alkyl or $C_1$ to $C_6$ alkyl substituted with one or more substituents, each of the one or more substituents being independently selected from halogen, $C_1$ to $C_6$ alkyl, halogenated $C_1$ to $C_6$ alkyl, or oxo (=O), and $R_6$ is hydrogen or selected from halogen, hydroxyl, cyano, amino, $C_1$ to $C_6$ alkyl, $C_3$ to $C_7$ cycloalkyl, $C_1$ to $C_6$ alkoxy, or -O-$C_3$-$C_7$ cycloalkyl, wherein $R_6$ is independently substituted with 0, 1, 2, 3, or 4 substituents selected from halogen, hydroxyl, amino, cyano, $C_1$ to $C_6$ alkyl, or $C_3$ to $C_7$ cycloalkyl, when a plurality of substituents are present, the plurality of substituents are the same or different;

$R_1$ and $R_3$ are each independently selected from hydroxyl, cyano, amino, oxo (=O), $C_1$ to $C_6$ alkyl, $C_3$ to $C_7$ cycloalkyl, $C_1$ to $C_6$ alkoxy, or -O-$C_3$-$C_7$ cycloalkyl;

$R_1$ and $R_3$ are each independently substituted with 0, 1, 2, 3, or 4 substituents selected from halogen, hydroxyl, amino, cyano, $C_1$ to $C_6$ alkyl, or $C_3$ to $C_7$ cycloalkyl, when a plurality of substituents are present, the plurality of substituents are the same or different;

$R_2$ is selected from $C_1$ to $C_6$ alkyl, halogenated $C_1$ to $C_6$ alkyl, $C_3$ to $C_7$ cycloalkyl, $C_1$ to $C_6$ alkoxy, or $C_3$ to $C_7$ cycloalkyl-$C_1$ to $C_6$ alkyl;

$R_2$ is substituted with 0, 1, 2, 3, or 4 substituents selected from halogen, hydroxyl, amino, cyano, $C_1$ to $C_6$ alkyl, $C_3$ to $C_7$ cycloalkyl, 5- to 6-membered aryl, or 5- to 6-membered heteroaryl, when a plurality of substituents are present, the plurality of substituents are the same or different;

Z is selected from -O- or -$NR_4$-;

$R_4$ is $C_1$ to $C_3$ alkyl or H;

ring A is a 5- to 6-membered heteroaryl;

n is selected from 1, 2, 3, 4, or 5, when a plurality of substituents $R_5$ are present, the plurality of substituents $R_5$ are the same or different; and

$R_5$ is selected from H, halogen, -CN, $C_1$ to $C_6$ alkyl, $C_1$ to $C_6$ alkoxy, $C_3$ to $C_7$ cycloalkyl, or -O-$C_3$-$C_7$ cycloalkyl, when $R_5$ is $C_1$ to $C_6$ alkyl, $C_1$ to $C_6$ alkoxy, $C_3$ to $C_7$ cycloalkyl, or -O-$C_3$-$C_7$ cycloalkyl, the $C_1$ to $C_6$ alkyl, $C_1$ to $C_6$ alkoxy, $C_3$ to $C_7$ cycloalkyl, or -O-$C_3$-$C_7$ cycloalkyl is independently substituted with 0, 1, 2, 3, or 4 substituents selected from halogen, hydroxyl, $C_1$ to $C_6$ alkyl, or $C_3$ to $C_7$ cycloalkyl, and when a plurality of substituents are present, the plurality of substituents are the same or different.

In a preferred embodiment of the present disclosure, the compound has the following structure:

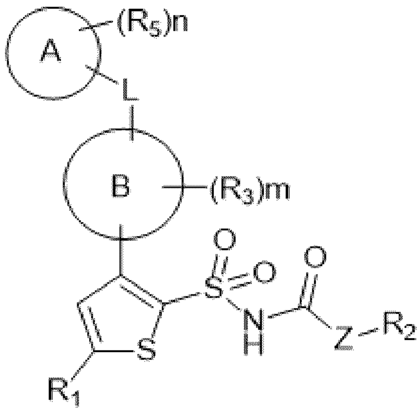

II,

wherein:

L is unsubstituted $C_1$ to $C_6$ alkyl or $C_1$ to $C_6$ alkyl substituted with one or more substituents, each of the one or more substituents being independently selected from halogen, $C_1$ to $C_6$ alkyl, halogenated $C_1$ to $C_6$ alkyl, or oxo (=O);
ring B is selected from a benzene ring or a 6 membered heteroaromatic ring, when ring B is the benzene ring, m is selected from 1, 2, 3, or 4, and when ring B is the 6 membered heteroaromatic ring, m is selected from 0, 1, 2, 3, or 4;
$R_1$ and $R_3$ are each independently selected from hydroxyl, cyano, amino, oxo (=O), $C_1$ to $C_6$ alkyl, $C_3$ to $C_7$ cycloalkyl, $C_1$ to $C_6$ alkoxy, or -O-$C_3$-$C_7$ cycloalkyl, and $R_1$ and $R_3$ are each independently substituted with 0, 1, 2, 3, or 4 substituents selected from halogen, hydroxyl, amino, cyano, $C_1$ to $C_6$ alkyl, or $C_3$ to $C_7$ cycloalkyl, when a plurality of substituents are present, the plurality of substituents are the same or different;
$R_2$ is selected from $C_1$ to $C_6$ alkyl, halogenated $C_1$ to $C_6$ alkyl, $C_3$ to $C_7$ cycloalkyl, $C_1$ to $C_6$ alkoxy, or $C_3$ to $C_7$ cycloalkyl-$C_1$ to $C_6$ alkyl;
$R_2$ is substituted with 0, 1, 2, 3, or 4 substituents selected from halogen, hydroxyl, amino, cyano, $C_1$ to $C_6$ alkyl, $C_3$ to $C_7$ cycloalkyl, 5- to 6-membered aryl, or 5- to 6-membered heteroaryl, when a plurality of substituents are present, the plurality of substituents are the same or different;
Z is selected from -O- or -$NR_4$-;
$R_4$ is $C_1$ to $C_3$ alkyl or H;
ring A is 5- to 6-membered heteroaryl;
n is selected from 1, 2, 3, 4, or 5, when a plurality of substituents $R_5$ are present, the plurality of substituents $R_5$ are the same or different; and
$R_5$ is selected from H, halogen, -CN, $C_1$ to $C_6$ alkyl, $C_1$ to $C_6$ alkoxy, $C_3$ to $C_7$ cycloalkyl, or -O-$C_3$-$C_7$ cycloalky, when $R_5$ is $C_1$ to $C_6$ alkyl, $C_1$ to $C_6$ alkoxy, $C_3$ to $C_7$ cycloalkyl, or -O-$C_3$-$C_7$ cycloalkyl, the $C_1$ to $C_6$ alkyl, $C_1$ to $C_6$ alkoxy, $C_3$ to $C_7$ cycloalkyl, or -O-$C_3$-$C_7$ cycloalkyl is independently substituted with 0, 1, 2, 3, or 4 substituents selected from halogen, hydroxyl, $C_1$ to $C_6$ alkyl, or $C_3$ to $C_7$ cycloalkyl, and when a plurality of substituents are present, the plurality of substituents are the same or different.

In a preferred embodiment of the present disclosure, the compound has the following structure:

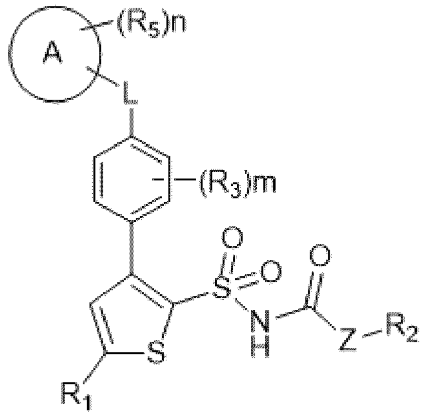

III,

wherein:

L is $C_1$ to $C_6$ alkyl substituted with one or more substituents, each of the one or more substituents being independently selected from halogen, $C_1$ to $C_6$ alkyl, halogenated $C_1$ to $C_6$ alkyl, or oxo (=O); and
m is selected from 0, 1, 2, 3, or 4; preferably, m is 0;
$R_1$, $R_2$, $R_3$, $R_5$, Z and n are as defined in the first aspect of the present disclosure; and
ring A is as defined in the first aspect of the present disclosure.

In a preferred embodiment of the present disclosure, the compound has the following structure:

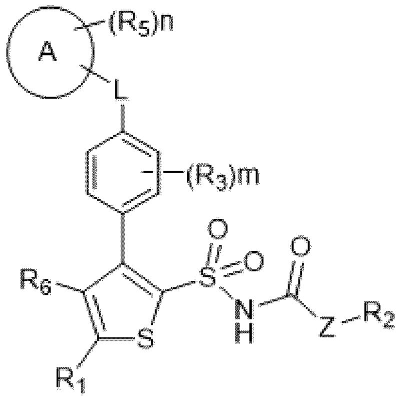

IV,

wherein:

when $R_6$ is selected from halogen, hydroxyl, cyano, amino, $C_1$ to $C_6$ alkyl, $C_3$ to $C_7$ cycloalkyl, $C_1$ to $C_6$ alkoxy, or -O-$C_3$-$C_7$ cycloalkyl, $R_6$ is independently substituted with 0, 1, 2, 3, or 4 substituents selected from halogen, hydroxyl, amino, cyano, $C_1$ to $C_6$ alkyl, or $C_3$ to $C_7$ cycloalkyl, when a plurality of substituents are present, the plurality of substituents are the same or different;
m is selected from 0, 1, 2, 3, or 4;
L is unsubstituted $C_1$ to $C_6$ alkyl or $C_1$ to $C_6$ alkyl substituted with one or more substituents, each of the one or more substituents being independently selected from halogen, $C_1$ to $C_6$ alkyl, halogenated $C_1$ to $C_6$ alkyl, or oxo (=O);
$R_1$, $R_2$, $R_3$, and $R_5$ are as defined in the first aspect of the present disclosure;
Z and n are as defined in the first aspect of the present disclosure; and
ring A is as defined in the first aspect of the present disclosure.

In a preferred embodiment of the present disclosure, the compound has the following structure:

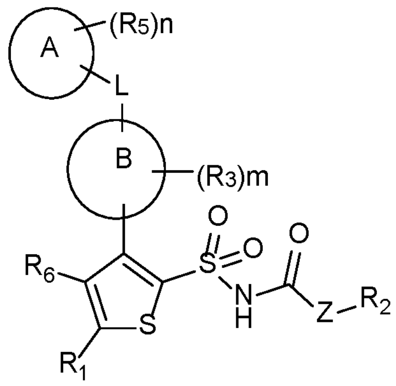

V,

wherein:

ring B is selected from a 5- to 6-membered heteroaryl;
$R_5$ is selected from H;
when $R_6$ is hydrogen or selected from halogen, hydroxyl, cyano, amino, $C_1$ to $C_6$ alkyl, $C_3$ to $C_7$ cycloalkyl, $C_1$ to $C_6$ alkoxy, or -O-$C_3$-$C_7$ cycloalkyl, $R_6$ is independently substituted with 0, 1, 2, 3, or 4 substituents selected from halogen, hydroxyl, amino, cyano, $C_1$ to $C_6$ alkyl, or $C_3$ to $C_7$ cycloalkyl, when a plurality of substituents are present, the plurality of substituents are the same or different;
m is selected from 0, 1, 2, 3, or 4;
L is unsubstituted $C_1$ to $C_6$ alkyl or $C_1$ to $C_6$ alkyl substituted with one or more substituents, each of the one or more substituents being independently selected from halogen, $C_1$ to $C_6$ alkyl, halogenated $C_1$ to $C_6$ alkyl, or oxo (=O);
$R_1$, $R_2$, and $R_3$ are as defined in the first aspect of the present disclosure;
Z and n are as defined in the first aspect of the present disclosure; and
ring A is as defined in the first aspect of the present disclosure.

In a preferred embodiment of the present disclosure, L is $C_1$ to $C_6$ alkyl; and L is optionally substituted with halogen, $C_1$ to $C_6$ alkyl, halogenated $C_1$ to $C_6$ alkyl, or oxo (=O).

In a preferred embodiment of the present disclosure, L is $C_1$ to $C_3$ alkyl or $C_1$ to $C_3$ alkyl substituted with $C_1$ to $C_6$ alkyl.

In a preferred embodiment of the present disclosure, L is $-CH_2-$ or $-CH(CH_3)-$.

In a preferred embodiment of the present disclosure, $R_3$ is selected from cyano, amino, $C_1$ to $C_3$ alkyl, $C_3$ to $C_7$ cycloalkyl, $C_1$ to $C_3$ alkoxy, or -O-$C_3$-$C_7$ cycloalkyl; and $R_3$ is independently substituted with 0, 1, 2, 3, or 4 substituents selected from halogen, hydroxyl, amino, cyano, $C_1$ to $C_6$ alkyl, or $C_3$ to $C_7$ cycloalkyl, when a plurality of substituents are present, the plurality of substituents are the same or different.

In a preferred embodiment of the present disclosure, $R_3$ is selected from cyano, methyl, methoxy, halogenated methyl, or cyclopropyl.

In a preferred embodiment of the present disclosure, ring B is a 5- to 6-membered heteroaromatic ring having 1 or 2 heteroatoms of N.

In a preferred embodiment of the present disclosure, ring B is selected from a pyridine ring, a pyrimidine ring, a pyrazine ring, or a pyridazine ring; and $R_3$ is selected from hydroxy, cyano, amino, oxo (=O), $C_1$ to $C_6$ alkyl, $C_3$ to $C_7$ cycloalkyl, $C_1$ to $C_6$ alkoxy, or -O-$C_3$-$C_7$ cycloalkyl, and $R_3$ is independently substituted with 0, 1, 2, 3, or 4 substituents selected from halogen, hydroxyl, amino, cyano, $C_1$ to $C_6$ alkyl, or $C_3$ to $C_7$ cycloalkyl, when a plurality of substituents are present, the plurality of substituents are the same or different.

In a preferred embodiment of the present disclosure, $R_3$ is selected from cyano, $C_1$ to $C_3$ alkyl, $C_3$ to $C_6$ cycloalkyl, halogenated $C_1$ to $C_3$ alkyl, or $C_1$ to $C_3$ alkoxy.

In a preferred embodiment of the present disclosure, $R_3$ is selected from cyano, methyl, methoxy, halogenated methyl, or cyclopropyl.

In a preferred embodiment of the present disclosure, ring B is a benzene ring substituted with 1, 2, 3, or 4 of substituents $R_3$, and $R_3$ is selected from hydroxyl, cyano, amino, oxo (=O), $C_1$ to $C_6$ alkyl, $C_3$ to $C_7$ cycloalkyl, $C_1$ to $C_6$ alkoxy, or -O-$C_3$-$C_7$ cycloalkyl; and $R_3$ is independently substituted with 0, 1, 2, 3, or 4 substituents selected from halogen, hydroxyl, amino, cyano, $C_1$ to $C_6$ alkyl, or $C_3$ to $C_7$ cycloalkyl, when a plurality of substituents are present, the plurality of substituents are the same or different.

In a preferred embodiment of the present disclosure, $R_3$ is selected from cyano, $C_1$ to $C_3$ alkyl, $C_3$ to $C_6$ cycloalkyl, halogenated $C_1$ to $C_3$ alkyl, or $C_1$ to $C_3$ alkoxy.

In a preferred embodiment of the present disclosure, $R_3$ is selected from cyano, methyl, methoxy, halogenated methyl, or cyclopropyl.

In a preferred embodiment of the present disclosure, $R_1$ is selected from $C_1$ to $C_6$ alkyl, halogenated $C_1$ to $C_6$ alkyl, $C_3$ to $C_7$ cycloalkyl, or $C_3$ to $C_7$ cycloalkyl-$C_1$ to $C_6$ alkyl.

In a preferred embodiment of the present disclosure, $R_1$ is selected from $C_1$ to $C_6$ alkyl.

In a preferred embodiment of the present disclosure, $R_1$ is isobutyl.

In a preferred embodiment of the present disclosure, $R_2$ is selected from $C_1$ to $C_6$ alkyl or halogenated $C_1$ to $C_6$ alkyl; and $R_2$ is substituted with 0, 1, 2, 3, or 4 substituents selected from halogen, hydroxyl, amino, cyano, $C_1$ to $C_6$ alkyl, $C_3$ to $C_7$ cycloalkyl, 5- to 6-membered aryl, or 5- to 6-membered heteroaryl, when a plurality of substituents are present, the plurality of substituents are the same or different.

In a preferred embodiment of the present disclosure, $R_2$ is selected from methyl, ethyl, propyl, isopropyl, trifluoropropyl, butyl, isobutyl, or $C_1$ to $C_3$ alkyl-6 membered heteroaryl.

In a preferred embodiment of the present disclosure, $R_2$ is selected from methyl, ethyl, propyl, isopropyl, butyl, or isobutyl.

In a preferred embodiment of the present disclosure, $R_2$ is methyl, trifluoropropyl, butyl, or pyridylmethyl.

In a preferred embodiment of the present disclosure, $R_2$ is butyl.

In a preferred embodiment of the present disclosure, Z is selected from -O-, -NH-, or -N-$C_1$ to $C_3$ alkyl-.

In a preferred embodiment of the present disclosure, Z is -O- or -NH-.

In a preferred embodiment of the present disclosure, Z is -NH-.

In a preferred embodiment of the present disclosure, ring A is 5- to 6-membered heteroaryl containing 1, 2, and 3 heteroatoms; and n is 1, 2, 3, 4, or 5.

In a preferred embodiment of the present disclosure, the heteroatom is selected from N, O, or S.

In a preferred embodiment of the present disclosure, the heteroatom is N.

In a preferred embodiment of the present disclosure, ring A is selected from pyrrole, pyrazole, imidazole, triazole, pyridine, pyrimidine, pyridazine, pyrazine, or triazine.

In a preferred embodiment of the present disclosure,

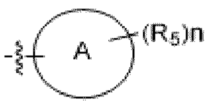

has a structure of

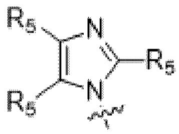
,
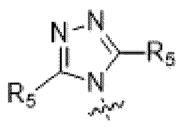
,
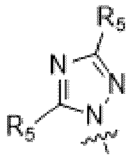
,
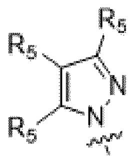
, or
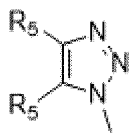
;

and $R_5$ is selected from H, halogen, -CN, $C_1$ to $C_6$ alkyl, $C_1$ to $C_6$ alkoxy, $C_3$ to $C_7$ cycloalkyl, or -O-$C_3$-$C_7$ cycloalkyl, when a plurality of substituents $R_5$ are present, the plurality of substituents $R_5$ are the same or different.

In a preferred embodiment of the present disclosure, $R_5$ is selected from H, halogen, methyl, ethyl, tert-butyl, cyclopropyl, or halogenated methyl.

In a preferred embodiment of the present disclosure,

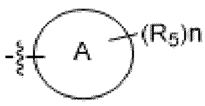

has a structure of

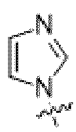, 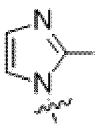, 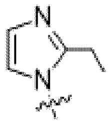, or 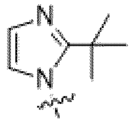.

In a preferred embodiment of the present disclosure, $R_6$ is selected from H, halogen, hydroxyl, cyano, amino, $C_1$ to $C_3$ alkyl, $C_1$ to $C_3$ alkoxy, $C_3$ to $C_7$ cycloalkyl, or -O-$C_3$-$C_7$ cycloalkyl; and $R_6$ is independently substituted with 0, 1, 2, 3, or 4 substituents selected from halogen, hydroxyl, amino, cyano, $C_1$ to $C_6$ alkyl, or $C_3$ to $C_7$ cycloalkyl, when a plurality of substituents are present, the plurality of substituents are the same or different.

In a preferred embodiment of the present disclosure, $R_6$ is selected from H, halogen, hydroxyl, cyano, amino, $C_1$ to $C_3$ alkyl, $C_1$ to $C_3$ alkoxy, or $C_3$ to $C_7$ cycloalkyl.

In a preferred embodiment of the present disclosure, $R_6$ is selected from H, halogen, or $C_1$ to $C_3$ alkyl.

In a preferred embodiment of the present disclosure, $R_6$ is selected from H, fluorine, or methyl.

In a preferred embodiment of the present disclosure, when ring B is the benzene ring, when $R_6$ is H, and when m is selected from 1, 2, 3, or 4, L is unsubstituted $C_1$ to $C_3$ alkyl or $C_1$ to $C_3$ alkyl substituted with $C_1$ to $C_6$ alkyl;

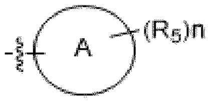

has a structure of

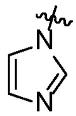,

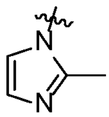, 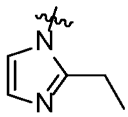, 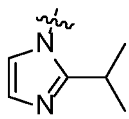, or 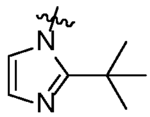;

$R_1$ is selected from isobutyl; $R_3$ is selected from cyano or methyl; Z is selected from -O- or -NH-; and $R_2$ is selected from $C_1$ to $C_6$ alkyl, and $R_2$ is substituted with 0, 1, 2, or 3 substituents selected from halogen, hydroxyl, or 5- to 6-membered heteroaryl.

In a preferred embodiment of the present disclosure, L is -$CH_2$- or -$CH(CH_3)$-.

In a preferred embodiment of the present disclosure, when ring B is the benzene ring, when $R_6$ is H, and when m is 0, L is unsubstituted $C_1$ to $C_3$ alkyl or $C_1$ to $C_3$ alkyl substituted with $C_1$ to $C_6$ alkyl;

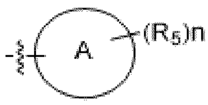

has a structure of

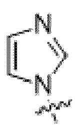 or 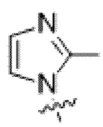;

$R_1$ is selected from isobutyl; $R_3$ is selected from cyano or methyl; Z is selected from -O-; and $R_2$ is selected from $C_1$ to $C_6$ alkyl.

In a preferred embodiment of the present disclosure, L is -$CH_2$- or -$CH(CH_3)$-.

In a preferred embodiment of the present disclosure, when ring B is the benzene ring, when $R_6$ is not hydrogen and is selected from halogen or $C_1$ to $C_3$ alkyl, and when m is 0 or 1, L is $C_1$ to $C_3$ alkyl or $C_1$ to $C_3$ alkyl substituted with $C_1$ to

$C_6$ alkyl;

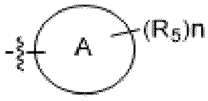

has a structure of

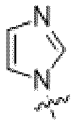, 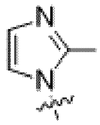, 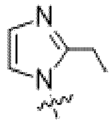, 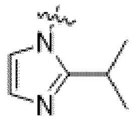, 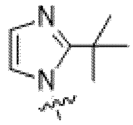, or 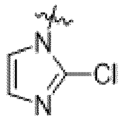;

$R_1$ is selected from isobutyl; $R_3$ is selected from methyl; Z is selected from -O-; and $R_2$ is selected from $C_1$ to $C_6$ alkyl.

In a preferred embodiment of the present disclosure, $R_6$ is selected from fluorine or methyl.

In a preferred embodiment of the present disclosure, ring B is the 5- to 6-membered heteroaryl, $R_5$ and $R_6$ are both hydrogen; m is 0;

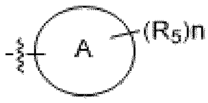

has a structure of

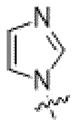,

$R_1$ is selected from isobutyl; Z is selected from -O-, and $R_2$ is selected from $C_1$ to $C_6$ alkyl.

In a preferred embodiment of the present disclosure, the 5- to 6-membered heteroaryl is selected from a pyridine ring, a pyrimidine ring, a pyrazine ring, or a pyridazine ring.

In a preferred embodiment of the present disclosure, for the compound represented by Formula I, or the tautomer, stereoisomer, hydrate, solvate, pharmaceutically acceptable salt, or prodrug thereof, the compound includes:

| | | |
|---|---|---|
| 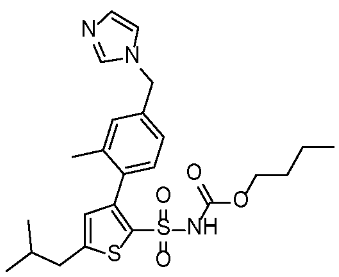 | 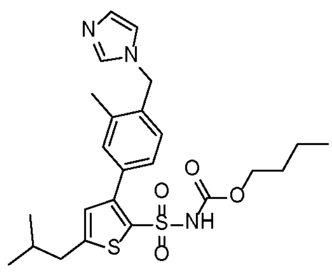 | 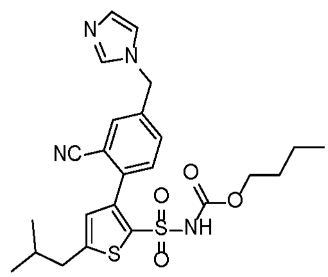 |
| I-1 | I-2 | I-3 |
| 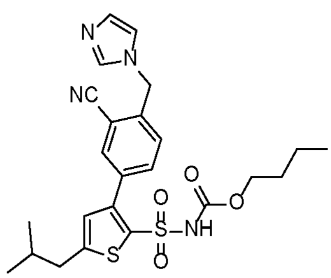 | 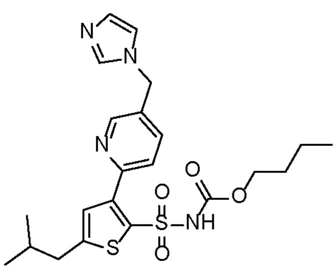 | 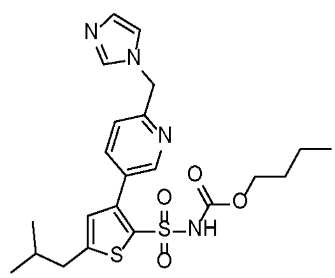 |

(continued)

| | | |
|---|---|---|
| I-4 | I-5 | I-6 |
| 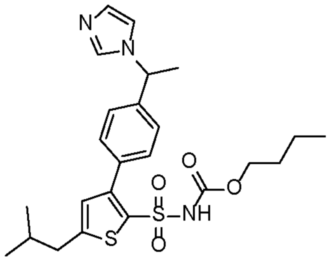 | 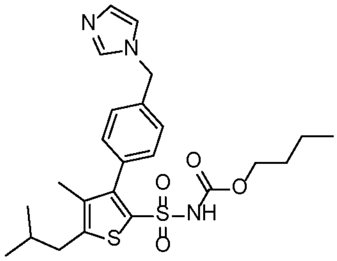 | 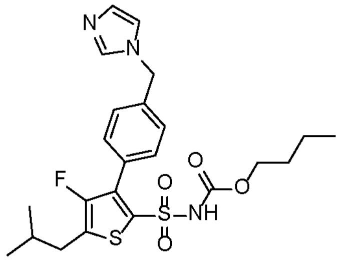 |
| I-7 | I-8 | I-9 |
| 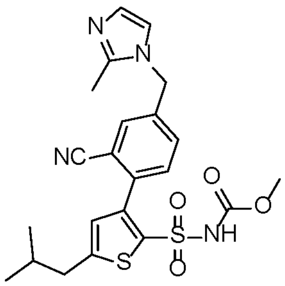 | 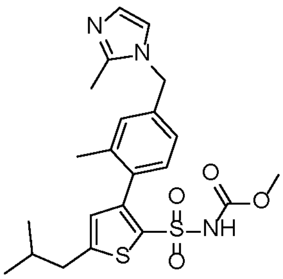 | 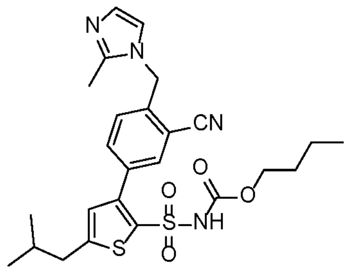 |
| I-10 | I-11 | I-12 |
| 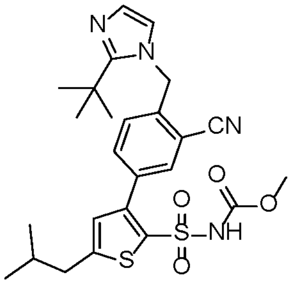 | 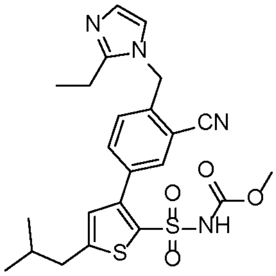 | 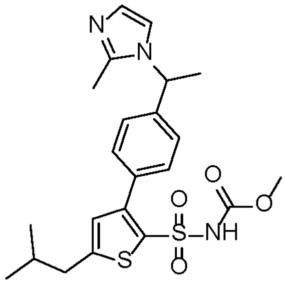 |
| I-13 | I-14 | I-15 |
| 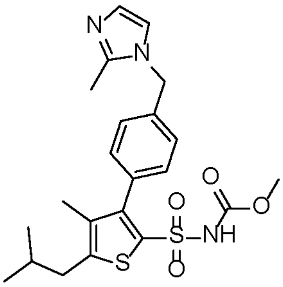 | 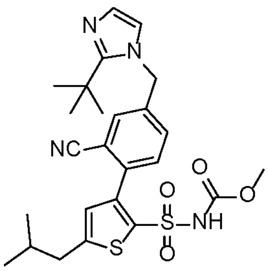 | 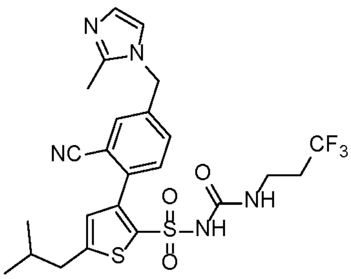 |
| I-16 | I-17 | I-18 |
| 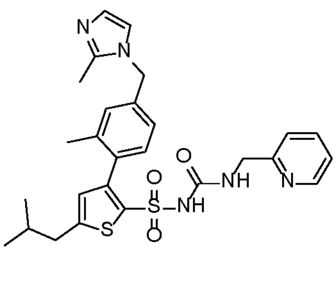 | 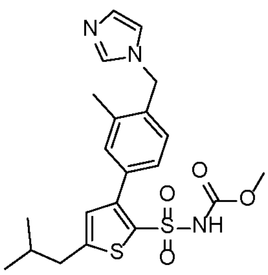 | 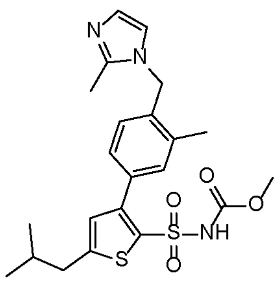 |
| I-19 | I-20 | I-21 |

(continued)

| | | |
|---|---|---|
| 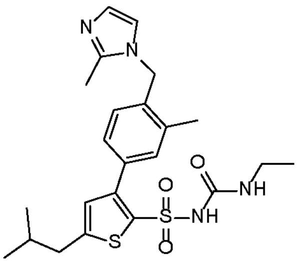 | 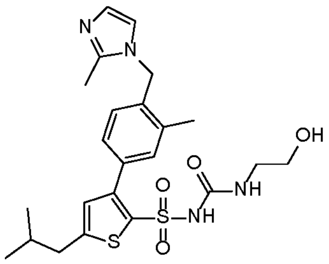 | 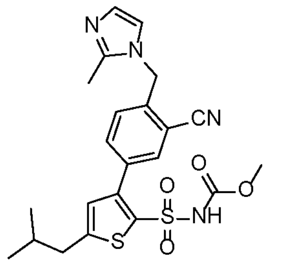 |
| I-22 | I-23 | I-24 |
| 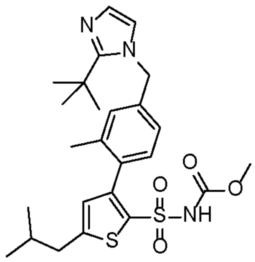 | 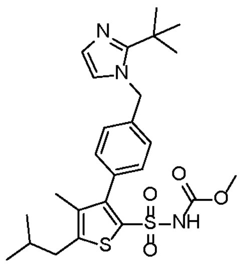 | 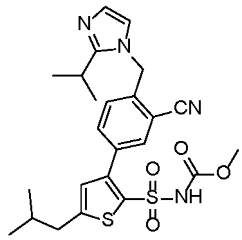 |
| I-25 | I-26 | I-27 |
| 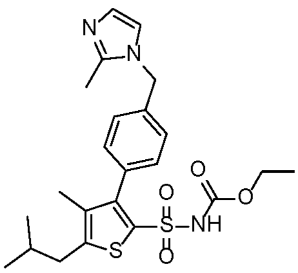 | 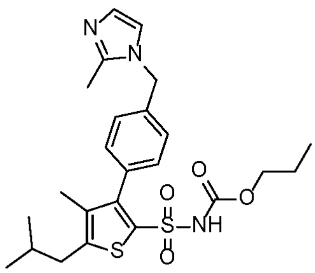 | 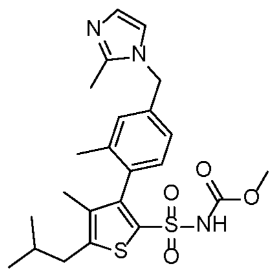 |
| I-28 | I-29 | I-30 |
| 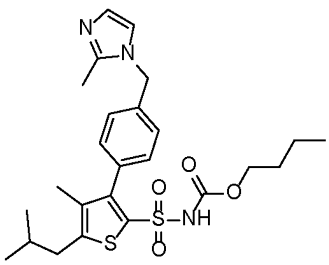 | 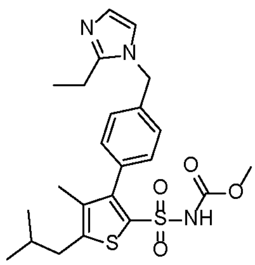 | 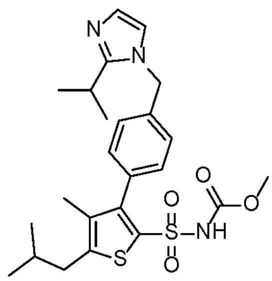 |
| I-31 | I-32 | I-33 |
| 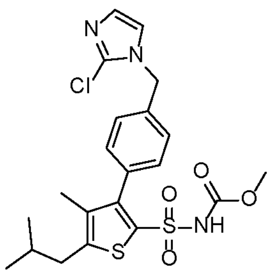 | 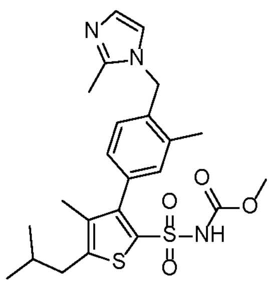 | 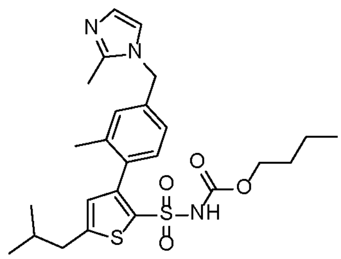 |
| I-34 | I-35 | I-36. |

In a second aspect, the present disclosure provides a pharmaceutical composition. The pharmaceutical composition includes the compound represented by Formula I, or the tautomer, stereoisomer, hydrate, solvate, pharmaceutically acceptable salt, or prodrug thereof according to the first aspect, and a pharmaceutically acceptable carrier.

In a third aspect, the present disclosure provides use of the compound represented by Formula I, or the tautomer, stereoisomer, hydrate, solvate, pharmaceutically acceptable salt, or prodrug thereof according to the first aspect, or the pharmaceutical composition according to the second aspect. The use includes: acting as an AT2 receptor agonist; and/or preventing and/or treating a disease of insufficient endogenous generation of Ang II; and/or preventing and/or treating a disease in which an increased effect of Ang II is desired or required; and/or preparing a medicament, pharmaceutical composition, or formulation as an AT2 receptor agonist, and/or a medicament, pharmaceutical composition, or formulation for preventing and/or treating a disease in which the AT2 receptor is expressed and a stimulation of the AT2 receptor is desired or necessary.

The compound represented by Formula I, or the tautomer, stereoisomer, hydrate, solvate, pharmaceutically acceptable salt, or prodrug thereof according to the first aspect, or the pharmaceutical composition according to the second aspect can be used to treat a disease of gastrointestinal tract, cardiovascular system, respiratory tract, kidney, eyes, female reproductive system, or central nervous system (CNS).

It should be noted that the gastrointestinal tract diseases include esophagitis, Barrett's esophagus, gastric ulcer, duodenal ulcer, dyspepsia (including non-ulcer dyspepsia), gastroesophageal reflux, allergic bowel syndrome, inflammatory enteritis, pancreatitis, liver disease (such as hepatitis), gallbladder disease, multiple organ failure, and sepsis. It should be noted that other gastrointestinal tract diseases include xerostomia, gastritis, gastric stasis, hyperacidity, biliary tract disease, abdominal disease, segmental ileitis, ulcerative colitis, diarrhea, constipation, acute colic, dysphagia, nausea, vomiting, and Sjögren's syndrome.

It should be noted that the respiratory diseases include inflammatory diseases such as asthma, obstructive pulmonary disease (e.g., chronic obstructive pulmonary disease), pneumonia, pulmonary hypertension, adult respiratory distress syndrome, or idiopathic pulmonary fibrosis.

It should be noted that the kidney diseases include renal failure, nephritis, and renal hypertension.

It should be noted that the eyes diseases include diabetic retinopathy, retinopathy of prematurity, and retinal microvascularization,

It should be noted that the female reproductive system diseases include ovulatory mechanism disorder.

It should be noted that the cardiovascular system diseases include hypertension, myocardial hypertrophy, heart failure, atherosclerosis, arterial thrombosis, venous thrombosis, endothelial dysfunction, endothelial damage, stenosis after balloon dilation, angiogenesis, diabetic complications, microvascular dysfunction, angina pectoris, arrhythmia, intermittent claudication, pre-eclampsia, myocardial infarction, reinfarction, ischemic damage, erectile dysfunction, and neointimal hyperplasia.

It should be noted that the CNS diseases include cognitive dysfunction, feeding dysfunction, thirst, stroke, cerebral hemorrhage, cerebral embolism, and cerebral infarction.

The compound represented by Formula I, or the tautomer, stereoisomer, hydrate, solvate, pharmaceutically acceptable salt, or prodrug thereof according to the first aspect, or the pharmaceutical composition according to the second aspect can also be used for the regulation of growth metabolism and proliferation, for example, for the treatment of hypertrophy, prostatic hyperplasia, autoimmune diseases, psoriasis, obesity, nerve regeneration, ulcers, inhibition of adipose tissue hypertrophy, stem cell differentiation and proliferation, cancer (such as gastrointestinal cancer, lung cancer, etc.), apoptosis, tumors (generally), proliferative diabetes, nerve damage, and organ rejection.

The compound represented by Formula I, or the tautomer, stereoisomer, hydrate, solvate, pharmaceutically acceptable salt, or prodrug thereof according to the first aspect, or the pharmaceutical composition according to the second aspect is suitable for the treatment and/or preventive treatment of the above-mentioned diseases.

In a fourth aspect, the present disclosure provides a method for treating a disease. The disease includes a disease of insufficient endogenous generation of Ang II, and/or a disease in which an increased effect of Ang II is desired or required, and/or a disease in which an AT2 receptor is expressed and a stimulation of the AT2 receptor is desired or necessary. The method includes: administering a therapeutically effective amount of the compound represented by Formula I, and tautomer, stereoisomer, hydrate, solvate, pharmaceutically acceptable salt, or prodrug thereof according to the first aspect or the pharmaceutical composition according to the second aspect of the present disclosure to a person suffering from or susceptible to the disease.

Additional aspects and advantages of the present disclosure will be provided in part in the following description, or will become apparent in part from the following description, or can be learned from practicing of the present disclosure.

## DETAILED DESCRIPTION

Embodiments of the present disclosure will be described in detail below. The embodiments described below are illustrative only, and they are intended to explain, rather than limiting, the present disclosure.

In addition, terms such as "first" and "second" are used herein for purposes of description and are not intended to indicate or imply relative importance, or to implicitly show the number of technical features indicated. Thus, the feature

defined with "first" and "second" may explicitly or implicitly comprise one or more this feature. In the description of the present disclosure, "a plurality of" means at least two, for example, two, three, etc., unless specified otherwise.

**Terms and Definitions**

Unless otherwise stated, the definitions of groups and terms described in the specification and claims include actual definitions, exemplary definitions, preferred definitions, definitions recorded in tables, and definitions of specific compounds in the examples, etc., which may be arbitrarily combined and integrated with each other. The group definitions and compound structures that are combined and integrated should fall within the scope of the present disclosure.

Unless defined otherwise, all technical and scientific terms herein have the same meaning as commonly understood by one of ordinary skill in the art to which the claimed subject matter belongs. The patents, patent applications, publications cited herein are hereby incorporated by reference in their entireties, unless stated otherwise. When a term has multiple definitions, that defined in this section will prevail.

It should be understood that the foregoing general description and the following detailed description are illustrative and merely for explanation, rather than limiting the subject matter of the present disclosure in any way. In the present disclosure, unless specifically stated otherwise, the use of the singular also includes the plural. It must be noted that, as used in this specification and the claims, the singular forms include plural referents unless the context clearly dictates otherwise. It should also be noted that the use of "or" or "either" means "and/or" unless stated otherwise. Furthermore, use of the term "include", as well as other forms, such as "comprising", "including", and "containing", is not for limitation.

Definitions of standard chemical terms may be found in reference document (including "ADVANCED ORGANIC CHEMISTRY 4THED.", Carey and Sundberg, Vols. A(2000)and B(2001), Plenum Press, New York). Unless otherwise indicated, conventional methods in the related art, for example, mass spectroscopy, NMR, IR and UV/Vis spectroscopy, and pharmacological methods, are employed. Unless specific definitions are set forth, the terms in the related description of analytical chemistry, organic synthetic chemistry, and medicinal and medicinal chemistry are those known in the art. Standard techniques may be used in chemical synthesis, chemical analysis, pharmaceutical preparation, formulation, and delivery, and treatment of patients. For example, reactions and purifications can be performed using the manufacturer's instructions of the kit, or in a manner well known in the related art or as described herein. The techniques and procedures described above may generally be performed with conventional methods well known in the art according to the description in a number of general and more specific documents cited and discussed throughout the present description. Throughout the description, groups and substituents thereof can be chosen by one skilled in the field to provide stable moieties and compounds.

Where substituent groups are depicted by conventional chemical formulae, written from left to right, the substituent groups also encompass the chemically equivalent substituents that would result from writing the structural formula from right to left. For example, $CH_2O$ is equivalent to $OCH_2$. As used herein,

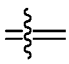 or 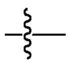

refers to the point of attachment of a group. As used herein, "$R_1$", "R1", and "$R^1$" have the same meaning and are interchangeable. For other symbols such as $R_2$, similar definitions have the same meanings.

The section headings used herein are for organizational purposes only and are not to be construed as limiting the subject matter described. All documents or portions of documents cited in the present disclosure, including but not limited to patents, patent applications, articles, books, manuals, and treatises, are incorporated herein by reference in their entireties.

In addition to the foregoing, the following terms, when used in the specification and claims of the present disclosure, have the meanings indicated below, unless otherwise specifically stated.

As used herein, numerical ranges recited in the description and claims, when interpreted as "integers", should be understood as including both endpoints of the range and each integer within the range. For example, "an integer ranging from 1 to 6" should be understood as reciting each integer of 1, 2, 3, 4, 5, and 6.

In the present disclosure, "AT2 receptor" and "AT2R" have the same definition.

In the present disclosure, the term "halogen" refers to fluorine, chlorine, bromine, and iodine, either alone or as part of other substituents.

In the present disclosure, the term "amino" refers to $-NH_2$, either alone or as part of other substituents.

In the present disclosure, the term "hydroxy" refers to -OH, either alone or as part of other substituents.

In the present disclosure, the term "cyano" refers to -CN, either alone or as part of other substituents.

As used herein, the term "alkyl", either alone or as part of other substituents, means a straight or branched hydrocarbon chain group consisting solely of carbon and hydrogen atoms, containing no unsaturated bonds, and having,

for example, 1 to 6 carbon atoms and connected to the rest of the molecule by a single bond. Examples of alkyl include, but are not limited to, methyl, ethyl, n-propyl, isopropyl, n-butyl, isobutyl, tert-butyl, pentyl, isopentyl, neopentyl, and hexyl. Alkyl may be unsubstituted or substituted with one or more suitable substituents. Alkyl may also be an isotopic isomer of the naturally abundant alkyl enriched in an isotope of carbon and/or hydrogen (i.e., deuterium or tritium). As used herein, the term "alkenyl" refers to an unbranched or branched monovalent hydrocarbon chain containing one or more carbon-carbon double bonds. As used herein, the term "alkynyl" refers to an unbranched or branched monovalent hydrocarbon chain containing one or more carbon-carbon triple bonds.

The term "$C_1$ to $C_6$ alkyl", either alone or as part of other substituents, should be interpreted as a straight or branched chain saturated hydrocarbon group having 1, 2, 3, 4, 5, or 6 carbon atoms. Examples of "$C_1$ to $C_6$ alkyl" are, for example, methyl, ethyl, propyl, butyl, pentyl, hexyl, isopropyl, isobutyl, sec-butyl, tert-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, 1,2-dimethylbutyl, or isomers thereof. In particular, the group has 1, 2, or 3 carbon atoms ("$C_1$ to $C_3$ alkyl"), for example methyl, methylene, ethyl, n-propyl, or isopropyl.

The term "$C_1$ to $C_6$ alkoxy", either alone or as part of other substituents, should be interpreted to include a straight or branched saturated hydrocarbon group having 1, 2, 3, 4, 5, or 6 carbon atoms and an oxygen atom, or present as $C_1$ to $C_6$ alkyl-O-$C_1$ to $C_6$ alkyl as defined in the present specification, the oxygen atom being attached to any carbon atom of the straight or branched chain of the $C_1$ to $C_6$ alkyl, including but not limited to: methoxy ($CH_3$-O-), ethoxy ($C_2H_5$-O-), propoxy ($C_3H_7$-O-), and butoxy ($C_4H_9$-O-).

The term "oxo", either alone or as part of other substituents, refers to that two hydrogen atoms of methylene group are substituted by oxygen atoms, i.e., the methylene group is substituted with a carbonyl group, denoted as =O.

The term "cycloalkyl" or "carbocyclyl", either alone or as part of other substituents, refers to a cyclic alkyl. The term "m- to n-membered cycloalkyl" or "$C_m$ to $C_n$ cycloalkyl" should be interpreted as a saturated, unsaturated, or partially saturated carbocyclic ring having from m to n atoms. For example, "3- to 15-membered cycloalkyl" or "$C_3$ to $C_{15}$ cycloalkyl" refers to a cyclic alkyl group containing 3 to 15, 3 to 9, 3 to 6, or 3 to 5 carbon atoms, which may contain 1 to 4 rings. "5- to 8-membered cycloalkyl" contains 5 to 8 carbon atoms. It includes monocyclic, bicyclic, tricyclic, spirocyclic, or bridged rings. Examples of unsubstituted cycloalkyl include, but are not limited to, cyclopropyl, cyclobutyl, cyclopentyl, cyclohexyl, and adamantyl, or a bicyclic hydrocarbon group such as a decalin ring. Cycloalkyl may be substituted with one or more substituents. In some embodiments, cycloalkyl may be a cycloalkyl fused to an aryl or heteroaryl. The term "$C_3$ to $C_6$ cycloalkyl" should be interpreted as a saturated monocyclic or bicyclic hydrocarbon ring having 3 to 6 carbon atoms, including fused or bridged polycyclic ring systems. For example, cyclopropyl, cyclobutyl, cyclopentyl, and cyclohexyl.

"Halogenated cycloalkyl", either alone or as part of other substituents, refers to a cycloalkyl as described above, in which a number (at least one) of hydrogen atoms of cycloalkyl is substituted with fluorine, chlorine, bromine, or iodine.

"Halogenated alkyl", either alone or as part of other substituents, refers to a saturated aliphatic hydrocarbon group including branched and straight chains having a specified number of carbon atoms, and substituted with one or more halogens (e.g., $-C_vF_w$, where v=1 to 3, and w=1 to (2v+1)). Examples of halogenated alkyl include, but are not limited to, trifluoromethyl, trichloromethyl, pentafluoroethyl, pentachloroethyl, 2,2,2-trifluoroethyl, heptafluoropropyl, and heptachloropropyl.

The term "aryl", either alone or as part of other substituents, refers to a monocyclic or polycyclic carbocyclic ring having from 6 to 20 carbon atoms, in which at least one ring is aromatic. When one of the rings is a non-aromatic ring, the group may be attached via the aromatic ring or the non-aromatic ring. Examples of aryl include, but are not limited to, phenyl, naphthyl, tetrahydronaphthyl, 2,3-dihydroindenyl, biphenyl, phenanthrenyl, anthracenyl, and acenaphthenyl.

The term "heteroaromatic ring" is used interchangeably with "heteroaryl", either alone or as part of other substituents, and refers to a monocyclic or polycyclic carbocyclic ring, in which at least one ring atom is a heteroatom independently selected from oxygen, sulfur and nitrogen, and the remaining ring atoms are C, and in which at least one ring is aromatic. The group may be a carbon group or a heteroatom group (i.e., C-attached or N-attached, as long as this is possible). When one of the rings is a non-aromatic ring, the group may be attached via the aromatic ring or the non-aromatic ring. Examples of heteroaryl include, but are not limited to, imidazolyl, acridinyl, carbazolyl, cinnolinyl, quinoxalinyl, pyrazolyl, indolyl, benzotriazolyl, furanyl, thienyl, benzothienyl, benzofuranyl, quinolinyl, isoquinolinyl, oxazolyl, isoxazolyl, indolyl, pyrazinyl, pyridazinyl, pyridinyl, pyrimidinyl, pyrrolyl, N-methylpyrrolyl, and tetrahydroquinoline. The term "heteroaromatic ring" may be used interchangeably with the term "heteroaryl" or "heteroaromatic ring group".

The term "5- to 6-membered heteroaromatic ring", either alone or as part of other substituents, may be used interchangeably with "5- to 6-membered heteroaryl", and should be understood as an aromatic ring group having 5 or 6 ring atoms and containing 1 to 3 heteroatoms independently selected from N, O, and S. The term "5- to 6-membered heteroaromatic ring" is to be understood as an aromatic ring group having 5 or 6 ring atoms and containing 1 to 3 heteroatoms independently selected from N, O, and S. In particular, heteroaryl is selected from thienyl, furanyl, pyrrolyl, oxazolyl, thiazolyl, imidazolyl, or pyrazolyl; or pyridinyl, pyridazinyl, pyrimidinyl, or pyrazinyl.

In the present application, "optional" or "optionally" means that the subsequently described event or situation may or may not occur, and such an expression includes both occurrences and non-occurrences of the event or condition. For example, "optionally substituted aryl" means that the aryl is substituted or unsubstituted, and such an expression includes both substituted and unsubstituted aryl.

In the present disclosure, the term "salt" or "pharmaceutically acceptable salt" includes pharmaceutically acceptable acid addition salts and pharmaceutically acceptable base addition salts. The term "pharmaceutically acceptable" means the compounds, materials, compositions and/or dosage forms that are suitable for use in contact with human and animal tissues without excess toxicity, irritation, allergic reactions or other problems or complications within the scope of reliable medical judgment, and are commensurate with a reasonable benefit/risk ratio.

"Pharmaceutically acceptable acid addition salt" refers to a salt formed with an inorganic acid or organic acid that retains the biological effectiveness of the free base without other adverse effects. "Pharmaceutically acceptable base addition salt" refers to a salt formed with an inorganic base or an organic base that retains the biological effectiveness of the free acid without other adverse effects. In addition to the pharmaceutically acceptable salts, other salts may be adopted in the present disclosure, and they can serve as intermediates in the purification of compounds or in the preparation of other pharmaceutically acceptable salts or can be used for identifying, characterizing, or purifying the compounds of the present disclosure.

The term "amine salt" refers to a product obtained by neutralizing an alkyl primary, secondary, or tertiary amine with an acid. The acid includes the inorganic acid or organic acid described in the present disclosure.

The term "stereoisomer" refers to isomers formed due to different spatial arrangements of atoms in a molecule, including a cis-trans isomer, an enantiomer, a diastereoisomer, and a conformational isomer.

According to selected raw materials and methods, the compound of the present disclosure may be present in the form of a possible isomer or a mixture of isomers, for example, in the form of a pure optical isomer or a mixture of isomers such as a mixture of racemate and diastereoisomer, depending on the number of asymmetric carbon atoms. When an optically active compound is described, prefixes *D* and *L*, or *R* and *S* are used to represent an absolute configuration of a molecule with respect to a chiral center (or multiple chiral centers) in the molecule. Prefixes *D* and *L,* or (+) and (-) are symbols used for designating the rotation of plane polarized light caused by a compound, and (-) or Z indicates that a compound is levorotatory. Compounds with the prefix (+) or *D* are dextrorotatory.

When a bond to a chiral carbon in the formula of the present disclosure is depicted as a straight line, it is to be understood that (*R*) and (*S*) configurations of the chiral carbon and produced enantiomerically pure compounds and mixtures are all included within the scope of the general formula. The racemate or enantiomerically pure compounds of the present disclosure are graphically represented with reference to Maehr, J. Chem. Ed. 1985, 62: 114-120. A wedge bond and a dashed bond are used to represent an absolute configuration of a stereocenter.

The term "tautomer" refers to functional group isomers formed by rapid movement of a certain atom in a molecule between two positions. The compound of the present disclosure may have a tautomerism phenomenon. The tautomeric compound may be present in two or more interconvertible forms. A prototropic tautomer is formed by migration of a hydrogen atom covalently bonded between two atoms. Tautomer is generally present in an equilibrium form, and separation of a single tautomer usually yields a mixture whose physicochemical properties are consistent with those of a mixture of compounds. The position of equilibrium depends on intramolecular chemical properties. For example, in several aliphatic aldehydes and ketones such as acetaldehyde, ketonic configurations prevail, while in phenol, an enolic configuration prevails. The present disclosure covers all tautomer of the compound.

In the present disclosure, "pharmaceutical composition" refers to a formulation of the compound of the present disclosure and a medium commonly accepted in the art for delivery of a biologically active compound to a mammal (e.g., a human). The medium includes a pharmaceutically acceptable carrier. The purpose of the pharmaceutical composition is to facilitate administration to an organism, facilitate the absorption of the active ingredients, and further facilitate the exertion of the biological activity.

In the present disclosure, "pharmaceutically acceptable carrier" includes, but is not limited to, any adjuvant, carrier, excipient, glidant, sweetener, diluent, preservative, dye/colorant, flavoring agent, surfactant, wetting agent, dispersant, suspending agent, stabilizer, isotonic agent, solvent, or emulsifier approved by the relevant governmental regulatory authorities as acceptable for human or livestock use.

The term "solvate" refers to the compound of the present disclosure or a salt thereof including a stoichiometric or non-stoichiometric solvent bonded through an intermolecular noncovalent force. When the solvent is water, the solvate is a hydrate.

The term "prodrug" refers to a substance that can be transformed into the compound of the present disclosure having bioactivity under the physiological conditions or by dissolving in a solvent. The prodrug of the present disclosure is prepared by modifying functional groups in the compound, and the modification can be removed by conventional operations or removed *in vivo* to obtain a parent compound. The prodrug includes a compound formed by linking one hydroxyl group or amino group in the compound of the present disclosure to any group, and when administered to an individual mammal, the prodrug of the compound of the present disclosure is cleaved to form a free hydroxyl group or free

amino group.

The compound of the present disclosure may contain an unnatural proportion of atomic isotopes at one or more atoms forming the compound. For example, the compound can be labeled with radioisotopes such as deuterium ($^2H$), tritium ($^3H$), iodine-125 ($^{125}I$), and C-14 ($^{14}C$). All changes made to the isotope composition of the compound of the present disclosure, regardless of whether they are radioactive or not, shall fall within the scope of the present disclosure.

The term "adjuvant" refers to medicinal inert ingredients. Examples of types of the term "excipient" include, but are not limited to, an adhesive, a disintegrant, a lubricant, a glidant, a stabilizer, a filler, a diluent, etc. Excipients can enhance the operating characteristics of pharmaceutical preparations, that is, improve the fluidity and/or adhesiveness to enable preparations to be more suitable for direct compression.

As used herein, the term "treatment" and other similar synonyms include the following meanings: (i) preventing a disease or condition from occurring in a mammal, particularly where such mammal is susceptible to the disease or condition but has not yet been diagnosed as having the disease or condition; (ii) inhibiting a disease or condition, i.e., suppressing development thereof; (iii) alleviating a disease or condition, that is, enabling the state of the disease or condition to regress; or (iv) alleviating the symptoms caused by the disease or condition.

The present disclosure is further described below in conjunction with specific embodiments. It should be understood that the following description is only the most preferred embodiment of the present disclosure, and should not be regarded as limiting the protection scope of the present disclosure. On the basis of fully understanding the present disclosure, the experimental methods in the following embodiments for which specific conditions are not specified are generally carried out under conventional conditions or under conditions recommended by the manufacturer. Those skilled in the art can make non-essential changes to the technical solutions of the present disclosure, and such changes should fall within the protection scope of the present disclosure.

**Definitions**

Symbol or unit:

$IC_{50}$: half inhibitory concentration, which refers to the concentration at which half of the maximum inhibitory effect is achieved.
M: mol/L, for example, n-butyllithium (14.56 mL, 29.1 mmol, 2.5 M n-hexane solution) means a solution of n-butyllithium in n-hexane with a molar concentration of 2.5 mol/L.
N: equivalent concentration, for example, 2N hydrochloric acid means 2 mol/L hydrochloric acid solution.
RT: retention time

Reagents:

DCM: dichloromethane
DMF: N,N-dimethylformamide
DIPEA: N,N-diisopropylethylamine
EA: ethyl acetate
HATU: 2-(7-azobenzotriazole)-N,N,N',N'-tetramethyluronium hexafluorophosphate
MeOH: methanol
PE: petroleum ether
Toluene: methylbenzene
THF: tetrahydrofuran
XPhos Pd G4: methanesulfonate (2-dicyclohexylphosphino-2',4',6'-triisopropyl-1,1'-biphenyl)(2'-methylamino-1,1'-biphenyl-2-yl)palladium(II)

Test method:

LCMS: liquid chromatography-mass spectrometry
TLC: thin layer chromatography

**Intermediate A1:** Preparation of Intermediate **A1**

(2-(N-(tert-butyl)sulfamoyl)-5-isobutylthiophen-3-yl)boronic acid

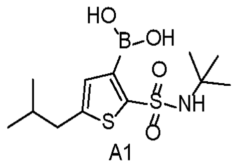

A1

The synthetic scheme of **Intermediate A1** is as follows:

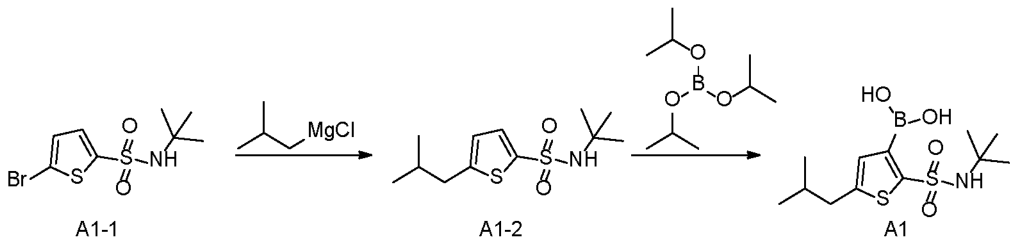


A1-1 A1-2 A1

Step 1: Synthesis of N-(tert-butyl)-5-isobutylthiophene-2-sulfonamide

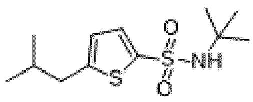

A1-2

Zinc chloride (20.0 mL, 1 mol/L THF solution) and isobutylmagnesium chloride (13.0 mL, 2 mol/L) were added to a 100 mL three-necked flask and stirred at room temperature under $N_2$ atmosphere for 10 minutes. Then, a solution of 5-bromothiophene-2-tert-butylsulfonamide (5.0 g, 16.77 mmol) and di(tri-tert-butylphosphine)palladium (428 mg, 0.83 mmol) in toluene (50 mL) was added dropwise. The mixture was heated and stirred at 120°C for 1 hour. TLC (PE: EA (V/V) =3:1) spot plate indicated complete disappearance of raw materials and generation of an obvious target spot. The reaction solution was filtered. The resulting filtrated stock was spin-dried and mixed with silica gel, and separated and purified by a silica gel column (petroleum ether: ethyl acetate (V/V)=(20:1) to (3:1)) to obtain N-(tert-butyl)-5-isobutylthiophene-2-sulfonamide (3.8 g, yield 82.3%).
$^1$H NMR (400 MHz, DMSO-d6) $\delta$ 7.66 (s, 1H), 7.39 (d, J = 3.7 Hz, 1H), 6.84 (d, J = 3.7 Hz, 1H), 2.70 (d, J = 7.1 Hz, 2H), 1.91-1.71 (m, 1H), 1.20-1.07 (m, 9H), 0.89 (d, J = 6.6 Hz, 6H)

Step 2: Synthesis of (2-(N-(tert-butyl)sulfamoyl)-5-isobutylthiophen-3-yl)boronic acid

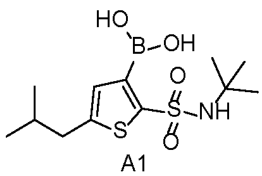

A1

In a 50 mL three-necked flask, N-(tert-butyl)-5-isobutylthiophene-2-sulfonamide (2.0 g, 7.26 mmol) was added and dissolved in THF (20 mL), and n-butyllithium (7.3 mL, 2.5 mol/L n-hexane) solution was added dropwise at room temperature under $N_2$ atmosphere. After stirring for half an hour, triisopropyl borate (2.05 g, 10.89 mmol) was added to the

reaction system. In this case, solids slowly precipitated out of the solution, and the solution was light yellow. The reaction system was stirred at room temperature overnight. The reaction was monitored with TLC (PE: EA (V/V)=3:1), until TLC indicated complete rection of raw materials and generation of a new spot of the target compound. HCl aqueous solution (5.0 mL, 2.0 mol/L) was slowly added dropwise to the reaction solution to adjust pH to 2. The reaction solution was diluted by adding 30 mL of water and extracted with ethyl acetate (50 mL×2). The organic phase was washed with saturated brine (30 mL) for extraction. The resulting organic phase was dried over anhydrous sodium sulfate and then filtered. The filtrated stock was spin-dried and mixed with silica gel, and separated and purified by a silica gel column (petroleum ether: ethyl acetate (V/V)=(20:1) to (3:1)) to obtain (2-(N-(tert-butyl)sulfamoyl)-5-isobutylthiophen-3-yl)boronic acid (**A1**) (1.8 g, yield 77.6%).

**Intermediate A2:** Preparation of Intermediate **A2**

1-(4-chlorobenzyl)-1H-imidazole

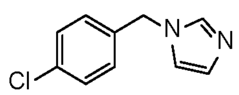

A2

The synthetic scheme of **Intermediate A2** is as follows:

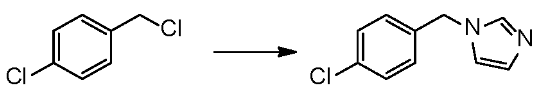

A2-1 A2

1H-imidazole (1.90 g, 27.9 mmol) and 1-chloro-4-(chloromethyl)benzene (3.00 g, 18.6 mmol) were dissolved in acetonitrile (40.0 mL), and potassium carbonate (7.72 g, 55.8 mmol) was added. The mixture was heated to 80°C and reacted for 2 hours. The reaction solution was concentrated, and the crude product was separated and purified by a silica gel column (petroleum ether: ethyl acetate (V/V)=(20:1) to (10:1)), $R_f$=0.5) to obtain 1-(4-chlorobenzyl)-1H-imidazole (3.00 g, 15.5 mmol, yield 83.3%).

LC-MS, M/Z (ESI): 193.0 $[M+H]^+$
$^1$H NMR (400 MHz, DMSO-$d_6$) $\delta$ 8.21 (s, 2H), 7.48 (d, 2H), 7.34 (d, 2H), 7.06 (s, 1H), 5.28 (s, 2H)

Example 1: Preparation of target compound I-1

Butyl ((3-(4-((1H-imidazol-1-yl)methyl)-2-methylphenyl)-5-isobutylthiophen-2-yl)sulfonyl)carbamate

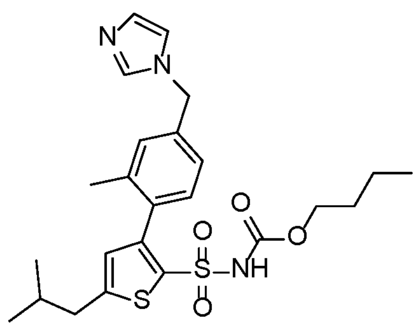

**I-1**

The synthetic scheme of the target compound 1-1 is as follows:

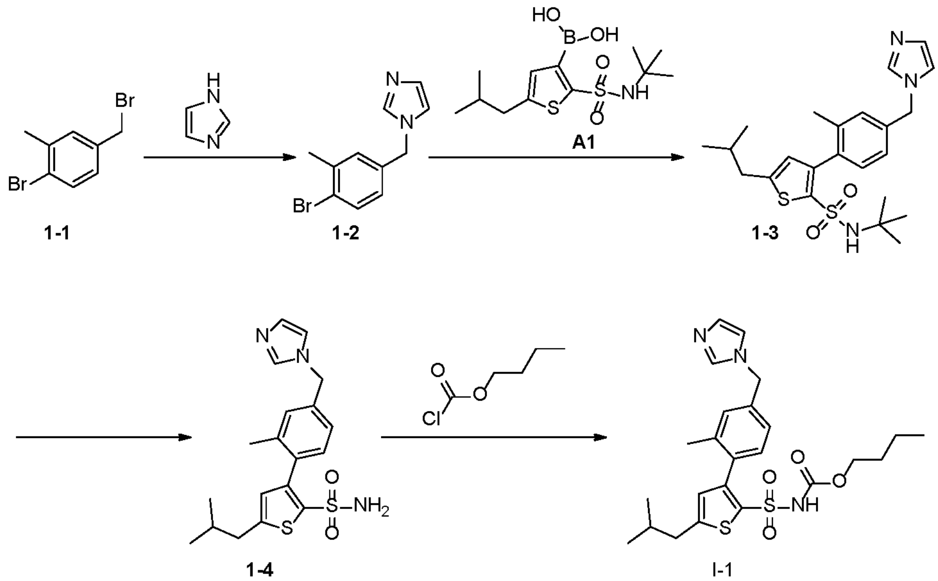


Step 1: Synthesis of 1-(4-bromo-3-methylbenzyl)-1H-imidazole

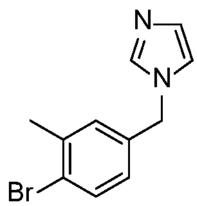

1-2

Imidazole (0.2 g, 2.9 mmol) was added to 4 mL of dimethyl sulfoxide, and potassium hydroxide (0.81 g, 14.5 mmol) was added. The mixture reacted at room temperature for 3 hours. Then, 1-bromo-4-(bromomethyl)-2-methylbenzene (0.92 g, 3.5 mmol) was added, and the mixture reacted at room temperature overnight, until LCMS indicated complete reaction of the raw materials. The reaction system was diluted by adding 30 mL of water and was extracted with ethyl acetate (50 mL×2). The organic phase was concentrated to dryness, and the residue was separated and purified by a silica gel column (DCM: MeOH (V/V)=10:1) to obtain 1-(4-bromo-3-methylbenzyl)-1H-imidazole (600 mg, yield 82.4%). LC-MS, M/Z (ESI): 251.0 $[M+H]^+$

Step 2: Synthesis of 3-(4-((1H-imidazol-1-yl)methyl)-2-methylphenyl)-N-(tert-butyl)-5-isobutylthiophene-2-sulfonamide

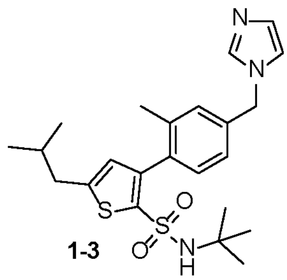

1-(4-bromo-3-methylbenzyl)-1H-imidazole (0.15 g, 0.6 mmol) was added to 2 mL of N,N-dimethylformamide, and then (2-(N-(tert-butyl)sulfamoyl)-5-isobutylthiophen-3-yl)boric acid (0.287 g, 0.9 mmol), 1,1'-bis(diphenylphosphino) ferrocene palladium(II) dichloride (0.16 g, 0.2 mmol), and potassium carbonate (0.33 g, 2.4 mmol) were added. The mixture was heated to 90°C and reacted overnight, until LCMS indicated complete reaction of the raw materials. The

reaction system was diluted by adding 5 mL of water and was extracted with ethyl acetate (10 mL×2). The organic phase was washed with water (10 mL×5), collected, concentrated, and dried. The residue was separated and purified by a silica gel column (PE: EA (V/V)=1:1) to obtain 3-(4-((1H-imidazol-1-yl)methyl)-2-methylphenyl)-N-(tert-butyl)-5-isobutylthiophene-2-sulfonamide (200 mg, yield 74.9%).
LC-MS, M/Z (ESI): 446.2 $[M+H]^+$

Step 3: Synthesis of 3-(4-((1H-imidazol-1-yl)methyl)-2-methylphenyl)-5-isobutylthiophene-2-sulfonamide

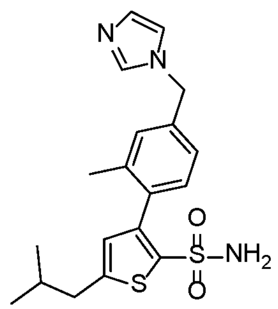

**1-4**

3-(4-((1H-imidazol-1-yl)methyl)-2-methylphenyl)-N-(tert-butyl)-5-isobutylthiophene-2-sulfonamide (0.2 g, 0.45 mmol) was added to 5 mL of trifluoroacetic acid and reacted at room temperature overnight, until LCMS indicated complete reaction of the raw materials. The reaction solution was concentrated to dryness to obtain 3-(4-((1H-imidazol-1-yl)methyl)-2-methylphenyl)-5-isobutylthiophene-2-sulfonamide (256 mg, yield 100%).
LC-MS, M/Z (ESI): 390.1 $[M+H]^+$

Step 4: Synthesis of butyl ((3-(4-((1H-imidazol-1-yl)methyl)-2-methylphenyl)-5-isobutylthiophen-2-yl)sulfonyl)carbamate

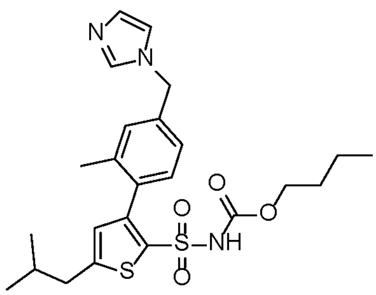

**I-1**

3-(4-((1H-imidazol-1-yl)methyl)-2-methylphenyl)-5-isobutylthiophene-2-sulfonamide (0.2 g, 0.5 mmol) was added to 5 mL of dichloromethane, and then triethylamine (0.3 g, 3.0 mmol) and butyl chloroformate (0.14 g, 1.0 mmol) were added. The mixture reacted at room temperature overnight, until LCMS indicated complete reaction of the raw materials. The reaction system was diluted by adding 5 mL of water and extracted with ethyl acetate (10 mL×2). The organic phase was concentrated to dryness, and the residue was separated and purified by a silica gel column (DCM: MeOH (V/V)=10:1) to obtain butyl ((3-(4-((1H-imidazol-1-yl)methyl)-2-methylphenyl)-5-isobutylthiophen-2-yl)sulfonyl) carbamate (I-1) (87 mg, yield 34.7%).

LC-MS, M/Z (ESI): 490.2 $[M+H]^+$
$^1H$ NMR (400 MHz, DMSO-d6) δ 8.04 (s, 1H), 7.45 (d, 1H), 7.41 (s, 1H), 7.26 (s, 1H), 7.10 (d, 1H), 6.92 (d, 1H) , 6.84 (s, 1H) ,5.29 (s, 2H), 3.80 (t, 2H), 2.65 (d, 2H) ,2.27 (s, 3H), 1.87-1.83(m, 1H), 1.39-1.34(m, 2H), 1.21-1.16(m, 2H), 0.92 (d, 6H), 0.81 (t, 3H) **Example 2:** Preparation of target compound **I-2**

Butyl ((3-(4-((1H-imidazol-1-yl)methyl)-3-methylphenyl)-5-isobutylthiophen-2-yl)sulfonyl)carbamate

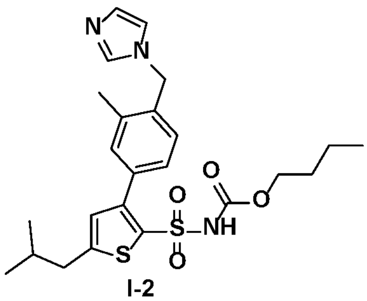

I-2

The synthetic scheme of the target compound 1-2 is as follows:

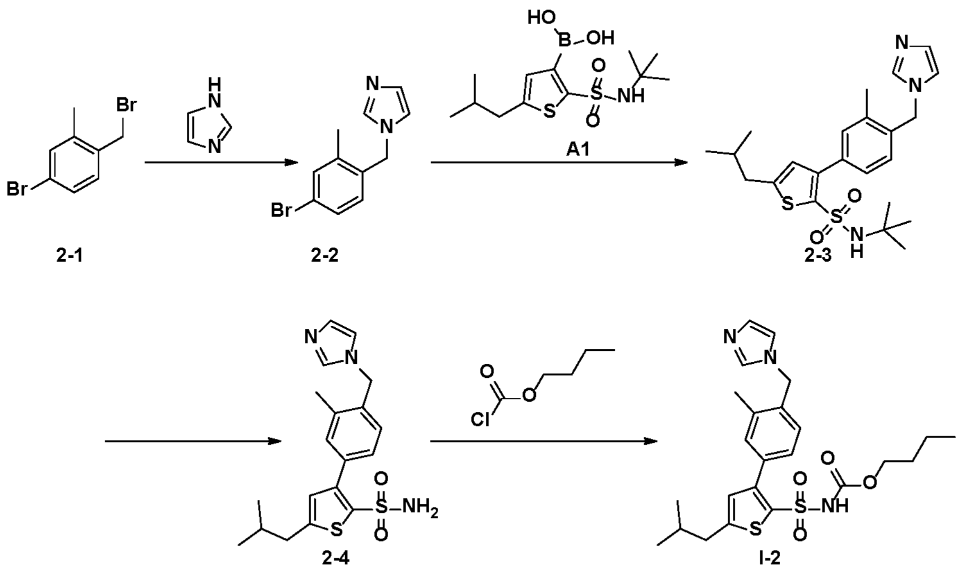


Step 1: Synthesis of 1-(4-bromo-2-methylbenzyl)-1H-imidazole

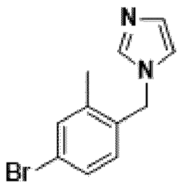

2-2

Imidazole (0.2 g, 2.9 mmol) was added to 4 mL of dimethyl sulfoxide, and potassium hydroxide (0.81 g, 14.5 mmol) was added. The mixture reacted at room temperature for 3 hours. Then, 4-bromo-1-(bromomethyl)-2-methylbenzene (0.92 g, 3.5 mmol) was added, and the mixture reacted at room temperature overnight, until LCMS indicated complete reaction of the raw materials. The reaction system was diluted by adding 30 mL of water and was extracted with ethyl acetate (50 mL×2). The organic phase was washed with water (50 mL×5), collected, concentrated, and dried, and the residue was separated and purified by a silica gel column (DCM: MeOH (V/V)=10:1) to obtain 1-(4-bromo-2-methylbenzyl)-1H-imidazole (600 mg, yield 82.4%).
LC-MS, M/Z (ESI): 251.0 $[M+H]^+$

Step 2: Synthesis of 3-(4-((1H-imidazol-1-yl)methyl)-3-methylphenyl)-N-(tert-butyl)-5-isobutylthiophene-2-sulfonamide

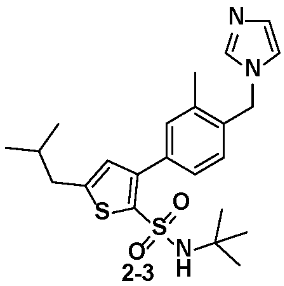

2-3

1-(4-bromo-2-methylbenzyl)-1H-imidazole (0.15 g, 0.6 mmol) was added to 2 mL of N,N-dimethylformamide, and then (2-(N-(tert-butyl)sulfamoyl)-5-isobutylthiophen-3-yl)boric acid (0.287 g, 0.9 mmol), 1,1'-bis(diphenylphosphino) ferrocene palladium(II) dichloride (0.16 g, 0.2 mmol), and potassium carbonate (0.33 g, 2.4 mmol) were added. The mixture was heated to 90°C and reacted overnight, until LCMS indicated complete reaction of the raw materials. The reaction system was diluted by adding 5 mL of water and was extracted with ethyl acetate (10 mL×2). The organic phase was washed with water (10 mL×5), collected, concentrated, and dried, and the residue was separated and purified by a silica gel column (PE: EA (V/V)=1:1) to obtain 3-(4-((1H-imidazol-1-yl)methyl)-3-methylphenyl)-N-(tert-butyl)-5-isobutylthiophene-2-sulfonamide (210 mg, yield 78.6%).
LC-MS, M/Z (ESI): 446.2 $[M+H]^+$

Step 3: Synthesis of 3-(4-((1H-imidazol-1-yl)methyl)-3-methylphenyl)-5-isobutylthiophene-2-sulfonamide

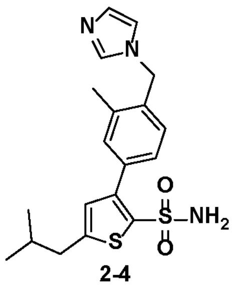

2-4

3-(4-((1H-imidazol-1-yl)methyl)-3-methylphenyl)-N-(tert-butyl)-5-isobutylthiophene-2-sulfonamide (0.21 g, 0.47 mmol) was added to 5 mL of trifluoroacetic acid and reacted at room temperature overnight, until LCMS indicated complete reaction of the raw materials. The reaction system was concentrated to dryness to obtain 3-(4-((1H-imidazol-1-yl)methyl)-3-methylphenyl)-5-isobutylthiophene-2-sulfonamide (260 mg, yield 100%).
LC-MS, M/Z (ESI): 390.1 $[M+H]^+$

Step 4: Synthesis of butyl ((3-(4-((1H-imidazol-1-yl)methyl)-3-methylphenyl)-5-isobutylthiophen-2-yl)sulfonyl)carbamate

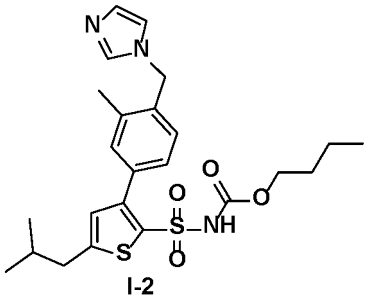

I-2

3-(4-((1H-imidazol-1-yl)methyl)-3-methylphenyl)-5-isobutylthiophene-2-sulfonamide (0.2 g, 0.5 mmol) was added to 5 mL of dichloromethane, and then triethylamine (0.3 g, 3.0 mmol) and butyl chloroformate (0.14 g, 1.0 mmol) were added. The mixture reacted at room temperature overnight. LCMS indicated complete reaction of the raw materials. The reaction system was diluted by adding 5 mL of water and extracted with ethyl acetate (10 mL × 2). The organic phase was concentrated to dryness, and the residue was separated and purified by a silica gel column (DCM: MeOH (V/V)=10:1) to obtain butyl ((3-(4-((1H-imidazol-1-yl)methyl)-3-methylphenyl)-5-isobutylthiophen-2-yl)sulfonyl)carbamate (I-2) (62 mg, yield 24.7%).

LC-MS, M/Z (ESI): 490.2 $[M+H]^+$
$^1H$ NMR (400 MHz, DMSO-d6) δ 7.75 (s, 1H), 7.20 (s, 1H), 7.12 (d, 1H), 7.01 (s, 1H), 6.93 (d, 1H), 6.90 (s, 1H) , 6.44 (s, 1H) ,5.15 (s, 2H), 3.59 (t, 2H), 2.58 (d, 2H) ,2.02 (s, 3H), 1.82-1.79(m, 1H), 1.37-1.31(m, 2H), 1.25-1.19(m, 2H) , 0.90 (d, 6H) , 0.83 (t, 3H)

Example 3: Preparation of target compound I-3

Butyl ((3-(4-((1H-imidazol-1-yl)methyl)-2-cyanophenyl)-5-isobutylthiophen-2-yl)sulfonyl)carbamate

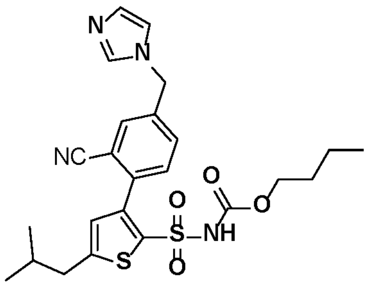

I-3

The synthetic scheme of the target compound 1-3 is as follows:

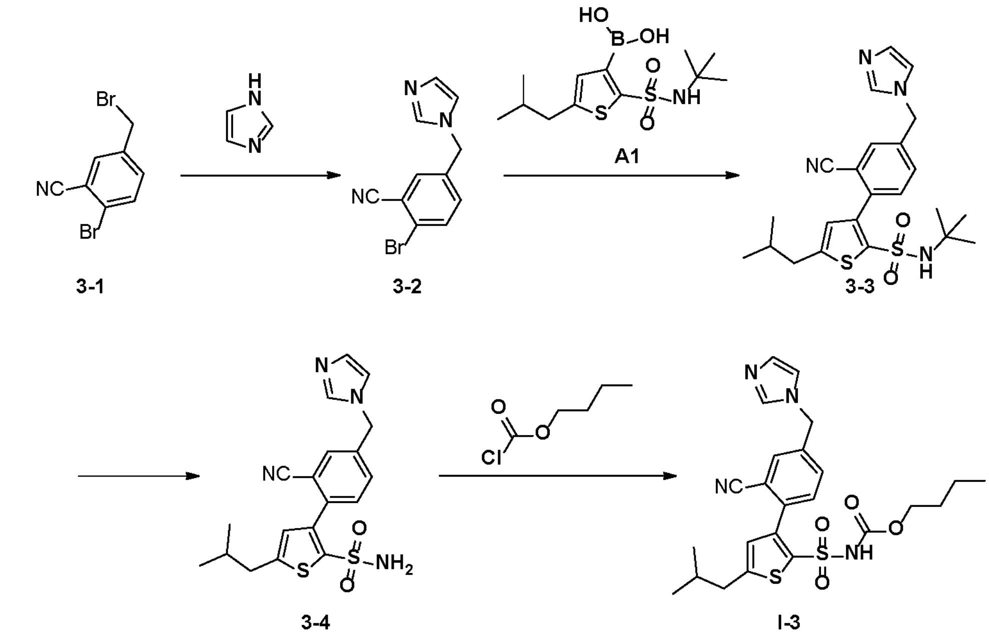

3-1 3-2 A1 3-3

3-4 I-3

Step 1: Synthesis of 5-((1H-imidazol-1-yl)methyl)-2-bromobenzonitrile

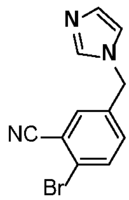

3-2

In a 50 mL single-necked flask, imidazole (136 mg, 2.00 mmol) was added and dissolved in DMF (5 mL). Then, sodium hydrogen (183 mg, 60%, 4.55 mmol) was slowly added to the reaction solution in batches under $N_2$ atmosphere. After stirring for half an hour, a solution of 2-bromo-5-(bromomethyl)benzonitrile (500 mg, 1.82 mmol) in DMF (2.5 mL) was slowly added to the reaction solution. The reaction system was stirred at room temperature for 1 hour. The reaction was monitored with TLC (DCM: MeOH (V/V)=10:1), until TLC indicated complete rection of raw materials and generation of a new spot of the target compound. The reaction solution was slowly poured into an ice saturated solution of $NH_4Cl$ (aq. 10 mL), and was extracted with ethyl acetate (5 mL×3). The organic phase was washed with water (10 mL × 5) and then with saturated brine (10 mL). The resulting organic phase was dried over anhydrous sodium sulfate and then filtered. The filtrated stock was spin-dried and mixed with silica gel, and separated and purified by a silica gel column (dichloromethane: methanol (V/V)=(50:1) to (10:1)) to obtain 5-((1H-imidazol-1-yl)methyl)-2-bromobenzonitrile (350.0 mg, yield 73.4%). LC-MS, M/Z (ESI): 262.2[M+H]$^+$

Step 2: Synthesis of 3-(4-((1H-imidazol-1-yl)methyl)-2-cyanophenyl)-N-(tert-butyl)-5-isobutylthiophene-2-sulfonamide

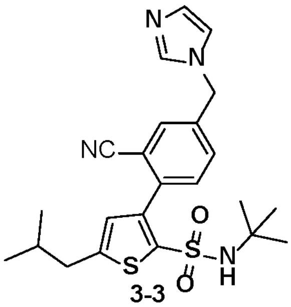

3-3

In a 50 mL single-necked flask, 5-((1H-imidazol-1-yl)methyl)-2-bromobenzonitrile (300 mg, 1.14 mmol) and (2-(N-(tert-butyl)sulfamoyl)-5-isobutylthiophen-3-yl)boric acid (365 mg, 1.14 mmol) were added and dissolved in toluene (6 mL). Then, sodium carbonate (243 mg, 2.29 mmol) was added, tetrakis(triphenylphosphine)palladium (133 mg, 0.11 mmol) was added under $N_2$ atmosphere, and the reaction system was heated to 120°C and stirred for 24 hours. The reaction was monitored with TLC (PE: EA (V/V)=1:2), until TLC indicated generation of a new spot of the target compound. The reaction solution was filtered. The filtrated stock was spin-dried and mixed with silica gel, and separated and purified by a silica gel column (petroleum ether: ethyl acetate (V/V)=(10:1) to (0:1) to obtain 3-(4-((1H-imidazol-1-yl)methyl)-2-cyanophenyl)-N-(tert-butyl)-5-isobutylthiophene-2-sulfonamide (120.0 mg, yield 23.0%).
LC-MS, M/Z (ESI): 457.3 $[M+H]^+$

Step 3: Synthesis of 3-(4-((1H-imidazol-1-yl)methyl)-2-cyanophenyl)-5-isobutylthiophene-2-sulfonamide

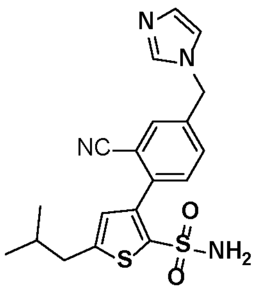

3-4

In a 50 mL single-necked flask, 3-(4-((1H-imidazol-1-yl)methyl)-2-cyanophenyl)-N-(tert-butyl)-5-isobutylthiophene-2-sulfonamide (120 mg, 0.26 mmol) was added and dissolved in trifluoroacetic acid (2 mL), and the reaction solution was stirred at room temperature for 24 hours. In this case, the solution turned dark brown. The reaction was monitored with TLC (PE: EA (V/V)=1:2), until TLC indicated complete reaction of raw materials. The reaction solution was directly spin-dried to obtain a crude product of 3-(4-((1H-imidazol-1-yl)methyl)-2-cyanophenyl)-5-isobutylthiophene-2-sulfonamide (100 mg), which was directly used in the next step.
LC-MS, M/Z (ESI): 401.3$[M+H]^+$

Step 4: Synthesis of butyl ((3-(4-((1H-imidazol-1-yl)methyl)-2-cyanophenyl)-5-isobutylthiophen-2-yl)sulfonyl)carbamate

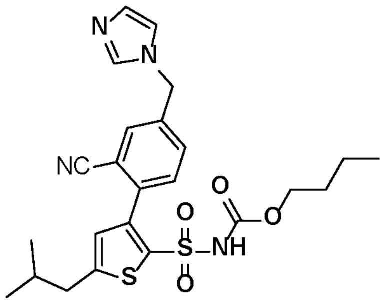

I-3

In a 50 mL single-necked flask, the crude product of 3-(4-((1H-imidazol-1-yl)methyl)-2-cyanophenyl)-5-isobutylthiophene-2-sulfonamide (100 mg) was added and dissolved in dichloromethane (2 mL). Then, triethylamine (0.1 mL) was added to the reaction solution. After stirring for half an hour, butyl chloroformate (100 mg, 0.73 mmol) was added to the reaction solution. The reaction system was stirred at room temperature for 16 hours. The reaction was monitored with TLC (DCM: MeOH (V/V)=10:1), until TLC indicated complete rection of raw materials and generation of one new spot of the target compound. The reaction solution was slowly poured into a saturated solution of $NH_4Cl$ (aq. 10 mL), and extracted with ethyl acetate (5 mL×3). The resulting organic phase was dried over anhydrous sodium sulfate and then filtered. The filtrated stock was spin-dried for preparative separation and purification (alkaline column: Phenomenex Titank C18 10μ Bulk 30mm*40cm, A ($H_2O$)/ B (ACN)=100%/0~40%/60%) to obtain butyl ((3-(4-((1H-imidazol-1-yl)methyl)-2-cyanophenyl)-5-isobutylthiophen-2-yl)sulfonyl)carbamate (9.0 mg, yield 7.2%).

LC-MS, M/Z (ESI): 499.1 $[M-H]^+$
$^1H$ NMR (400 MHz, DMSO-d6) δ 8.13 (s, 1H), 7.77 (d, 1H), 7.67 (d, 1H), 7.58 (dd, 1H), 7.39 (s, 1H), 7.10 (s, 1H), 6.81 (s, 1H), 5.33 (s,2H), 3.76 (t, 2H), 3.08 (dd, 2H), 2.66 (d, 1H), 1.94 - 1.73 (m, 1H), 1.48 - 1.30 (m, 2H), 1.19 (dt, 4H), 0.92 (d, 3H), 0.83 (t, 3H). **Example 4:** Preparation of target compound I-**4**

Butyl ((3-(4-((1H-imidazol-1-yl)methyl)-3-cyanophenyl)-5-isobutylthiophen-2-yl)sulfonyl)carbamate

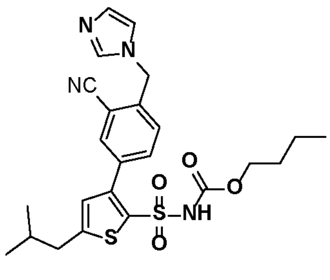

I-4

The synthetic scheme of the target compound I-**4** is as follows:

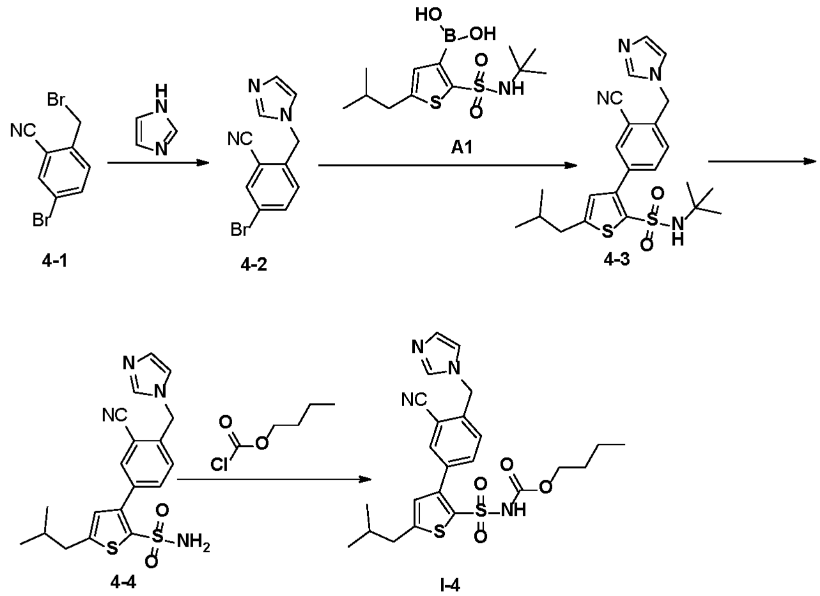
4-1 4-2 A1 4-3 4-4 I-4

Step 1: Synthesis of 2-((1H-imidazol-1-yl)methyl)-5-bromobenzonitrile

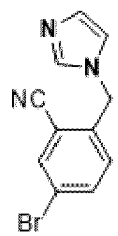
4-2

In a 50 mL single-necked flask, imidazole (136 mg, 2.00 mmol) was added and dissolved in DMF (5 mL). Then, sodium hydride (183 mg, 60%, 4.55 mmol) was slowly added to the reaction solution in batches under $N_2$ atmosphere. After stirring for 0.5 hour, 5-bromo-2-(bromomethyl)benzonitrile (500 mg, 1.82 mmol) dissolved in DMF (2.5 mL) was slowly added to the reaction solution. The reaction system was stirred at room temperature for 1 hour. Thereafter, the reaction was monitored with TLC (DCM: MeOH (V/V)=10:1), until TLC indicated complete rection of raw materials and generation of a new spot of the target compound. The reaction solution was slowly poured into an ice saturated solution of $NH_4Cl$ (aq. 10 mL) and extracted with ethyl acetate (5 mL×3). The organic phase was washed with water (10 mL × 5) and then with saturated brine (10 mL). The resulting organic phase was dried over anhydrous sodium sulfate and then filtered. The filtrated stock was spin-dried and mixed with silica gel, and separated and purified by a silica gel column (dichloromethane: methanol (V/V)=(50:1) to (10:1)) to obtain 2-((1H-imidazol-1-yl)methyl)-5-bromobenzonitrile (350 mg, yield 73.4%).
LC-MS, M/Z (ESI): 264.1 $[M+H]^+$

Step 2: Synthesis of 3-(4-((1H-imidazol-1-yl)methyl)-3-cyanophenyl)-N-(tert-butyl)-5-isobutylthiophene-2-sulfonamide

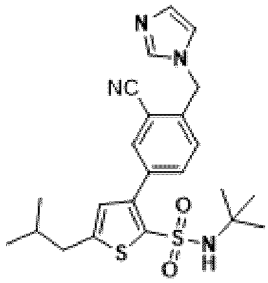

4-3

In a 50 mL single-necked flask, 2-((1H-imidazol-1-yl)methyl)-5-bromobenzonitrile (300 mg, 1.14 mmol) and (2-(N-(tert-butyl)sulfamoyl)-5-isobutylthiophen-3-yl)boric acid (365 mg, 1.14 mmol) were added and dissolved in toluene (6 mL). Then, sodium carbonate (243 mg, 2.29 mmol) was added, and tetrakis(triphenylphosphine)palladium (133 mg, 0.11 mmol) was added under $N_2$ atmosphere. The reaction system was heated to 120°C and stirred for 24 hours. The reaction was monitored with TLC (PE: EA (V/V)=1:2), until TLC indicated complete reaction of raw materials and generation of a new spot of the target compound. The reaction solution was filtered. The filtrated stock was spin-dried and mixed with silica gel, and separated and purified by a silica gel column (petroleum ether: ethyl acetate (V/V)=(10:1) to (0:1) to obtain 3-(4-((1H-imidazol-1-yl)methyl)-3-cyanophenyl)-N-(tert-butyl)-5-isobutylthiophene-2-sulfonamide (120 mg, yield 23.0%).
LC-MS, M/Z (ESI): 457.3 $[M+H]^+$

Step 3: Synthesis of 3-(4-((1H-imidazol-1-yl)methyl)-3-cyanophenyl)-5-isobutylthiophene-2-sulfonamide

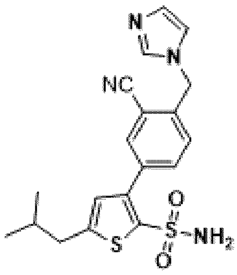

4-4

In a 50 mL single-necked flask, 3-(4-((1H-imidazol-1-yl)methyl)-3-cyanophenyl)-N-(tert-butyl)-5-isobutylthiophene-2-sulfonamide (120 mg, 0.26 mmol) was added and dissolved in trifluoroacetic acid (2 mL), and the reaction solution was stirred at room temperature for 24 hours. At this time, the solution turned dark brown. The reaction was monitored with TLC (PE: EA (V/V)=1:2), until TLC indicated complete reaction of raw materials. The reaction solution was directly spin-dried to obtain a crude product of 3-(4-((1H-imidazol-1-yl)methyl)-3-cyanophenyl)-5-isobutylthiophene-2-sulfonamide (100 mg), which was directly used in the next step.
LC-MS, M/Z (ESI): 401.3 $[M+H]^+$

Step 4: Synthesis of butyl ((3-(4-((1H-imidazol-1-yl)methyl)-3-cyanophenyl)-5-isobutylthiophen-2-yl)sulfonyl)carbamate

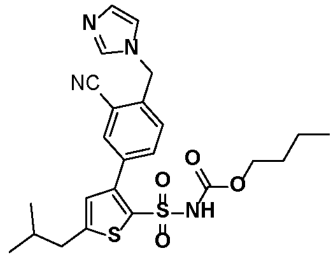

I-4

In a 50 mL single-necked flask, the crude product of 3-(4-((1H-imidazol-1-yl)methyl)-3-cyanophenyl)-5-isobutylthiophene-2-sulfonamide (100 mg) was added and dissolved in DCM (2 mL). Then, pyridine (0.2 mL) was added to the reaction solution, and the mixture was stirred for 0.5 hour. Then, butyl chloroformate (100 mg, 0.73 mmol) was added to the reaction solution, and the reaction system was stirred at room temperature for 16 hours. The reaction was monitored with TLC (DCM: MeOH (V/V)=10:1), until TLC indicated complete reaction of raw materials and generation of a new spot of the target compound. The reaction solution was slowly poured into a saturated solution of $NH_4Cl$ (aq. 10 mL), and extracted with ethyl acetate (5 mL×3). The organic phase was washed with saturated brine (10 mL). The resulting organic phase was dried over anhydrous sodium sulfate and then filtered. The filtrated stock was spin-dried for preparative separation and purification (preparative purification: alkaline column: Phenomenex Titank C18 10μ Bulk 30mm*40cm, A ($H_2O$)/ B (ACN) =100%/0~40%/60%) to obtain butyl ((3-(4-((1H-imidazol-1-yl)methyl)-3-cyanophenyl)-5-isobutylthiophen-2-yl)sulfonyl) carbamate (12.0 mg, yield 9.6%).

LC-MS, M/Z (ESI): 500.9[M+H]$^+$

$^1$H NMR (400 MHz, DMSO-d6) δ 8.41 (s, 1H), 8.13 (d, 1H), 7.92 (dd, 1H), 7.46 (s, 1H), 7.33 (d, 2H), 6.99 (s, 1H), 5.59 (s, 2H), 3.85 (t, 2H), 2.69 (d, 2H), 1.98 - 1.73 (m, 1H), 1.39 (dd, 2H), 1.20 (dd, 2H), 0.93 (t, 6H), 0.83 (t, 3H)

**Example 5**: Preparation of target compound **I-5**

Butyl ((3-(5-((1H-imidazol-1-yl)methyl)pyridin-2-yl)-5-isobutylthiophen-2-yl)sulfonyl)carbamate

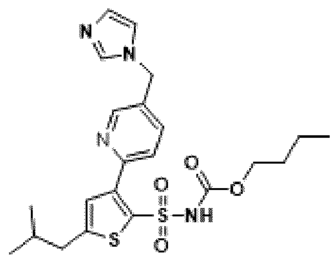

I-5

The synthetic scheme of the target compound 1-5 is as follows:

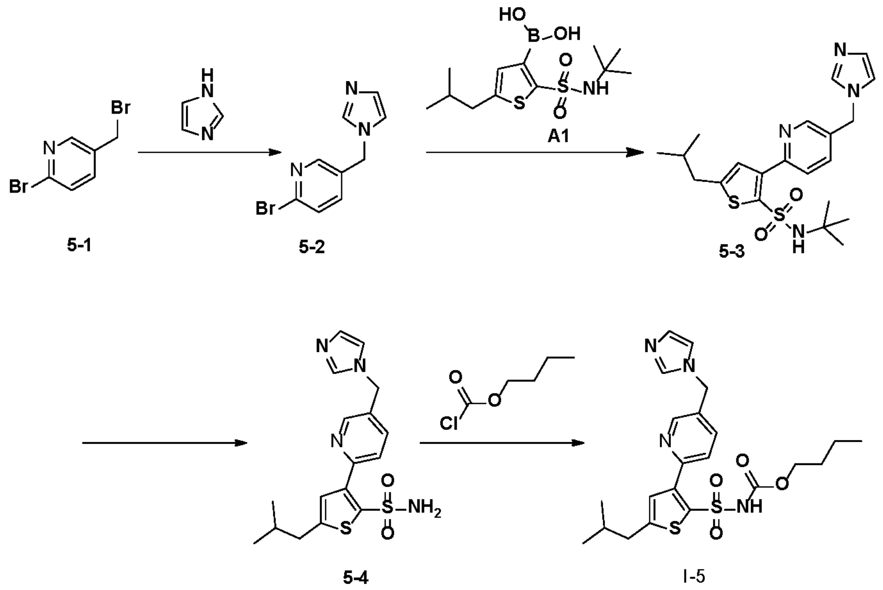

5-1 5-2 A1 5-3

5-4 I-5

Step 1: Synthesis of 5-((1H-imidazol-1-yl)methyl)-2-bromopyridine

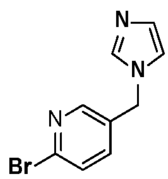

5-2

Compounds 2-bromo-5-(bromomethyl)pyridine (1.0 g, 3.98 mmol), imidazole (0.56 g, 8.38 mmol), and potassium carbonate (0.88 g, 6.38 mmol) were added to DMF (10 ml) at room temperature, and the mixture was stirred at 25°C under nitrogen protection for 12 hours. After the reaction was complete, the reaction system was diluted by adding water (30 mL), extracted with ethyl acetate (20 mL×3), and separated. The organic phases were combined, washed with water (20 mL×5), dried over anhydrous sodium sulfate, filtered, and concentrated. The residue was separated and purified by chromatographic column (dichloromethane: methanol (V/V)=10:1) to obtain 5-((1H-imidazol-1-yl)methyl)-2-bromopyridine (0.7 g, yield 73.12%).
LC-MS, M/Z (ESI): 238.0 $[M+H]^+$

Step 2: Synthesis of 3-(5-((1H-imidazol-1-yl)methyl)pyridin-2-yl)-N-(tert-butyl)-5-isobutylthiophene-2-sulfonamide

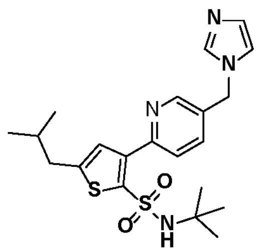

5-3

Compounds 5-((1H-imidazol-1-yl)methyl)-2-bromopyridine (0.3 g, 1.26 mmol), (2-(N-(tert-butyl)sulfamoyl)-5-isobutylthiophen-3-yl)boric acid (0.44 g, 1.38 mmol), potassium carbonate (0.522 g, 3.78 mmol), and [1,1'-bis(diphenylphosphino)ferrocene]palladium dichloride (0.092 g, 0.126 mmol) were dissolved in dioxane (5 ml), replaced with nitrogen,

and heated to 90°C and stirred for 10 hours. After the reaction was completed, the reaction system was diluted by adding water (10 mL), extracted with ethyl acetate (10 mL×3), and separated. The organic phases were combined, dried over anhydrous sodium sulfate, filtered, and concentrated. The residue was separated and purified by chromatographic column (dichloromethane: methanol (V/V)=10:1) to obtain 3-(5-((1H-imidazol-1-yl)methyl)pyridin-2-yl)-N-(tert-butyl)-5-isobutylthiophene-2-sulfonamide (0.1 g, yield 18.35%).
LC-MS, M/Z (ESI): 433.2 $[M+H]^+$

Step 3: 3-(5-((1H-imidazol-1-yl)methyl)pyridin-2-yl)-5-isobutylthiophene-2-sulfonamide

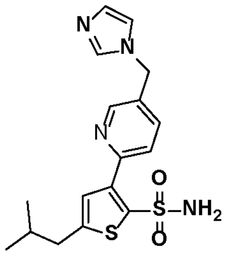

5-4

The raw material, 3-(5-((1H-imidazol-1-yl)methyl)pyridin-2-yl)-N-(tert-butyl)-5-isobutylthiophene-2-sulfonamide (0.1 g, 0.231 mmol), was dissolved in dichloromethane (1 mL), and 1.0 mL of trifluoroacetic acid was added thereto. The mixture was stirred at room temperature for 12 hours. The reaction solution was diluted by adding water (5 mL), and pH of the reaction solution was adjusted to range from 9 to 10 with a 2M sodium hydroxide aqueous solution. The reaction system was extracted with dichloromethane (5 mL×3), and separated. The organic phases were combined, dried over anhydrous sodium sulfate, filtered, and concentrated. The residue was separated and purified by a thin-layer chromatographic silica gel plate (dichloromethane: methanol (V/V)=10:1) to obtain 3-(5-((1H-imidazol-1-yl)methyl)pyridin-2-yl)-5-isobutylthiophene-2-sulfonamide (0.04 g, yield 46%).
LC-MS, M/Z (ESI): 377.1 $[M+H]^+$

Step 4: Synthesis of butyl ((3-(5-((1H-imidazol-1-yl)methyl)pyridin-2-yl)-5-isobutylthiophen-2-yl)sulfonyl)carbamate

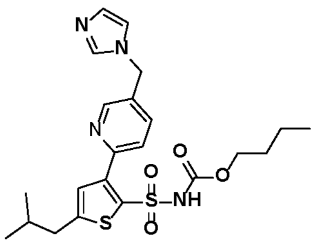

I-5

Compounds 3-(5-((1H-imidazol-1-yl)methyl)pyridin-2-yl)-5-isobutylthiophene-2-sulfonamide (0.04 g, 0.106 mmol), triethylamine (0.021 g, 0.212 mmol), and 4-dimethylaminopyridine (7 mg, 0.053 mmol) were added to DMF (0.5 mL) and dichloromethane (0.5 mL) at room temperature. Finally, butyl chloroformate (0.029 g, 0.212 mmol) was added dropwise to the reaction system, and the reaction system was stirred for 12 hours. The reaction system was diluted by adding water (5 mL), extracted with ethyl acetate (5 mL×3), and separated. The organic phases were combined, dried over anhydrous sodium sulfate, filtered, and concentrated. The residue was subjected to a preparative separation and purification by means of high performance liquid chromatography (preparative purification: chromatographic column: Phenomenex Luna C18 150×25mm×10μm; mobile phase: A=water + 0.01% by volume of trifluoroacetic acid (99%), B=acetonitrile; gradient 35% to 65% B, 10 minutes) to obtain butyl ((3-(5-((1H-imidazol-1-yl)methyl)pyridin-2-yl)-5-isobutylthiophen-2-yl)sulfonyl)carbamate (7 mg, yield 13.82%).

LC-MS, M/Z (ESI): 477.2 $[M+H]^+$
$^1H$ NMR (400 MHz, $CDCl_3$) δ 8.57 (s, 1H), 7.67 - 7.59 (m, 2H), 7.54 - 7.48 (m, 1H), 7.16 (s, 1H), 7.05 (d, 1H), 6.94 (s,

1H), 5.23 (s, 2H), 4.15 (t, 2H), 2.73 (d, 2H), 1.96 (dt, 1H), 1.61 (dd, 2H), 1.34 (dd, 2H), 1.04 - 0.93 (m, 6H), 0.90 (t, 3H).

**Example 6:** Preparation of target compound **I-6**

Butyl ((3-(6-((1H-imidazol-1-yl)methyl)pyridin-3-yl)-5-isobutylthiophen-2-yl)sulfonyl)carbamate

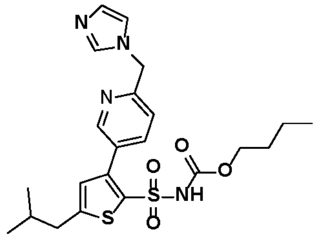

**I-6**

The synthetic scheme of the target compound **I-6** was as follows:

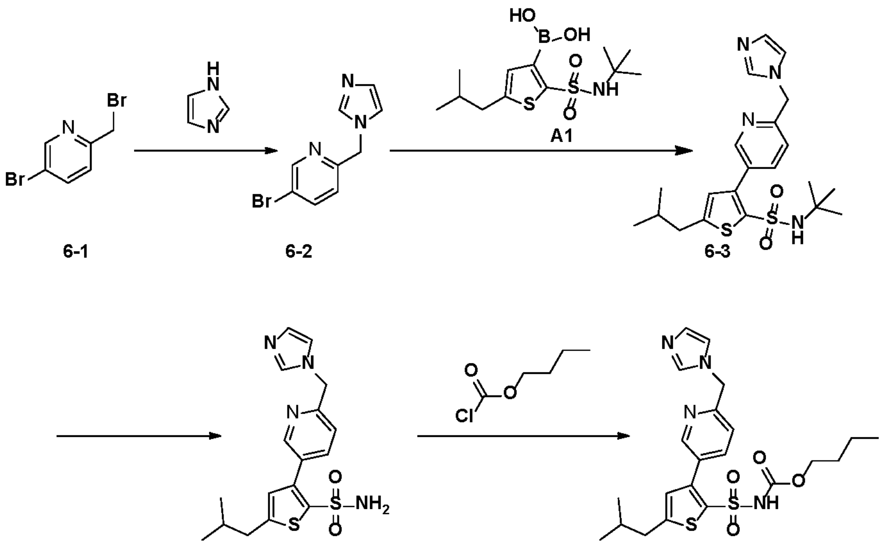

**A1**

**6-1** **6-2** **6-3**

**6-4** **I-6**

Step 1: 2-((1H-imidazol-1-yl)methyl)-5-bromopyridine

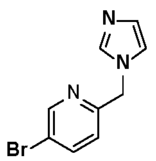

**6-1**

Compounds, 5-bromo-2-(bromomethyl)pyridine (1.0 g, 3.98 mmol), imidazole (0.56 g, 8.38 mmol), and potassium carbonate (0.88 g, 6.38 mmol), were added to DMF (10.0 ml) at room temperature, and the mixture was stirred at 25°C under nitrogen protection for 12 hours. After the reaction was complete, the reaction system was diluted by adding water (30 mL), extracted with ethyl acetate (20 mL×3), washed with water (20 mL×5), and separated. The organic phases were combined, dried over anhydrous sodium sulfate, filtered, and concentrated. The residue was separated and purified by chromatographic column (dichloromethane: methanol (V/V)=10:1)) to obtain 2-((1H-imidazol-1-yl)methyl)-5-bromopyridine (0.7 g, yield 73.12%).

LC-MS, M/Z (ESI): 238.0 [M+H]+

Step 2: Synthesis of 3-(6-((1H-imidazol-1-yl)methyl)pyridin-3-yl)-N-(tert-butyl)-5-isobutylthiophene-2-sulfonamide

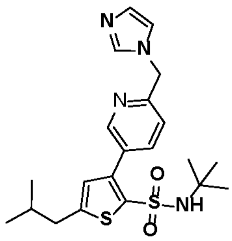

6-3

Compounds, 2-((1H-imidazol-1-yl)methyl)-5-bromopyridine (0.3 g, 1.26 mmol), (2-(N-(tert-butyl)sulfamoyl)-5-isobutylthiophen-3-yl)boric acid (0.44 g, 1.38 mmol), potassium carbonate (0.522 g, 3.78 mmol), and [1,1'-bis(diphenylphosphino)ferrocene]palladium dichloride (0.092 g, 0.126 mmol), were dissolved in dioxane (5 mL), replaced with nitrogen, and heated to 90°C and stirred for 10 hours. After the reaction was completed, the reaction system was diluted by adding water (10 mL), extracted with ethyl acetate (10 mL×3), and separated. The organic phases were combined, dried over anhydrous sodium sulfate, filtered, and concentrated. The residue was separated and purified by chromatographic column (dichloromethane: methanol (V/V)=10:1) to obtain 3-(6-((1H-imidazol-1-yl)methyl)pyridin-3-yl)-N-(tert-butyl)-5-isobutylthiophene-2-sulfonamide (0.3 g, yield 60.2%).
LC-MS, M/Z (ESI): 433.2 [M+H]+

Step 3: Synthesis of 3-(6-((1H-imidazol-1-yl)methyl)pyridin-3-yl)-5-isobutylthiophene-2-sulfonamide

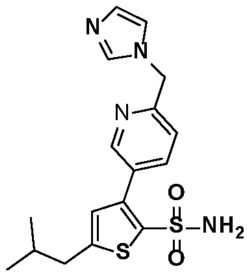

6-4

3-(6-((1H-imidazol-1-yl)methyl)pyridin-3-yl)-N-(tert-butyl)-5-isobutylthiophene-2-sulfonamide (0.3 g, 0.693 mmol) was dissolved in dichloromethane (3 ml), 3 mL of trifluoroacetic acid was added thereto, and the mixture was stirred at room temperature for 12 hours. The reaction system was diluted by adding water (5 mL), and pH of the reaction system was adjusted to range from 9 to 10. The reaction system was extracted with dichloromethane (5 mL×3) and separated. The organic phases were combined, dried over anhydrous sodium sulfate, filtered, and concentrated. The residue was separated and purified by a thin-layer chromatographic silica gel plate (dichloromethane: methanol (V/V) =10:1) to obtain 3-(6-((1H-imidazol-1-yl)methyl)pyridin-3-yl)-5-isobutylthiophene-2-sulfonamide (0.18 g, yield 69.2%).
LC-MS, M/Z (ESI): 377.1[M+H]+

Step 4: Synthesis of butyl ((3-(6-((1H-imidazol-1-yl)methyl)pyridin-3-yl)-5-isobutylthiophen-2-yl)sulfonyl)carbamate

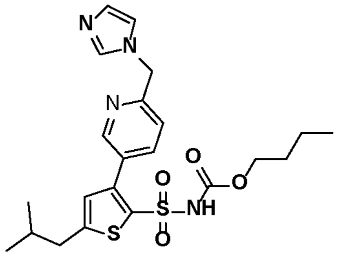

**I-6**

Compounds, 3-(6-((1H-imidazol-1-yl)methyl)pyridin-3-yl)-5-isobutylthiophene-2-sulfonamide (0.18 g, 0.477 mmol), triethylamine (0.095 g, 0.951 mmol), and 4-dimethylaminopyridine (29.3 mg, 0.239 mmol), were added to DMF (2.0 mL) and dichloromethane (2.0 mL) at room temperature. Finally, butyl chloroformate (0.131 g, 0.954 mmol) was added dropwise to the reaction system. The mixture was stirred for 12 hours. The reaction system was diluted by adding water (5 mL), extracted with ethyl acetate (5 mL×3), and separated. The organic phases were combined, dried over anhydrous sodium sulfate, filtered, and concentrated. The residue was subjected to a preparative separation and purification by means of high performance liquid chromatography (preparative purification: chromatographic column: Phenomenex Luna C18 150×25mm×10μm; mobile phase: A=water + 0.01% by volume of trifluoroacetic acid (99%), B=acetonitrile; gradient 35% to 65% B, 10 minutes) to obtain butyl ((3-(6-((1H-imidazol-1-yl)methyl)pyridin-3-yl)-5-isobutylthiophen-2-yl)sulfonyl)carbamate (50 mg, yield 22.2%).

LC-MS, M/Z (ESI): 477.2 $[M+H]^+$
$^1H$ NMR (400 MHz, $CDCl_3$) δ 8.77 (s, 1H), 7.88 (dd, 1H), 7.57 (s, 1H), 6.99 - 6.85 (m, 3H), 6.68 (d, 1H), 5.16 (s, 2H), 3.70 (t, 2H), 2.59 (d, 2H), 1.84 (dt, 1H), 1.32 (dd, 2H), 1.13 (dd, 2H), 0.95 - 0.86 (m, 6H), 0.75 (t, 3H)

**Example 7**: Preparation of target compound **I-7**

Butyl ((3-(4-(1-(1H-imidazol-1-yl)ethyl)phenyl)-5-isobutylthiophen-2-yl)sulfonyl)carbamate

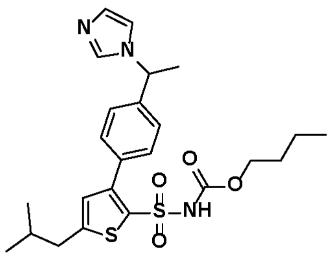

I-7

The synthetic scheme of the target compound **I-7** was as follows:

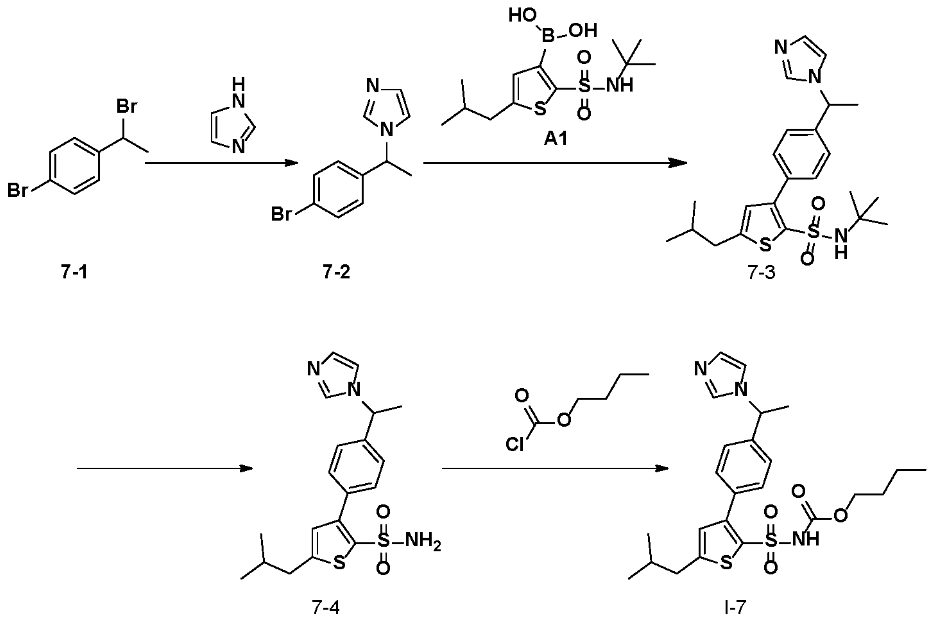


7-1 7-2 7-3 7-4 I-7

Step 1: Synthesis of 1-(1-(4-bromophenyl)ethyl)-1H-imidazole

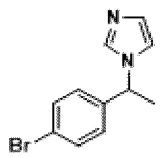

7-2

Compounds, 1-bromo-4-(1-bromoethyl)benzene (1.0 g, 3.82 mmol), imidazole (0.56 g, 8.38 mmol), and potassium carbonate (0.88 g, 6.38 mmol), were added to DMF (10.0 mL) at room temperature, and the mixture was stirred at 25°C under nitrogen protection for 12 hours. After the reaction was complete, the reaction system was diluted by adding water (30 mL), extracted with ethyl acetate (20 mL×3), washed with water (20 mL×5), and separated. The organic phases were combined, dried over anhydrous sodium sulfate, filtered, and concentrated. The residue was separated and purified by chromatographic column (dichloromethane: methanol (V/V)=10:1) to obtain 1-(1-(4-bromophenyl)ethyl)-1H-imidazole (0.7 g, yield 73.68%).
LC-MS, M/Z (ESI): 251.0 $[M+H]^+$

Step 2: Synthesis of 3-(4-(1-(1H-imidazol-1-yl)ethyl)phenyl)-N-(tert-butyl)-5-isobutylthiophene-2-sulfonamide

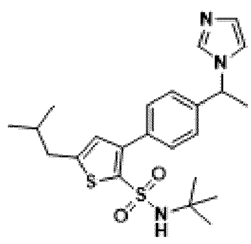

7-3

Compounds, 1-(1-(4-bromophenyl)ethyl)-1H-imidazole (0.3 g, 1.26 mmol), (2-(N-(tert-butyl)sulfamoyl)-5-isobutylthiophen-3-yl)boric acid (0.44 g, 1.38 mmol), potassium carbonate (0.522 g, 3.78 mmol), and [1,1'-bis(diphenylphosphino)ferrocene]palladium dichloride (0.092 g, 0.126 mmol), were dissolved in dioxane (5 ml), replaced with nitrogen,

and heated to 90°C and stirred for 10 hours. After the reaction was completed, the reaction system was diluted by adding water (10 mL), extracted with ethyl acetate (10 mL×3), and separated. The organic phases were combined, dried over anhydrous sodium sulfate, filtered, and concentrated. The residue was separated and purified by chromatographic column (dichloromethane: methanol (V/V)=10:1) to obtain 3-(4-(1-(1H-imidazol-1-yl)ethyl)phenyl)-N-(tert-butyl)-5-isobutylthiophene-2-sulfonamide (0.4 g, yield 75.41%).
LC-MS, M/Z (ESI): 446.1 $[M+H]^+$

Step 3: Synthesis of 3-(4-(1-(1H-imidazol-1-yl)ethyl)phenyl)-5-isobutylthiophene-2-sulfonamide

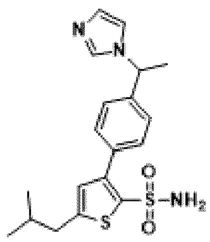

7-4

The raw material, 3-(4-(1-(1H-imidazol-1-yl)ethyl)phenyl)-N-(tert-butyl)-5-isobutylthiophene-2-sulfonamide (0.3 g, 0.693 mmol), was dissolved in dichloromethane (3 ml), and 3 mL of trifluoroacetic acid was added thereto. The mixture was stirred at room temperature for 12 hours. The reaction system was diluted by adding water (5 mL), and pH of the reaction system was adjusted to range from 9 to 10. The reaction system was extracted with dichloromethane (5 mL×3), and separated. The organic phases were combined, dried over anhydrous sodium sulfate, filtered, and concentrated. The residue was separated and purified by a thin-layer chromatographic silica gel plate (dichloromethane: methanol (V/V) =10:1) to obtain 3-(4-(1-(1H-imidazol-1-yl)ethyl)phenyl)-5-isobutylthiophene-2-sulfonamide (0.21 g, yield 80.71%).
LC-MS, M/Z (ESI): 390.1 $[M+H]^+$

Step 4: Synthesis of butyl ((3-(4-(1-(1H-imidazol-1-yl)ethyl)phenyl)-5-isobutylthiophen-2-yl)sulfonyl)carbamate

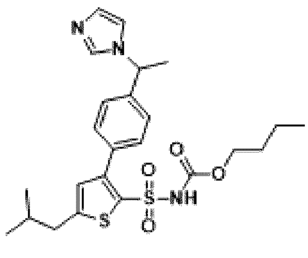

I-7

3-(4-(1-(1H-imidazol-1-yl)ethyl)phenyl)-5-isobutylthiophene-2-sulfonamide (0.21 g, 0.557 mmol), triethylamine (0.110 g, 1.103 mmol), and 4-dimethylaminopyridine (34 mg, 0.277 mmol) were added to DMF (2 mL) and dichloromethane (2 mL) at room temperature. Finally, butyl chloroformate (0.152 g, 1.103 mmol) was added dropwise to the reaction system, and the mixture was stirred for 12 hours. The reaction system was diluted by adding water (5 mL), extracted with ethyl acetate (5 mL×3), washed with water (5 mL×5), and separated. The organic phases were combined, dried over anhydrous sodium sulfate, filtered, and concentrated. The residue was subjected to a preparative separation and purification by means of high performance liquid chromatography (preparative purification: chromatographic column: Phenomenex Luna C18 150×25mm×10μm; mobile phase: A=water + 0.01% by volume of trifluoroacetic acid (99%), B=acetonitrile; gradient 35% to 65% B, 10 minutes) to obtain butyl ((3-(4-(1-(1H-imidazol-1-yl)ethyl)phenyl)-5-isobutylthiophen-2-yl)sulfonyl)carbamate (140 mg, yield 53.85%).

LC-MS, M/Z (ESI): 490.2 $[M+H]^+$
$^1H$ NMR (400 MHz, $CDCl_3$) δ 7.96 (s, 1H), 7.61 - 7.52 (m, 2H), 7.13 (d, 2H), 6.94 (s, 1H), 6.87 (s, 1H), 6.78 - 6.73 (m, 1H), 5.40 (q, 1H), 4.05 (t, 2H), 2.87 - 2.69 (m, 2H), 1.99 - 1.91 (m, 1H), 1.83 (t, 3H), 1.52 (tt, 2H), 1.31 - 1.24 (m, 2H), 1.04

- 0.93 (m, 6H), 0.88 (t, 3H). **Example 8:** Preparation of target compound **I-8**

Butyl ((3-(4-((1H-imidazol-1-yl)methyl)phenyl)-5-isobutyl-4-methylthiophen-2-yl)sulfonyl)carbamate

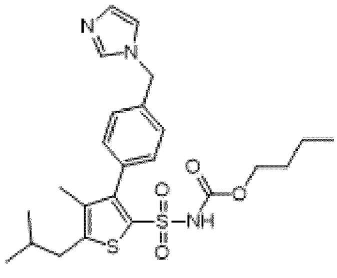

I-8

The synthetic scheme of the target compound 1-8 was as follows:

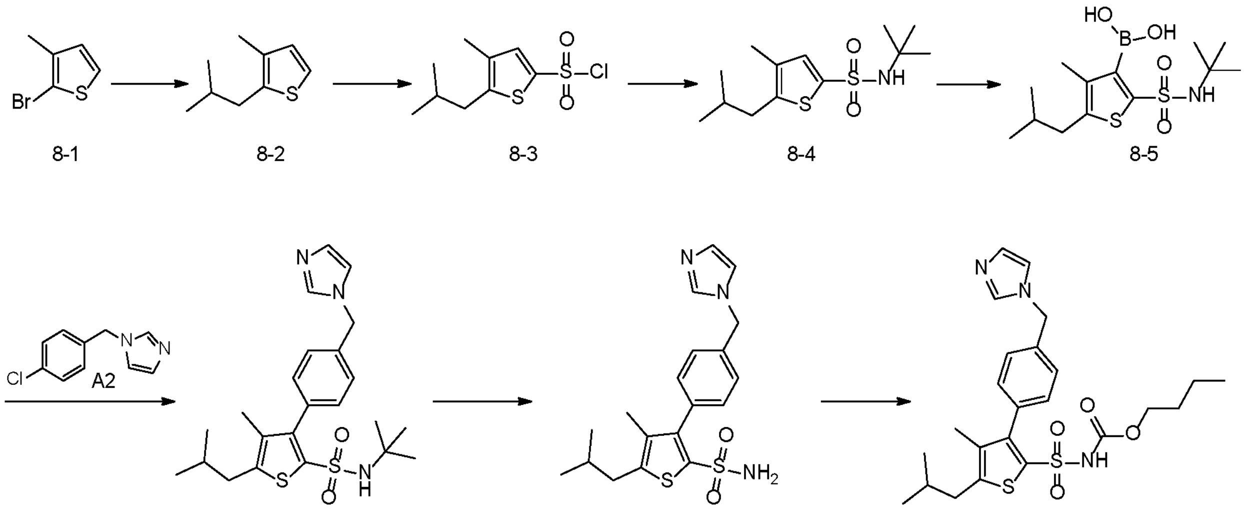


8-1 8-2 8-3 8-4 8-5

8-6 8-7 I-8

Step 1: Synthesis of 2-isobutyl-3-methylthiophene

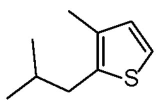

8-2

Zinc chloride (13.8 g, 101 mmol) was dissolved in tetrahydrofuran (100 mL). Isobutylmagnesium bromide (2.00 M, 45.1 mL), 2-bromo-3-methylthiophene (10.0 g, 56.5 mmol), and di(tri-tert-butylphosphine)palladium (1.44 g, 2.82 mmol) were added, and replaced with nitrogen after addition. The mixture was slowly heated to 90°C and reacted for 1 hour. The reaction solution was poured into water (200 mL), and extracted with ethyl acetate (200 mL) for three times. The organic phase was washed with saturated brine (50 mL), dried over anhydrous sodium sulfate, filtered, and concentrated to obtain 2-isobutyl-3-methylthiophene (6.00 g, yield 65.3%).

Step 2: Synthesis of 5-isobutyl-4-methylthiophene-2-sulfonyl chloride

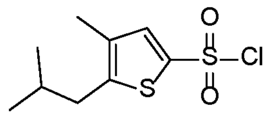

8-3

2-isobutyl-3-methylthiophene (6.00 g, 36.9 mmol) was dissolved in dichloromethane (30.0 mL), chlorosulfonic acid (13.6 g, 116 mmol) was added, and the mixture was stirred at room temperature for 1 hour. The reaction solution was concentrated to obtain a product 5-isobutyl-4-methylthiophene-2-sulfonyl chloride (9.80 g, crude product).

Step 3: Synthesis of N-(tert-butyl)-5-isobutyl-4-methylthiophene-2-sulfonamide

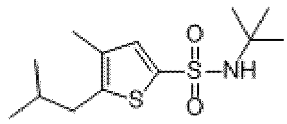

8-4

5-isobutyl-4-methylthiophene-2-sulfonyl chloride (9.80 g, 38.7 mmol) was dissolved in dichloromethane (40.0 mL), and N,N-diisopropylethylamine (10.0 g, 77.5 mmol) and 2-methylpropane-2-amine (3.40 g, 46.5 mmol) were added. The mixture was heated to 40°C and reacted for 1 hour. The reaction solution was poured into water (40.0 mL), and extracted with ethyl acetate (200 mL) for three times. The organic phase was washed with saturated brine (100 mL), dried over anhydrous sodium sulfate, filtered, and concentrated. The crude product was separated and purified by a silica gel column (petroleum ether: ethyl acetate (V/V)=(20:1) to (5:1), $R_f$=0.4) to obtain N-(tert-butyl)-5-isobutyl-4-methylthiophene-2-sulfonamide (1.50 g, yield 13.4%).
LC-MS, M/Z (ESI): 288.1 $[M-H]^+$.

Step 4: Synthesis of (2-(N-(tert-butyl)sulfamoyl)-5-isobutyl-4-methylthiophen-3-yl)boronic acid

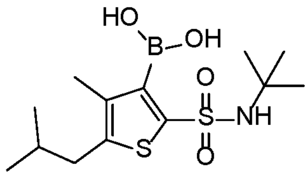

8-5

N-(tert-butyl)-5-isobutyl-4-methylthiophene-2-sulfonamide (900 mg, 3.34 mmol) was dissolved in tetrahydrofuran (20 mL). The reaction solution was cooled to -60°C in a dry ice ethanol bath. Under nitrogen protection, n-butyl lithium (2.50 M, 3.98 mL) was slowly added dropwise, and the mixture was stirred for 0.5 hour after addition. Then, triisopropyl borate (1.17 g, 6.22 mmol) was added, and the mixture reacted at -60°C for 1 hour. The reaction solution was poured into water (20 mL), and extracted with ethyl acetate (20 mL) for three times. The organic phase was washed with saturated brine (20 mL), dried over anhydrous sodium sulfate, filtered, and concentrated. The crude product was separated and purified by a silica gel column (petroleum ether: ethyl acetate (V/V)=(5:1) to (1:1), $R_f$=0.1) to obtain (2-(N-(tert-butyl) sulfamoyl)-5-isobutyl-4-methylthiophen-3-yl)boric acid (0.7 g, yield 62.8%).
LC-MS, M/Z (ESI): 332.1 $[M-H]^+$

Step 6: Synthesis of 3-(4-((1H-imidazol-1-yl)methyl)phenyl)-N-(tert-butyl)-5-isobutyl-4-methylthiophene-2-sulfonamide

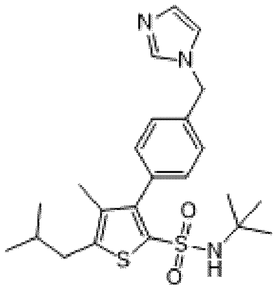

8-6

(2-(N-(tert-butyl)sulfamoyl)-5-isobutyl-4-methylthiophen-3-yl)boric acid (700 mg, 2.10 mmol) and 1-(4-chlorobenzyl)-1H-imidazole (610 mg, 3.15 mmol) were dissolved in tetrahydrofuran (10 mL). Potassium phosphate (1.34 g, 6.30 mmol) and methanesulfonato(2-dicyclohexylphosphino-2,6-dimethoxy-1,1-biphenyl)(2-amino-1, 1-biphenyl-2-yl)palladium (78 mg, 100 μmol) were added, and replaced with nitrogen for three times. The mixture was heated to 60°C under nitrogen protection and reacted for 6 hours. After the reaction solution was concentrated, the crude product was separated and purified by high performance liquid chromatography (column: Phenomenex luna C1875*30mm*3μm solvent: A=water + 0.05% by volume of formic acid (99.0%), B=acetonitrile; gradient: 20% to 50%, 7 minutes) to obtain 3-(4-((1H-imidazol-1-yl)methyl)phenyl)-N-(tert-butyl)-5-isobutyl-4-methylthiophene-2-sulfonamide (150 mg, yield 15.7%).
LC-MS, M/Z (ESI): 446.1 $[M+H]^+$

Step 7: Synthesis of 3-(4-((1H-imidazol-1-yl)methyl)phenyl)-5-isobutyl-4-methylthiophene-2-sulfonamide

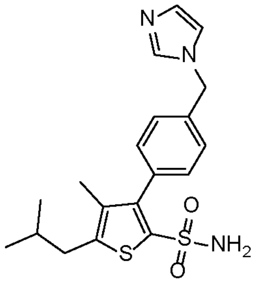

8-7

3-(4-((1H-imidazol-1-yl)methyl)phenyl)-N-(tert-butyl)-5-isobutyl-4-methylthiophene-2-sulfonamide (150 mg, 330 μmol) was added to dichloromethane (5 mL) and trifluoroacetic acid (5 mL), and the mixture was heated to 40°C and reacted for 6 hours. pH of the reaction solution was adjusted to 5 with saturated sodium bicarbonate aqueous solution, and then the reaction solution was concentrated to obtain 3-(4-((1H-imidazol-1-yl)methyl)phenyl)-5-isobutyl-4-methylthiophene-2-sulfonamide (200 mg, crude product).
LC-MS, M/Z (ESI): 388.1 $[M-H]^+$

Step 8: Synthesis of butyl ((3-(4-((1H-imidazol-1-yl)methyl)phenyl)-5-isobutyl-4-methylthiophen-2-yl)sulfonyl)carbamate

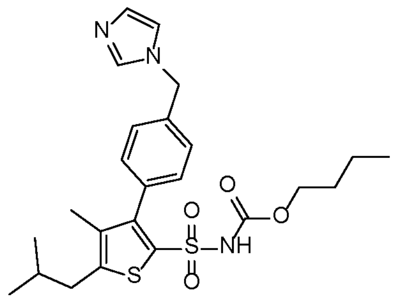

I-8

3-(4-((1H-imidazol-1-yl)methyl)phenyl)-5-isobutyl-4-methylthiophene-2-sulfonamide (200 mg, crude product) and N,N-diisopropylethylamine (129.1 mg, 0.99 mmol) were dissolved in dichloromethane (10 mL), and n-butyl chloroformate (67.32 mg, 0.5 mmol) was added. The mixture reacted at 25°C for 2 hours. The reaction solution was concentrated. The crude product was separated and purified by high performance liquid chromatography (column: Phenomenex luna C18 150*50mm*10μm; solvent: A=water + 0.05% by volume of formic acid (99.0%), B=acetonitrile; gradient: 22% to 52%, 10 minutes) to obtain a product butyl ((3-(4-((1H-imidazol-1-yl)methyl)phenyl)-5-isobutyl-4-methylthiophen-2-yl)sulfonyl)carbamate (**I-8**) (4.25 mg, 8.31 μmol).

LCMS, M/Z (ESI): 490.1 $[M+H]^+$

$^1$HNMR (400 MHz, $CDCl_3$) $\delta$ 7.54 (s, 1 H), 7.19-7.21 (m, 2H), 7.11 (d, 2H), 6.87 (s, 1H), 6.79 (s, 1H), 5.07 (s, 2H), 4.01 (t, 2H), 2.60 (d, 2H), 1.78 (s, 3H), 1.75-1.76 (m, 1H), 1.46-1.52 (m, 2H), 1.19-1.26 (m, 2H), 0.94 (d, 6H), 0.83 (t, 3H)

**Example 9:** Preparation of target compound **I-10**

Methyl (3-(2-cyano-4-((2-methyl-1H-imidazol-1-yl)methyl)phenyl)-5-isobutylthiophen-2-yl)sulfonylcarbamate

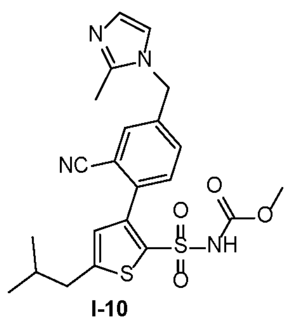

**I-10**

The synthetic scheme of the target compound 1-10 was as follows:

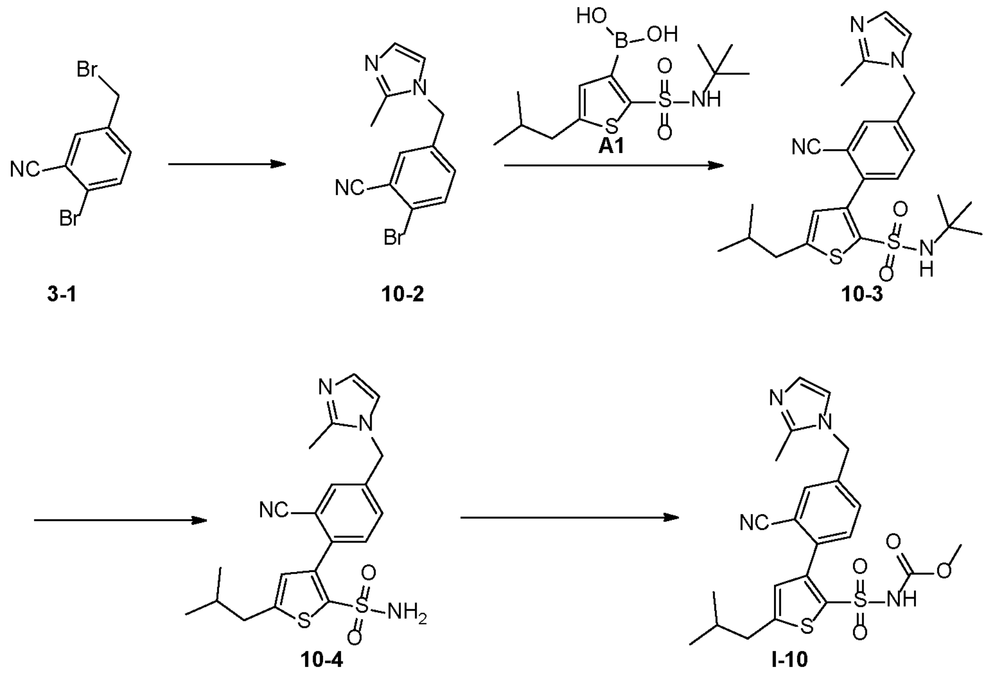

A1

3-1 10-2 10-3

10-4 I-10

Step 1: Synthesis of 2-bromo-5-((2-methyl-1H-imidazol-1-yl)methyl)benzonitrile

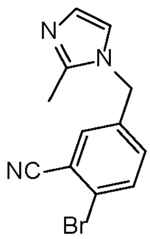

10-2

2-bromo-5-(bromomethyl)benzonitrile (1.00 g, 1.41 mmol) and 2-methyl-1H-imidazole (895 mg, 10.9 mmol) were dissolved in N,N-dimethylformamide (10 mL), and potassium carbonate (1.51 g, 10.9 mmol) was added. The mixture reacted at 50°C for 2 hours. The reaction solution was poured into water (50 mL), and extracted with ethyl acetate (100 mL) for three times. The organic phases were collected, dried over anhydrous sodium sulfate, filtered, and concentrated. The crude product was separated and purified by a silica gel column (petroleum ether: ethyl acetate (V/V)=(50:1) to (1:1)) to obtain 2-bromo-5-((2-methyl-1H-imidazol-1-yl)methyl)benzonitrile (800 mg, yield 79.6%).
LC-MS, M/Z (ESI): 276.0 $[M+H]^+$

Step 2: Synthesis of N-(tert-butyl)-3-(2-cyano-4-((2-methyl-1H-imidazol-1-yl)methyl)phenyl)-5-isobutylthiophene-2-sulfonamide

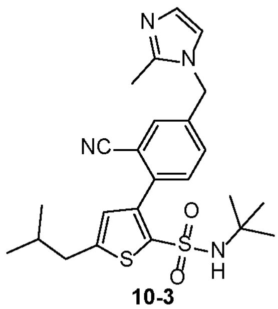

**10-3**

(2-(N-(tert-butyl)sulfamoyl)-5-isobutylthiophen-3-yl)boronic acid (500 mg, 1.57 mmol) was dissolved in tetrahydrofuran (10 mL) and water (5 mL), 2-bromo-5-((2-methyl-1H-imidazol-1-yl)methyl)benzonitrile (432 mg, 1.57 mmol) was added, and then potassium phosphate (1.66 g, 7.83 mmol) and XPhos Pd G4 (134 mg, 156 μmol) were added. The mixture was replaced with nitrogen for three times, and stirred and reacted at 60°C under nitrogen protection for 4 hours. The reaction solution was concentrated to obtain a crude product, and the crude product was separated and purified by a silica gel column (petroleum ether: ethyl acetate (V/V)=(50:1) to (0:1) to obtain N-(tert-butyl)-3-(2-cyano-4-((2-methyl-1H-imidazol-1-yl)methyl)phenyl)-5-isobutylthiophene-2-sulfonamide (650 mg, yield 88.1%).
LC-MS, M/Z (ESI): 471.1 $[M+H]^+$

Step 3: Synthesis of 3-(2-cyano-4-((2-methyl-1H-imidazol-1-yl)methyl)phenyl)-5-isobutylthiophene-2-sulfonamide

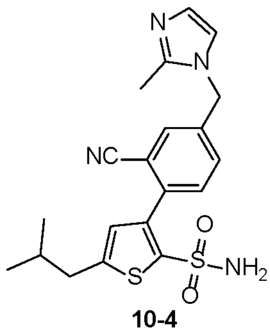

**10-4**

N-(tert-butyl)-3-(2-cyano-4-((2-methyl-1H-imidazol-1-yl)methyl)phenyl)-5-isobutylthiophene-2-sulfonamide (600 mg, 1.27 mmol) was dissolved in trifluoroacetic acid (3 mL) and dichloromethane (3 mL), and the mixture reacted at 20°C for 8 hours. The reaction solution was poured into water (10 mL), and then extracted with dichloromethane (50 mL) for three times. The organic phases were collected, dried over anhydrous sodium sulfate, filtered, and concentrated. The crude product was separated and purified by a silica gel column (petroleum ether: ethyl acetate (V/V)=(10:1) to (1:1)) to obtain 3-(2-cyano-4-((2-methyl-1H-imidazol-1-yl)methyl)phenyl)-5-isobutylthiophene-2-sulfonamide (500 mg, yield 94.6%).
LC-MS, M/Z (ESI): 415.1 $[M+H]^+$

Step 4: Synthesis of methyl (3-(2-cyano-4-((2-methyl-1H-imidazol-1-yl)methyl)phenyl)-5-isobutylthiophen-2-yl)sulfonylcarbamate

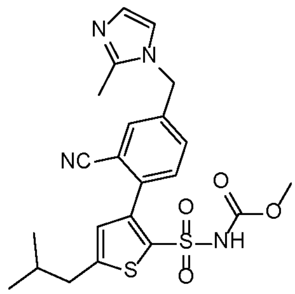

I-10

3-(2-cyano-4-((2-methyl-1H-imidazol-1-yl)methyl)phenyl)-5-isobutylthiophene-2-sulfonamide (500 mg, 1.21 mmol) and N,N-diisopropylethylamine (467 mg, 3.62 mmol) were dissolved in dichloromethane (10 mL), methyl chloroformate (455 mg, 4.82 mmol) was added, and the mixture was stirred at 0°C for 0.5 hour. The reaction solution was concentrated, and separated and purified by high performance liquid chromatography (column: Waters Xbridge 150*25mm*5$\mu$m; solvent: A=water + 0.05% by volume of ammonia water (30%), B=acetonitrile; gradient: 5% to 35%, 8 minutes) to obtain methyl (3-(2-cyano-4-((2-methyl-1H-imidazol-1-yl)methyl)phenyl)-5-isobutylthiophen-2-yl)sulfonylcarbamate (1-10) (82.9 mg, yield 18.6%).

LC-MS, M/Z (ESI): 473.2 $[M+H]^+$
$^1H$ NMR (DMSO-$d_6$) $\delta$ 7.71-7.84 (m, 2H), 7.52-7.64 (m, 2H), 7.40 (s, 1H), 6.78 (s, 1H), 5.39 (s, 2H), 3.31 (s, 3H), 2.66 (d, 2H), 2.54 (s, 3H), 1.82-1.89 (m, 1H), 0.93 (d, 6H) **Example 10:** Preparation of target compound **I-11**

Methyl (5-isobutyl-3-(2-methyl-4-((2-methyl-1H-imidazol-1-yl)methyl)phenyl)thiophen-2-yl)sulfonylcarbamate

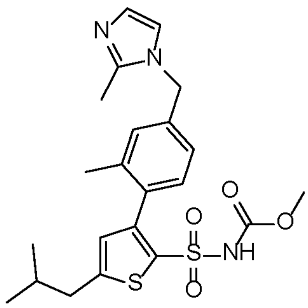

I-11

The synthetic scheme of the target compound 1-11 was as follows:

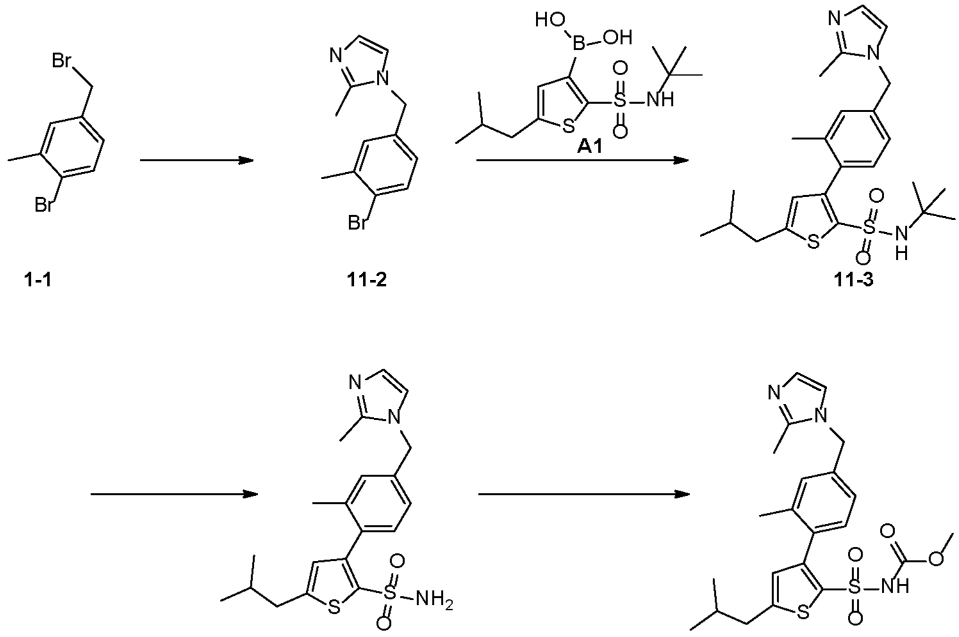


1-1 11-2 11-3

11-4 I-11

Step 1: Synthesis of 1-(4-bromo-3-methylbenzyl)-2-methyl-1H-imidazole

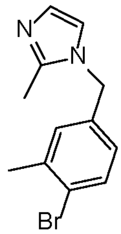

11-2

1-bromo-4-(bromomethyl)-2-methylbenzene (1.00 g, 3.79 mmol) and 2-methyl-1H-imidazole (933 mg, 11.3 mmol) were dissolved in N,N-dimethylformamide (10 mL), potassium carbonate (1.51 g, 11.3 mmol) was added, and the mixture reacted at 50°C for 2 hours. The reaction solution was poured into water (50 mL), extracted with ethyl acetate (100 mL) for three times. The organic phases were collected, dried over anhydrous sodium sulfate, filtered, and concentrated. The crude product was separated and purified by a silica gel column (petroleum ether: ethyl acetate (V/V)=(50:1) to (1:1)) to obtain 1-(4-bromo-3-methylbenzyl)-2-methyl-1H-imidazole (900 mg, yield 89.6%).
LC-MS, M/Z (ESI): 267.0 $[M+H]^+$

Step 2: Synthesis of N-(tert-butyl)-5-isobutyl-3-(2-methyl-4-((2-methyl-1H-imidazol-1 -yl)methyl)phenyl)thiophene-2-sulfonamide

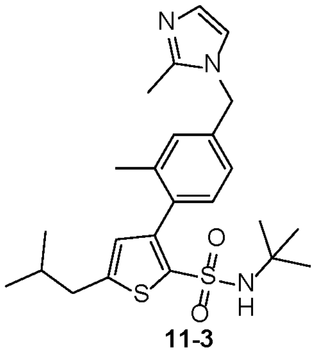

**11-3**

(2-(N-(tert-butyl)sulfamoyl)-5-isobutylthiophen-3-yl)boronic acid (500 mg, 1.57 mmol) was dissolved in tetrahydrofuran (10 mL) and water (5 mL), 1-(4-bromo-3-methylbenzyl)-2-methyl-1H-imidazole (415 mg, 1.57 mmol) was added, and then potassium phosphate (1.66 g, 7.83 mmol) and XPhos Pd G4 (134 mg, 156 μmol) were added. The mixture was replaced with nitrogen for three times, and stirred and reacted at 60°C under nitrogen protection for 4 hours. The reaction solution was concentrated to obtain a crude product, and the crude product was separated and purified by a silica gel column (petroleum ether: ethyl acetate (V/V)=(50:1) to (0:1)) to obtain N-(tert-butyl)-5-isobutyl-3-(2-methyl-4-((2-methyl-1H-imidazol-1-yl)methyl)phenyl)thiophene-2-sulfonamide (650 mg, yield 90.2%).
LC-MS, M/Z (ESI): 460.1 $[M+H]^+$

Step 3: Synthesis of 5-isobutyl-3-(2-methyl-4-((2-methyl-1H-imidazol-1-yl)methyl)phenyl)thiophene-2-sulfonamide

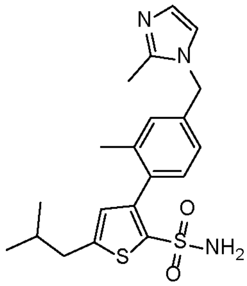

**11-4**

N-(tert-butyl)-5-isobutyl-3-(2-methyl-4-((2-methyl-1H-imidazol-1-yl)methyl)phenyl)thiophene-2-sulfonamide (550 mg, 1.20 mmol) was dissolved in trifluoroacetic acid (5 mL) and dichloromethane (5 mL), and the mixture reacted at 20°C for 8 hours. The reaction solution was poured into water (10 mL), and then extracted with dichloromethane (50 mL) for three times. The organic phases were collected, dried over anhydrous sodium sulfate, filtered, and concentrated. The crude product was separated and purified by a silica gel column (petroleum ether: ethyl acetate (V/V)=(10:1) to (0:1)) to obtain 5-isobutyl-3-(2-methyl-4-((2-methyl-1H-imidazol-1-yl)methyl)phenyl)thiophene-2-sulfonamide (400 mg, yield 89.4%).
LC-MS, M/Z (ESI): 404.1 $[M+H]^+$

Step 4: Synthesis of methyl (5-isobutyl-3-(2-methyl-4-((2-methyl-1H-imidazol-1-yl)methyl)phenyl)thiophen-2-yl)sulfonylcarbamate

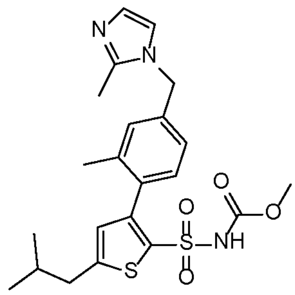

**I-11**

5-isobutyl-3-(2-methyl-4-((2-methyl-1H-imidazol-1-yl)methyl)phenyl)thiophene-2-sulfonamide (400 mg, 991 μmol) and N,N-diisopropylethylamine (384 mg, 2.97 mmol) were dissolved in dichloromethane (10.0 mL), methyl chloroformate (374 mg, 3.96 mmol) was added, and the mixture was stirred and reacted at 0°C for 0.5 hour. The reaction solution was concentrated, and separated and purified by high performance liquid chromatography (column: Waters Xbridge 150*25mm*5μm; solvent: A=water + 0.05% by volume of ammonia water (30%), B=acetonitrile; gradient: 9% to 39%, 8 minutes) to obtain methyl (5-isobutyl-3-(2-methyl-4-((2-methyl-1H-imidazol-1-yl)methyl)phenyl)thiophen-2-yl) sulfonylcarbamate (172 mg, yield 47.1%).

LC-MS, M/Z (ESI): 462.2 $[M+H]^+$
$^1H$ NMR (DMSO-$d_6$) $\delta$:7.48 (s, 1H), 7.26 (s, 1H), 7.16 (d, 1H), 7.11 (s, 1H), 7.00 (d, 1H), 6.57 (s, 1H), 5.24 (s, 2H), 3.31 (s, 3H), 2.64 (d, 2H), 2.48 (s, 3H), 2.05 (s, 3H), 1.85-1.86 (m, 1H), 0.92 (d, 6H)

**Example 11:** Preparation of target compound **I-12**

Butyl ((3-(3-cyano-4-((2-methyl-1H-imidazol-1-yl)methyl)phenyl)-5-isobutylthiophen-2-yl)sulfonyl)carbamate

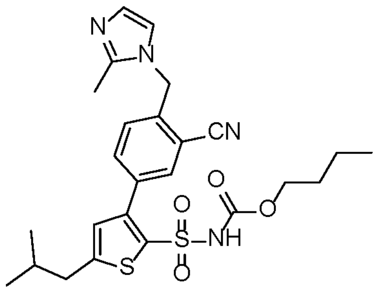

I-12

The synthetic scheme of the target compound 1-12 was as follows:

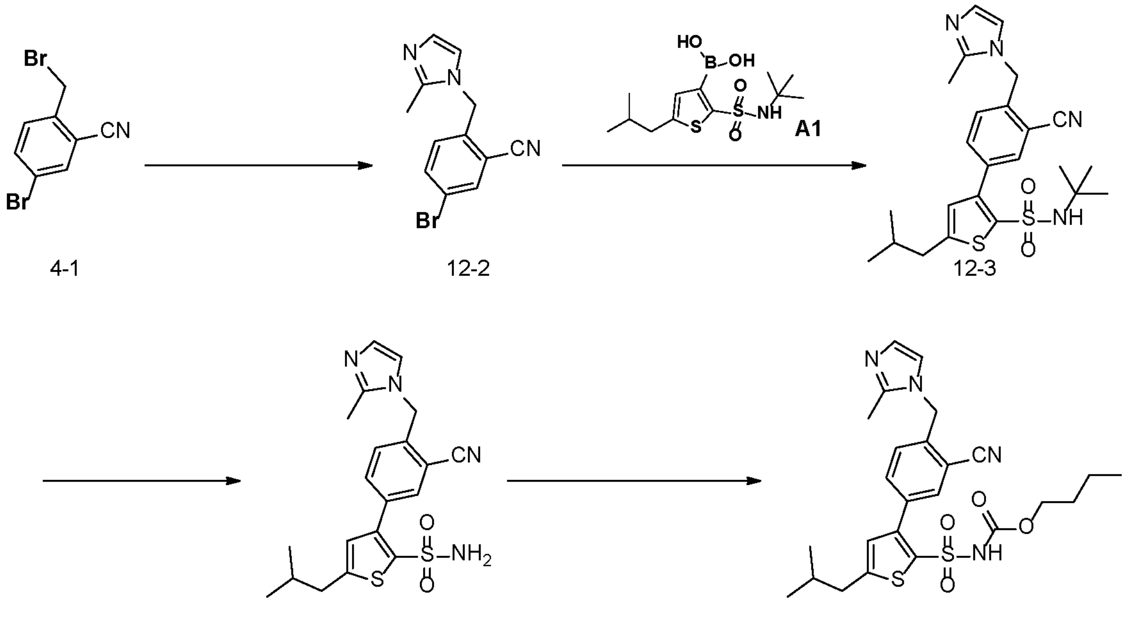


4-1 12-2 12-3

12-4 I-12

Step 1: Synthesis of 5-bromo-2-((2-methyl-1H-imidazol-1-yl)methyl)benzonitrile

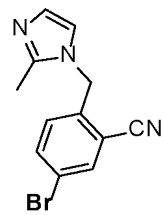

12-2

5-bromo-2-(bromomethyl)benzonitrile (300 mg, 1.1 mmol), 2-methyl-1H-imidazole (131.4 mg, 1.6 mmol), and potassium carbonate (221.1 mg, 1.6 mmol) were added to N,N-dimethylformamide (4.5 mL). The mixture reacted at 20°C for 3 hours, until LCMS indicated the completion of reaction. 50 mL of water was added, and the mixture was extracted with ethyl acetate (20 mL × 3) for three times. The organic phases were collected and concentrated to dryness. The residue was separated and purified by reversed-phase column (0.1% formic acid) to obtain 5-bromo-2-((2-methyl-1H-imidazol-1-yl)methyl)benzonitrile (250 mg, yield 83%).
LC-MS, M/Z (ESI): 275.9 $[M+H]^+$

Step 2: Synthesis of N-(tert-butyl)-3-(3-cyano-4-((2-methyl-1H-imidazol-1-yl)methyl)phenyl)-5-isobutylthiophene-2-sulfonamide

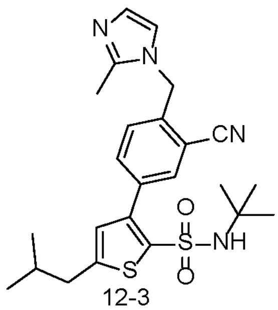

12-3

5-bromo-2-((2-methyl-1H-imidazol-1-yl)methyl)benzonitrile (100 mg, 0.4 mmol) was added to 2 mL of N,N-dimethylformamide. Then, (2-(N-(tert-butyl)sulfamoyl)-5-isobutylthiophen-3-yl)boric acid (191.5 mg, 0.9 mmol), 1,1'-bis(diphenylphosphino)ferrocene palladium(II) dichloride (73.1 mg, 0.1 mmol), and potassium carbonate (221.1 mg, 1.6 mmol) were added, and replaced with nitrogen for three times. The mixture was heated to 90°C under nitrogen protection for overnight reaction, until LCMS indicated the complete reaction of the raw material. The reaction system was diluted by adding 30 mL of water, and extracted with ethyl acetate (10 mL × 3). The organic phases were collected and concentrated to dryness. The residue was separated and purified with a silica gel column (DCM: MeOH (V/V)=10:1) to obtain N-(tert-butyl)-3-(3-cyano-4-((2-methyl-1H-imidazol-1-yl)methyl)phenyl)-5-isobutylthiophene-2-sulfonamide (170 mg, yield 80%).
LC-MS, M/Z (ESI): 471.1 $[M+H]^+$

Step 3: Synthesis of 3-(3-cyano-4-((2-methyl-1H-imidazol-1-yl)methyl)phenyl)-5-isobutylthiophene-2-sulfonamide

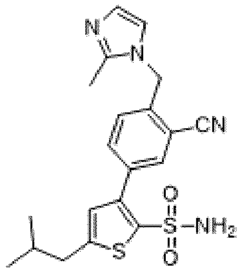

12-4

N-(tert-butyl)-3-(3-cyano-4-((2-methyl-1H-imidazol-1-yl)methyl)phenyl)-5-isobutylthiophene-2-sulfonamide (170 mg, 0.4 mmol) was added to 3.5 mL of trifluoroacetic acid, and the mixture reacted at room temperature overnight. LCMS showed that the raw material was completely reacted. The reaction mixture was concentrated and dried to obtain 3-(3-cyano-4-((2-methyl-1H-imidazol-1-yl)methyl)phenyl)-5-isobutylthiophene-2-sulfonamide (145 mg, yield 83%).
LC-MS, M/Z (ESI): 415.0 $[M+H]^+$

Step 4: Synthesis of butyl ((3-(3-cyano-4-((2-methyl-1H-imidazol-1-yl)methyl)phenyl)-5-isobutylthiophen-2-yl)sulfonyl) carbamate

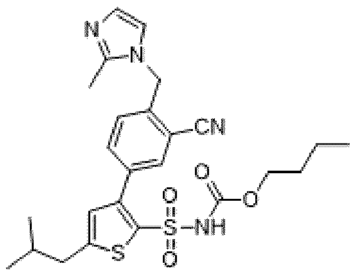

I-12

3-(3-cyano-4-((2-methyl-1H-imidazol-1-yl)methyl)phenyl)-5-isobutylthiophene-2-sulfonamide (145 mg, 0.3 mmol) was added to 4.5 mL of dichloromethane, and then triethylamine (303.6 mg, 3.0 mmol) and butyl chloroformate (122.9 mg, 0.9 mmol) were added. The mixture reacted at room temperature for 2 hours, until LCMS indicated the complete reaction of the raw material. The mixture was diluted by adding 30 mL of water, and extracted with ethyl acetate (10 mL × 3). The organic phases were collected and concentrated to dryness. The residue was subjected to a preparative purification by reversed-phase column (0.1% trifluoroacetic acid) to obtain butyl ((3-(3-cyano-4-((2-methyl-1H-imidazol-1-yl)methyl)phenyl)-5-isobutylthiophen-2-yl)sulfonyl)carbamate (59 mg, yield 32.8%).

LC-MS, M/Z (ESI): 515.1 $[M+H]^+$
$^1H$ NMR (400 MHz, DMSO-$d_6$) δ 8.24 (d, 1H), 8.02 (dd, 1H), 7.45 (s, 1H), 7.38 (s, 1H), 7.17 (d, 1H), 6.92 (s, 1H), 5.56 (s,

2H), 3.72 (t, 2H), 2.64 (d, 2H), 2.51 (s, 3H), 1.86 (dt, 1H), 1.36 (tt, 2H), 1.26 - 1.13 (m, 2H), 0.93 (s, 3H), 0.92 (s, 3H), 0.81 (t, 3H)

**Example 12:** Preparation of target compound **I-13**

Methyl ((3-(4-((2-(tert-butyl)-1H-imidazol-1-yl)methyl)-3-cyanophenyl)-5-isobutylthiophen-2-yl)sulfonyl)carbamate

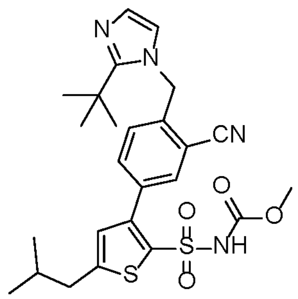

I-13

The synthetic scheme of the target compound 1-13 was as follows:

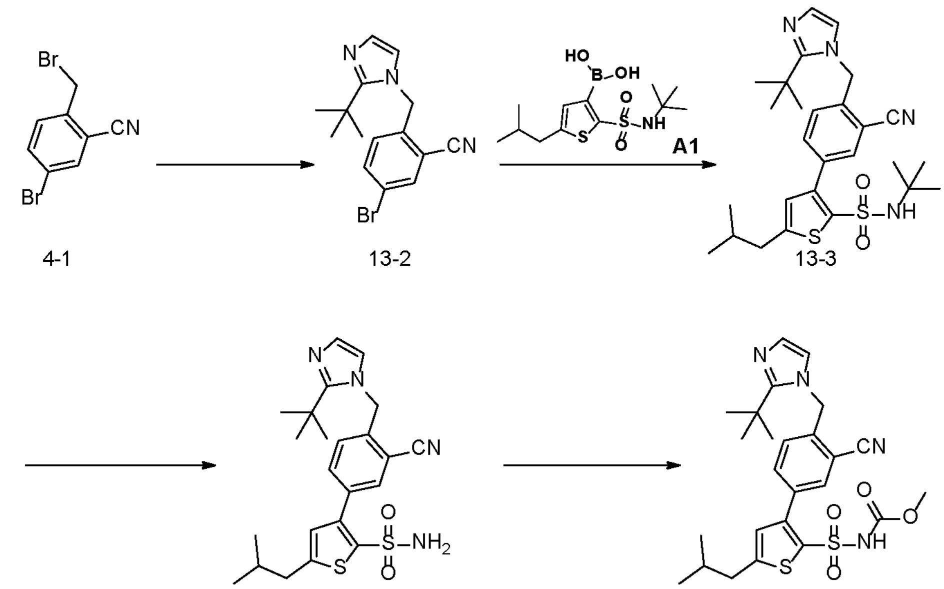


Step 1: Synthesis of 5-bromo-2-((2-(tert-butyl)-1H-imidazol-1-yl)methyl)benzonitrile

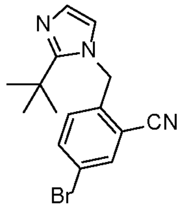

13-2

5-bromo-2-(bromomethyl)benzonitrile (300 mg, 1.1 mmol), 2-(tert-butyl)-1H-imidazole (198.7 mg, 1.6 mmol),

and potassium carbonate (221.1 mg, 1.6 mmol) were added to N,N-dimethylformamide (6 mL), and the mixture reacted at 20°C for 3 hours, until LCMS indicated the completion of reaction. 50 mL of water was added, and the mixture was extracted with ethyl acetate (20 mL×3). The organic phases were collected and concentrated to dryness. The residue was separated and purified by reversed-phase column (0.1% formic acid) to obtain 5-bromo-2-((2-(tert-butyl)-1H-imidazol-1-yl)methyl)benzonitrile (300 mg, yield 86.4%).
LC-MS, M/Z (ESI): 320.0 $[M+H]^+$

Step 2: Synthesis of N-(tert-butyl)-3-(4-((2-(tert-butyl)-1H-imidazol-1-yl)methyl)-3-cyanophenyl)-5-isobutylthiophene-2-sulfonamide

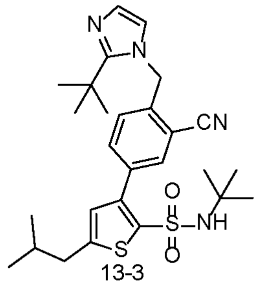

13-3

5-bromo-2-((2-(tert-butyl)-1H-imidazol-1-yl)methyl)benzonitrile (100 mg, 0.3 mmol) was added to 2 mL of N,N-dimethylformamide. Then, (2-(N-(tert-butyl)sulfamoyl)-5-isobutylthiophen-3-yl)boric acid (127.7 mg, 0.4 mmol), 1,1'-bis(diphenylphosphino)ferrocene palladium(II) dichloride (73.1 mg, 0.1 mmol), and potassium carbonate (165.8 mg, 1.2 mmol) were added and replaced with nitrogen for three times. The mixture was heated to 90°C under nitrogen protection and reacted overnight, until LCMS indicated complete reaction of the raw materials. The reaction system was diluted by adding 30 mL of water, and extracted with ethyl acetate (10 mL × 2). The organic phases were collected and concentrated to dryness. The residue was separated and purified by a silica gel column (DCM: MeOH (V/V)=10:1) to obtain N-(tert-butyl)-3-(4-((2-(tert-butyl)-1H-imidazol-1-yl)methyl)-3-cyanophenyl)-5-isobutylthiophene-2-sulfonamide (160 mg, yield 60%).
LC-MS, M/Z (ESI): 513.2 $[M+H]^+$

Step 3: Synthesis of 3-(4-((2-(tert-butyl)-1H-imidazol-1-yl)methyl)-3-cyanophenyl)-5-isobutylthiophene-2-sulfonamide

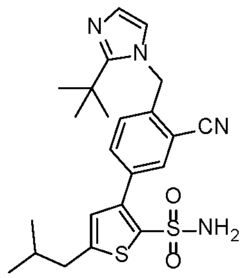

13-4

N-(tert-butyl)-3-(4-((2-(tert-butyl)-1H-imidazol-1-yl)methyl)-3-cyanophenyl)-5-isobutylthiophene-2-sulfonamide (160 mg, 0.3 mmol) was added to 3.5 mL of trifluoroacetic acid. The mixture reacted at room temperature overnight, until LCMS indicated complete reaction of the raw materials. The reaction mixture was concentrated and dried to obtain 3-(4-((2-(tert-butyl)-1H-imidazol-1-yl)methyl)-3-cyanophenyl)-5-isobutylthiophene-2-sulfonamide (140 mg, yield 50%).
LC-MS, M/Z (ESI): 457.1 $[M+H]^+$

Step 4: Synthesis of methyl ((3-(4-((2-(tert-butyl)-1H-imidazol-1-yl)methyl)-3-cyanophenyl)-5-isobutylthiophen-2-yl)sulfonyl)carbamate

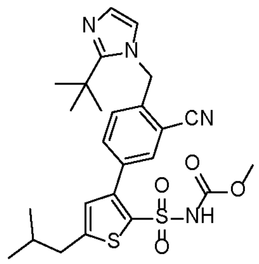

I-13

3-(4-((2-(tert-butyl)-1H-imidazol-1-yl)methyl)-3-cyanophenyl)-5-isobutylthiophene-2-sulfonamide (140 mg, 0.3 mmol) was added to 3 mL of dichloromethane, and then triethylamine (303.6 mg, 3.0 mmol) and methyl chloroformate (85 mg, 0.9 mmol) were added. The mixture reacted at room temperature for 2 hours, until LCMS indicated complete reaction of the raw materials. The mixture was diluted by adding 30 mL of water, and extracted with ethyl acetate (10 mL × 2). The organic phases were concentrated to dryness. The residue was subjected to a preparative purification by reversed-phase column (0.1% formic acid) to obtain methyl ((3-(4-((2-(tert-butyl)-1H-imidazol-1-yl)methyl)-3-cyanophenyl)-5-isobutylthiophen-2-yl)sulfonyl)carbamate (27 mg, yield 17.1%).

LC-MS, M/Z (ESI): 515.1 $[M+H]^+$
$^1H$ NMR (400 MHz, DMSO-$d_6$) δ 8.32 (s, 1H), 8.09 - 7.98 (m, 1H), 7.18 (d, 1H), 7.09 (d, 1H), 6.92 (s, 1H), 6.84 (d, 1H), 5.65 (s, 2H), 3.35 (s, 3H), 2.64 (d, 2H), 1.86 (dt, 1H), 1.37 (s, 9H), 0.94 (s, 3H), 0.93 (s, 3H)

**Example 13:** Preparation of target compound **I-14**

Methyl ((3-(3-cyano-4-((2-ethyl-1H-imidazol-1-yl)methyl)phenyl)-5-isobutylthiophen-2-yl)sulfonyl)carbamate

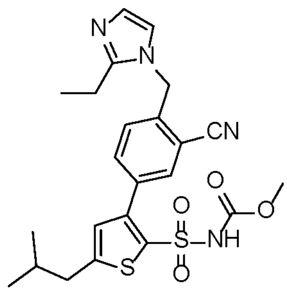

I-14

The synthetic scheme of the target compound 1-14 was as follows:

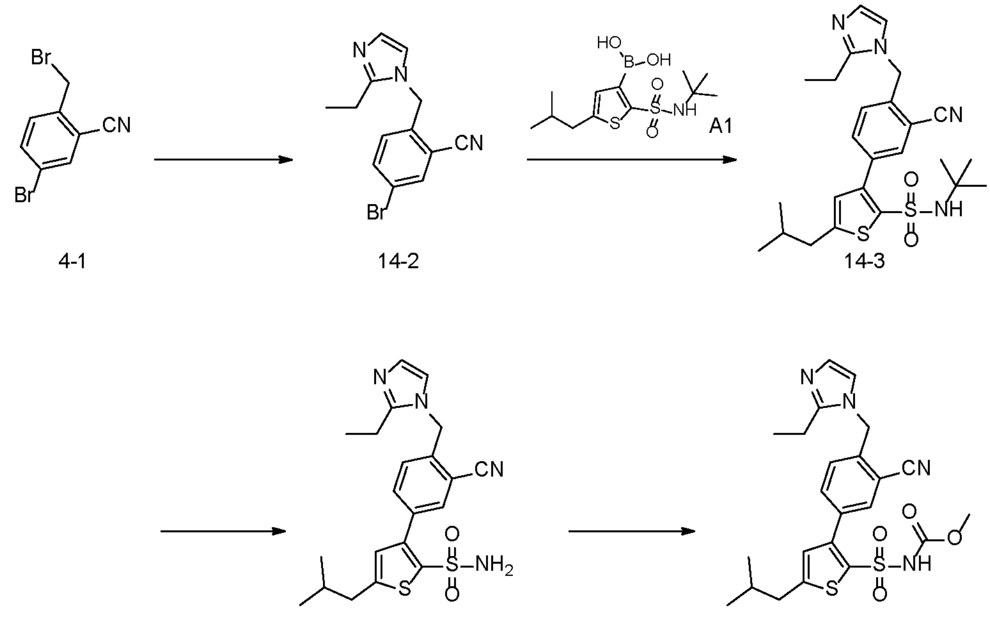


4-1 14-2 14-3

14-4 I-14

Step 1: Synthesis of 5-bromo-2-((2-ethyl-1H-imidazol-1-yl)methyl)benzonitrile

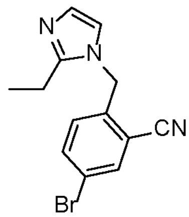

14-2

5-bromo-2-(bromomethyl)benzonitrile (300 mg, 1.1 mmol), 2-ethyl-1H-imidazole (153.8 mg, 1.6 mmol), and potassium carbonate (221.1 mg, 1.6 mmol) were added to N,N-dimethylformamide (6 mL), and the mixture reacted at 20°C for 3 hours, until LCMS indicated the completion of reaction. The reaction system was added with 50 mL of water and extracted with ethyl acetate (20 mL×3). The organic phases were collected and concentrated to dryness. The residue was separated and purified by reversed-phase column (0.1% formic acid) to obtain 5-bromo-2-((2-ethyl-1H-imidazol-1-yl)methyl)benzonitrile (300 mg, yield 94.8%).
LC-MS, M/Z (ESI): 290.5 $[M+H]^+$

Step 2: Synthesis of N-(tert-butyl)-3-(3-cyano-4-((2-ethyl-1H-imidazol-1-yl)methyl)phenyl)-5-isobutylthiophene-2-sulfonamide

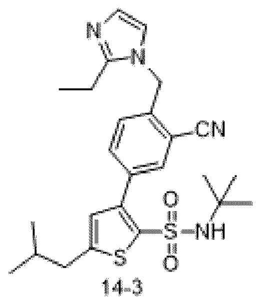

14-3

5-bromo-2-((2-ethyl-1H-imidazol-1-yl)methyl)benzonitrile (100 mg, 0.3 mmol) was added to 2 mL of N,N-dimethylformamide. Then, (2-(N-(tert-butyl)sulfamoyl)-5-isobutylthiophen-3-yl)boric acid (127.7 mg, 0.4 mmol), 1,1'-bis(diphenylphosphino)ferrocene palladium(II) dichloride (73.1 mg, 0.1 mmol), and potassium carbonate (165.8 mg, 1.2 mmol) were added and replaced with nitrogen for three times. The mixture was heated to 90°C under nitrogen protection and reacted overnight, until LCMS indicated complete reaction of the raw materials. The reaction system was diluted by adding 30 mL of water, extracted with ethyl acetate (10 mL × 2). The organic phases were collected and concentrated to dryness. The residue was separated and purified by a silica gel column (DCM: MeOH (V/V)=10:1) to obtain N-(tert-butyl)-3-(3-cyano-4-((2-ethyl-1H-imidazol-1-yl)methyl)phenyl)-5-isobutylthiophene-2-sulfonamide (160 mg, yield 66%).
LC-MS, M/Z (ESI): 485.1 $[M+H]^+$

Step 3: Synthesis of 3-(3-cyano-4-((2-ethyl-1H-imidazol-1-yl)methyl)phenyl)-5-isobutylthiophene-2-sulfonamide

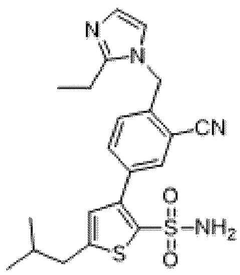

14-4

N-(tert-butyl)-3-(3-cyano-4-((2-ethyl-1H-imidazol-1-yl)methyl)phenyl)-5-isobutylthiophene-2-sulfonamide (160 mg, 0.3 mmol) was added to 3.5 mL of trifluoroacetic acid, and the mixture reacted at room temperature overnight, until LCMS indicated complete reaction of the raw materials. The reaction mixture was concentrated and dried to obtain a crude product of 3-(3-cyano-4-((2-ethyl-1H-imidazol-1-yl)methyl)phenyl)-5-isobutylthiophene-2-sulfonamide (140 mg, yield 48.5%).
LC-MS, M/Z (ESI): 429.1 $[M+H]^+$

Step 4: Synthesis of methyl ((3-(3-cyano-4-((2-ethyl-1H-imidazol-1-yl)methyl)phenyl)-5-isobutylthiophen-2-yl)sulfonyl) carbamate

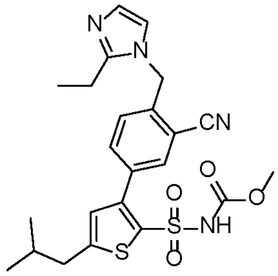

I-14

3-(3-cyano-4-((2-ethyl-1H-imidazol-1-yl)methyl)phenyl)-5-isobutylthiophene-2-sulfonamide (140 mg, 0.3 mmol) was added to 3 mL of dichloromethane, and then triethylamine (303.6 mg, 3.0 mmol) and methyl chloroformate (85 mg, 0.9 mmol) were added. The mixture reacted at room temperature for 2 hours, until LCMS indicated complete reaction of the raw materials. The reaction system was diluted by adding 30 mL of water, and extracted with ethyl acetate (10 mL × 2). The organic phases were collected and concentrated to dryness. The residue was subjected to a preparative purification by reversed-phase column (0.1% formic acid) to obtain methyl ((3-(3-cyano-4-((2-ethyl-1H-imidazol-1-yl)methyl)phenyl)-5-isobutylthiophen-2-yl)sulfonyl)carbamate (43 mg, yield 27.0%).

LC-MS, M/Z (ESI): 487.2 $[M+H]^+$

$^1H$ NMR (400 MHz, DMSO-$d_6$) δ 8.27 (d, 1H), 8.03 (dd, 1H), 7.49 (s, 2H), 7.15 (d, 1H), 6.92 (s, 1H), 5.60 (s, 2H), 3.32 (s, 3H), 2.90 (q, 2H), 2.64 (d, 2H), 1.86 (dt, 1H), 1.22 (t, 3H), 0.94 (s, 3H), 0.92 (s, 3H)

**Example 14:** Preparation of target compound **I-17**

Methyl (3-(4-((2-(tert-butyl)-1H-imidazol-1-yl)methyl)-2-cyanophenyl)-5-isobutylthiophen-2-yl)sulfonylcarbamate

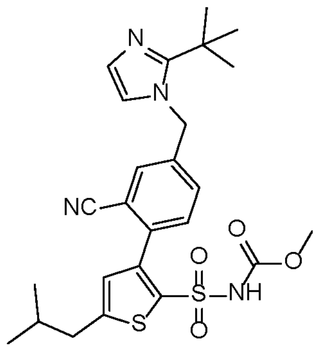

**I-17**

The synthetic scheme of the target compound 1-17 was as follows:

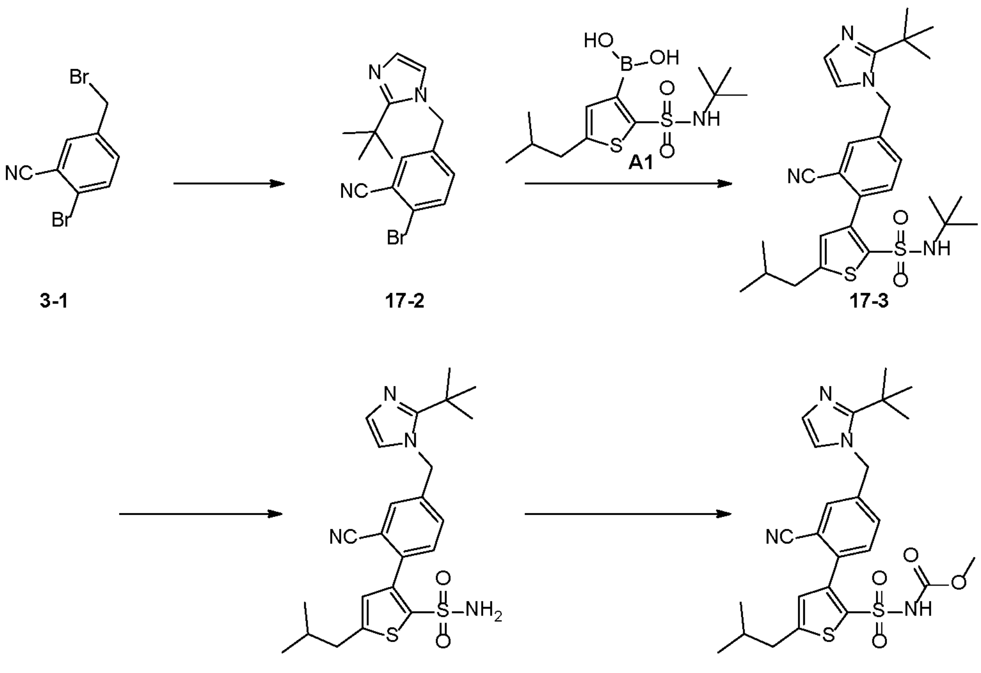

3-1 17-2 A1 17-3

17-4 I-17

Step 1: Synthesis of 2-bromo-5-((2-(tert-butyl)-1H-imidazol-1-yl)methyl)benzonitrile

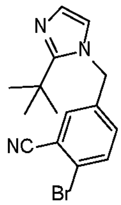

**17-2**

2-bromo-5-(bromomethyl)benzonitrile (1.00 g, 3.64 mmol) and 2-(tert-butyl)-1H-imidazole (1.36 g, 10.9 mmol) were dissolved in N,N-dimethylformamide (20 mL), and potassium carbonate (1.51 g, 10.9 mmol) was added. The mixture reacted at 50°C for 2 hours. The reaction solution was poured into water (50 mL), and extracted with ethyl acetate (100 mL) for three times. The organic phases were collected, dried over anhydrous sodium sulfate, filtered, and concentrated. The crude product was separated and purified by a silica gel column (petroleum ether: ethyl acetate (V/V)=(50:1) to (1:1)) to obtain 2-bromo-5-((2-(tert-butyl)-1H-imidazol-1-yl)methyl)benzonitrile (1.00 g, yield 86.4%).
LC-MS, M/Z (ESI): 318.0 $[M+H]^+$

Step 2: Synthesis of N-(tert-butyl)-3-(4-((2-(tert-butyl)-1H-imidazol-1-yl)methyl)-2-cyanophenyl)-5-isobutylthiophene-2-sulfonamide

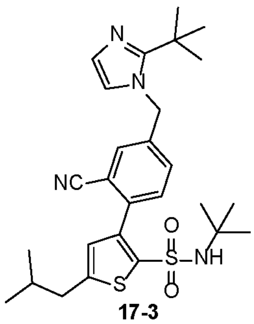

**17-3**

(2-(N-(tert-butyl)sulfamoyl)-5-isobutylthiophen-3-yl)boronic acid (500 mg, 1.57 mmol) was dissolved in tetrahydrofuran (10 mL) and water (5 mL). 2-bromo-5-((2-(tert-butyl)-1H-imidazol-1-yl)methyl)benzonitrile (498 mg, 1.57 mmol) was added, and then potassium phosphate (1.66 g, 7.83 mmol) and XPhos Pd G4 (134 mg, 156 μmol) were added. The mixture was stirred and reacted at 60°C under nitrogen protection for 4 hours. The reaction solution was concentrated to obtain a crude product, and the crude product was separated and purified by a silica gel column (petroleum ether: ethyl acetate (V/V)=(50: 1) to (0:1)) to obtain N-(tert-butyl)-3-(4-((2-(tert-butyl)-1H-imidazol-1-yl)methyl)-2-cyanophenyl)-5-isobutylthiophene-2-sulfonamide (550 mg, yield 68.4%).
LC-MS, M/Z (ESI): 513.1 $[M+H]^+$

Step 3: Synthesis of 3-(4-((2-(tert-butyl)-1H-imidazol-1-yl)methyl)-2-cyanophenyl)-5-isobutylthiophene-2-sulfonamide

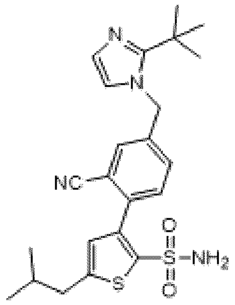

17-4

N-(tert-butyl)-3-(4-((2-(tert-butyl)-1H-imidazol-1-yl)methyl)-2-cyanophenyl)-5-isobutylthiophene-2-sulfonamide (500 mg, 957 μmol) was dissolved in trifluoroacetic acid (5 mL) and dichloromethane (5 mL), and the mixture reacted at 20°C for 8 hours. The reaction solution was poured into water (10 mL), and then extracted with dichloromethane (50 mL) for three times. The organic phases were collected, dried over anhydrous sodium sulfate, filtered, and concentrated. The crude product was separated and purified by a silica gel column (petroleum ether: ethyl acetate (V/V)=(10:1) to (0:1)) to obtain 3-(4-((2-(tert-butyl)-1H-imidazol-1-yl)methyl)-2-cyanophenyl)-5-isobutylthiophene-2-sulfonamide (410 mg, yield 92.1 %).
LC-MS, M/Z (ESI): 457.1 $[M+H]^+$

Step 4: Synthesis of methyl (3-(4-((2-(tert-butyl)-1H-imidazol-1-yl)methyl)-2-cyanophenyl)-5-isobutylthiophen-2-yl)sulfonylcarbamate

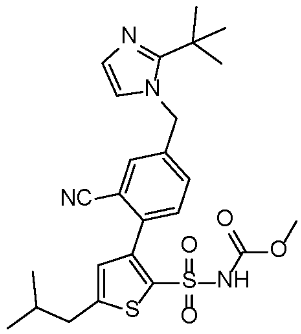

**I-17**

3-(4-((2-(tert-butyl)-1H-imidazol-1-yl)methyl)-2-cyanophenyl)-5-isobutylthiophene-2-sulfonamide (400 mg, 876 μmol) and N,N-diisopropylethylamine (339 mg, 2.63 mmol) were dissolved in dichloromethane (10 mL), methyl chloroformate (248 mg, 2.63 mmol) was added, and the mixture was stirred and reacted at 0°C for 0.5 hour. The reaction solution was concentrated, and separated and purified by high performance liquid chromatography (column: Waters Xbridge Prep OBD C18 150*40mm*10μm; solvent: A=water + 0.05% by volume of ammonia water (30%), B=acetonitrile; gradient: 0% to 30%, 10 minutes) to obtain methyl (3-(4-((2-(tert-butyl)-1H-imidazol-1-yl)methyl)-2-cyanophenyl)-5-isobutylthiophen-2-yl)sulfonylcarbamate (191 mg, yield 45.4%).

LC-MS, M/Z (ESI): 515.2 $[M+H]^+$
$^1H$ NMR (DMSO-$d_6$) $\delta$:7.75 (d, 1H), 7.55 (s, 1H), 7.30-7.39 (m, 2H), 7.24 (s, 1H), 6.82 (s, 1H), 5.59 (s, 2H), 3.36 (s, 3H), 2.67 (d, 2H), 1.82-1.86 (m, 1H), 1.38 (s, 9H), 0.93 (d, 6H)

**Example 15:** Preparation of target compound **I-16**

Methyl (5-isobutyl-4-methyl-3-(4-((2-methyl-1H-imidazol-1-yl)methyl)phenyl)thiophen-2-yl)sulfonylcarbamate

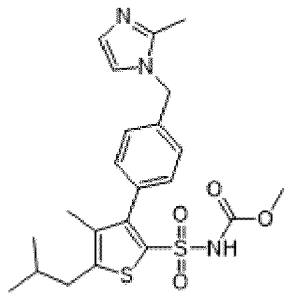

I-16

The synthetic scheme of the target compound **I-16** was as follows:

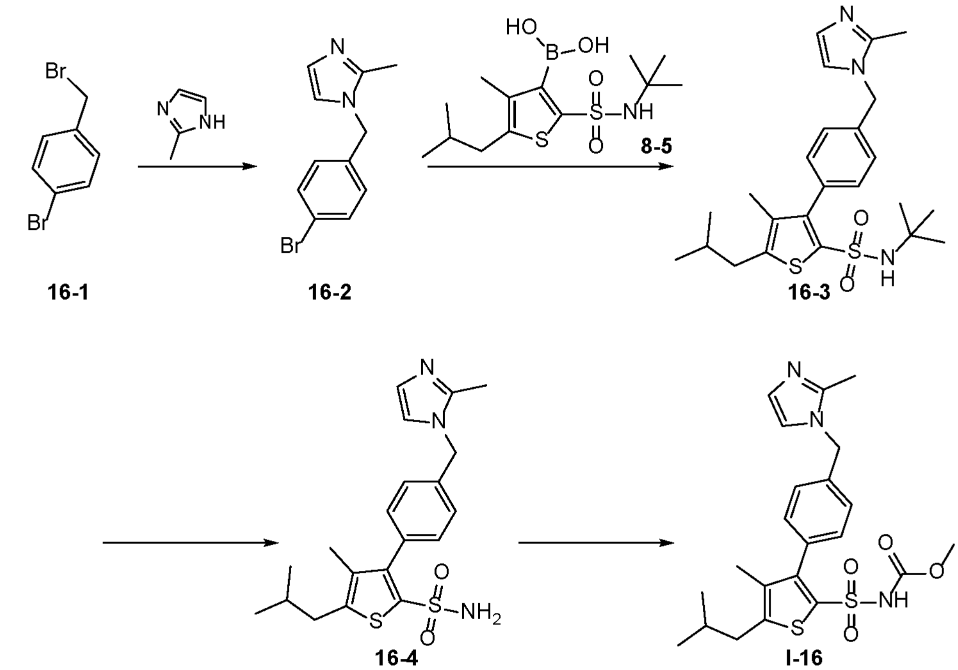

16-1 16-2 8-5 16-3 16-4 I-16

Step 1: Synthesis of 1-(4-bromobenzyl)-2-methyl-1H-imidazole

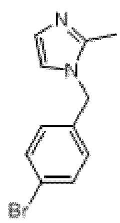

16-2

1-bromo-4-(bromomethyl)benzene (1.50 g, 6.00 mmol) and 2-methyl-1H-imidazole (492 mg, 6.00 mmol) were dissolved in N,N-dimethylformamide (30.0 mL), potassium carbonate (2.49 g, 18.0 mmol) was added, and the mixture reacted at 50°C for 2 hours. The reaction solution was poured into water (25 mL), and extracted with ethyl acetate (100 mL) for three times. The organic phases were collected, dried over anhydrous sodium sulfate, filtered, and concentrated. The crude product was separated and purified by a silica gel column (petroleum ether: ethyl acetate (V/V)=(50:1) to (1:1)) to obtain 1-(4-bromobenzyl)-2-methyl-1H-imidazole (1.40 g, yield 92.8%).
LC-MS, M/Z (ESI): 253.2 $[M+H]^+$

Step 2: Synthesis of N-(tert-butyl)-5-isobutyl-4-methyl-3-(4-((2-methyl-1H-imidazol-1 -yl)methyl)phenyl)thiophene-2-sulfonamide

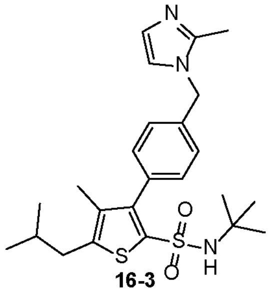

16-3

(2-(N-(tert-butyl)sulfamoyl)-5-isobutyl-4-methylthiophen-3-yl)boronic acid (2.00 g, 6.00 mmol) was dissolved in tetrahydrofuran (20 mL) and water (10 mL). 1-(4-bromobenzyl)-2-methyl-1H-imidazole (1.36 g, 5.40 mmol) was added, and then potassium phosphate (6.37 g, 30.0 mmol) and XPhos Pd G4 (258 mg, 300 μmol) were added. The mixture was stirred and reacted at 60°C under nitrogen protection for 4 hours. The reaction solution was concentrated to obtain a crude product, and the crude product was separated and purified by a silica gel column (petroleum ether: ethyl acetate (V/V)= (50:1) to (0:1)) to obtain N-(tert-butyl)-5-isobutyl-4-methyl-3-(4-((2-methyl-1H-imidazol-1-yl)methyl)phenyl)thiophene-2-sulfonamide (2.20 g, yield 79.7%).
LC-MS, M/Z (ESI): 460.3 $[M+H]^+$

Step 3 Synthesis of 5-isobutyl-4-methyl-3-(4-((2-methyl-1H-imidazol-1-yl)methyl)phenyl)thiophene-2-sulfonamide

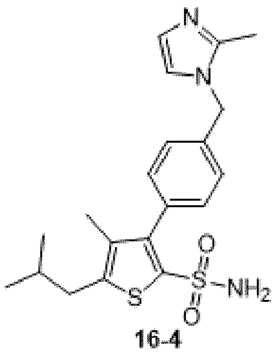

16-4

N-(tert-butyl)-5-isobutyl-4-methyl-3-(4-((2-methyl-1H-imidazol-1-yl)methyl)phenyl)thiophene-2-sulfonamide (2.10 g, 4.57 mmol) was dissolved in trifluoroacetic acid (20 mL) and dichloromethane (20 mL), and the mixture reacted at 40°C for 8 hours. The reaction solution was poured into water (10 mL), and then extracted with dichloromethane (50 mL) for three times. The organic phases were collected, dried over anhydrous sodium sulfate, filtered, and concentrated. The crude product was separated and purified by a silica gel column (petroleum ether: ethyl acetate (V/V)=(10:1) to (1:1)) to obtain 5-isobutyl-4-methyl-3-(4-((2-methyl-1H-imidazol-1-yl)methyl)phenyl)thiophene-2-sulfonamide (1.80 g, yield 97.6%).
LC-MS, M/Z (ESI): 404.2 $[M+H]^+$

Step 4: Synthesis of methyl (5-isobutyl-4-methyl-3-(4-((2-methyl-1H-imidazol-1-yl)methyl)phenyl)thiophen-2-yl)sulfonylcarbamate

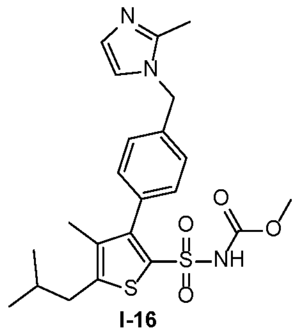

I-16

5-isobutyl-4-methyl-3-(4-((2-methyl-1H-imidazol-1-yl)methyl)phenyl)thiophene-2-sulfonamide (1.80 g, 4.46 mmol) and N,N-diisopropylethylamine (1.73 g, 13.4 mmol) were dissolved in dichloromethane (20 mL), methyl chloroformate (1.26 g, 13.4 mmol) was added, and the mixture was stirred and reacted at 0°C for 1 hour. The reaction solution was concentrated, separated and purified by high performance liquid chromatography (column: Phenomenex luna C18 150*40mm*15um; solvent: A=water + 0.05 volume of formic acid (99%), B=acetonitrile; gradient: 15% to 45%, 15 minutes), concentrated, and dried to obtain methyl (5-isobutyl-4-methyl-3-(4-((2-methyl-1H-imidazol-1-yl)methyl)phenyl) thiophen-2-yl)sulfonylcarbamate (833 mg, yield 43.8%).

$^1$H NMR (400 MHz, $CDCl_3$) $\delta$ 7.36 (d, 2H), 7.16 (d, 2H), 6.69 (d, 1H), 6.52 (d, 1H), 5.03 (s, 2H), 3.61 (s, 3H), 2.66 (d, 2H), 2.45 (s, 3H), 1.92-2.03 (m, 1H), 1.83 (s, 3H), 1.01 (d, 6H).
LC-MS, M/Z (ESI): 462.4 $[M+H]^+$

**Example 16:** Preparation of target compound **I-18**

3-(2-cyano-4-((2-methyl-1H-imidazol-1-yl)methyl)phenyl)-5-isobutyl-N-((3,3,3-trifluoropropyl)carbamoyl)thiophene-2-sulfonamide

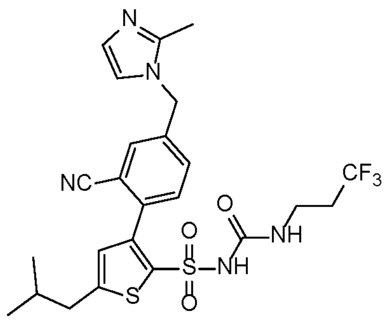

I-18

The synthetic scheme of the target compound 1-18 was as follows:

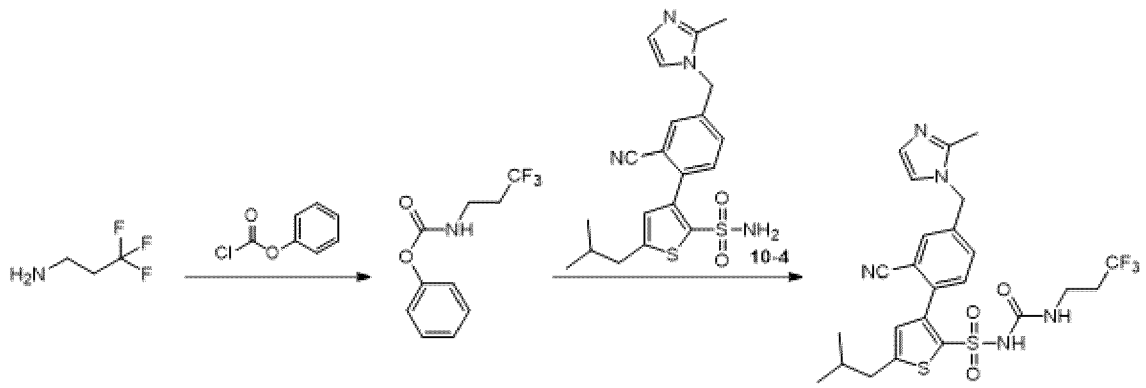

18-1 18-2 I-18

Step 1: Synthesis of phenyl (3,3,3-trifluoropropyl)carbamate

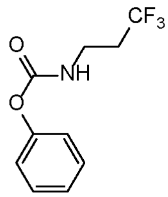

**18-2**

3,3,3-trifluoropropane-1-amine (500 mg, 4.42 mmol) was dissolved in tetrahydrofuran (10 mL), and then triethylamine (671 mg, 6.63 mmol) and phenyl chloroformate (831 mg, 5.31 mmol) were added. The mixture reacted at 25°C for 1 hour. The reaction solution was poured into water (50 mL), and extracted with ethyl acetate (50 mL) for three times. The organic phases were collected, dried over anhydrous sodium sulfate, filtered, and concentrated to obtain phenyl (3,3,3-trifluoropropyl)carbamate (200 mg, yield 19.4%).
LC-MS, M/Z (ESI): 234.1 $[M+H]^+$

Step 2: Synthesis of 3-(2-cyano-4-((2-methyl-1H-imidazol-1-yl)methyl)phenyl)-5-isobutyl-N-((3,3,3-trifluoropropyl)carbamoyl)thiophene-2-sulfonamide

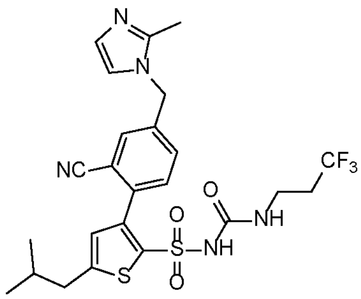

**I-18**

3-(2-cyano-4-((2-methyl-1H-imidazol-1-yl)methyl)phenyl)-5-isobutylthiophene-2-sulfonamide (200 mg, 482 μmol) and 1,8-diazabicyclo[5.4.0]undec-7-ene (220 mg, 1.45 mmol) were dissolved in acetonitrile (5 mL), and phenyl (3,3,3-trifluoropropyl)carbamate (124 mg, 531 μmol) was added, and the mixture was stirred and reacted at 25°C for 1 hour. After the reaction was completed, the reaction system was added with water (20 mL) to quench the reaction, and then the reaction system was extracted with ethyl acetate (25 mL×2). The organic phases were combined, dried over anhydrous sodium sulfate, filtered, and concentrated. The crude product was separated and purified by high performance liquid chromatography (column: Waters Xbridge 150*25 mm*5um; solvent: A=water + 0.05 volume of ammonia water (30%), B=acetonitrile; gradient: 5% to 35%, 8 minutes), and concentrated and dried to obtain 3-(2-cyano-4-((2-methyl-1H-imidazol-1-yl)methyl)phenyl)-5-isobutyl-N-((3,3,3-trifluoropropyl)carbamoyl)thiophene-2-sulfonamide (36.5 mg, yield 18.3%).

$^1$H NMR (400 MHz, DMSO_$d_6$) $\delta$ 7.72 (s, 1H), 7.63-7.65 (m, 1H), 7.47-7.49 (m, 1H), 7.33 (s, 1H), 7.00 (s, 1H), 6.89 (s, 1H), 6.22 (s, 1H), 5.39 (s, 2H), 3.15-3.17 (m, 2H), 2.70 (d, 2H), 2.36 (s, 3H), 2.32-2.34 (m, 2H), 1.85-1.89 (m, 1H), 0.93 (d, 6H).
LC-MS, M/Z (ESI): 554.3 $[M+H]^+$

**Example 17:** Preparation of target compound **I-19**

5-isobutyl-3-(2-methyl-4-((2-methyl-1H-imidazol-1-yl)methyl)phenyl)-N-((pyridin-2-ylmethyl)carbamoyl)thiophene-2-sulfonamide

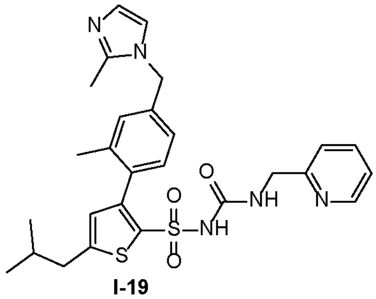

**I-19**

The synthetic scheme of the target compound 1-19 was as follows:

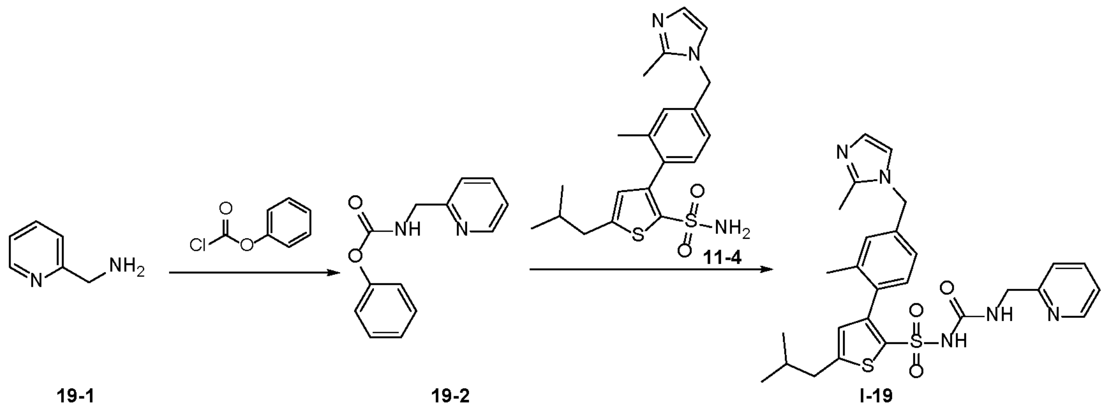


**19-1** **19-2** **I-19**

Step 1: Synthesis of phenyl (pyridin-2-ylmethyl)carbamate

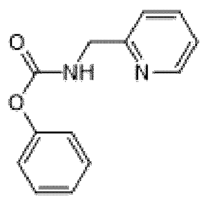

**19-2**

Pyridin-2-ylmethylamine (3.00 g, 27.7 mmol) was dissolved in dichloromethane (30 mL), and then triethylamine (2.81 g, 27.7 mmol) and phenyl chloroformate (5.21 g, 33.3 mmol) were added. The mixture reacted at 25°C for 1 hour. The reaction solution was poured into water (50 mL), and extracted with ethyl acetate (50 mL) for three times. The organic phases were collected, dried over anhydrous sodium sulfate, filtered, and concentrated to obtain phenyl (pyridin-2-ylmethyl)carbamate (3.00 g, yield 47.4%).
LC-MS, M/Z (ESI): 229.1 [M+H]$^+$

Step 2: Synthesis of 5-isobutyl-3-(2-methyl-4-((2-methyl-1H-imidazol-1-yl)methyl)phenyl)-N-((pyridin-2-ylmethyl)carbamoyl)thiophene-2-sulfonamide

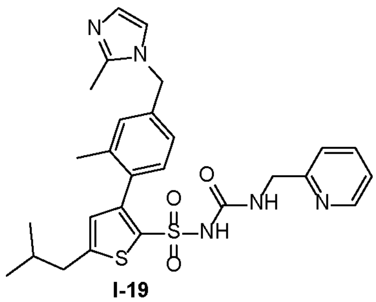

**I-19**

5-isobutyl-3-(2-methyl-4-((2-methyl-1H-imidazol-1-yl)methyl)phenyl)thiophene-2-sulfonamide (40.0 mg, 99.1 μmol) and phenyl (pyridin-2-ylmethyl)carbamate (22.6 mg, 99.1 μmol) were dissolved in acetonitrile (4.00 mL), 1,8-diazabicyclo[5.4.0]undec-7-ene (15.1 mg, 99.1 μmol) was added, and the mixture was stirred and reacted at 60°C for 1 hour. After the reaction was completed, water (10 mL) was added to quench the reaction, and then the reaction system was extracted with dichloromethane (20 mL×2). The organic phases were combined, dried over anhydrous sodium sulfate, filtered, and concentrated. The crude product was separated and purified by high performance liquid chromatography (column: Waters Xbridge 150*25 mm*5 um; solvent: A=water + 0.05 volume of ammonia water (30%), B=acetonitrile; gradient: % to 35%, 8 minutes), and concentrated and dried to obtain 5-isobutyl-3-(2-methyl-4-((2-methyl-1H-imidazol-1-yl)methyl)phenyl)-N-((pyridin-2-ylmethyl)carbamoyl)thiophene-2-sulfonamide (9.25 mg, yield 17.4%).

$^1$H NMR (400 MHz, $CDCl_3$) $\delta$ 8.33 (s, 1H), 7.47-7.49 (m, 1H), 7.09-7.14 (m, 2H), 7.02-7.03 (m, 1H), 6.76-6.84 (m, 4H), 6.43 (s, 1H), 4.91 (s, 2H), 4.13 (s, 2H), 2.51 (d, 2H), 2.25 (s, 3H), 1.93 (s, 3H), 1.82-1.86 (m, 1H), 0.87 (d, 6H).
LC-MS, M/Z (ESI): 538.2 $[M+H]^+$

**Example 18:** Preparation of target compound **I-21**

Methyl ((5-isobutyl-3-(3-methyl-4-((2-methyl-1H-imidazol-1-yl)methyl)phenyl)thiophen-2-yl)sulfonyl)carbamate

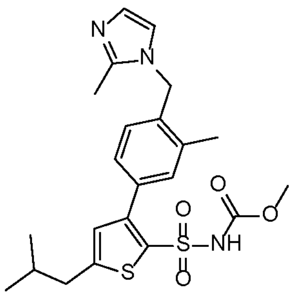

**I-21**

The synthetic scheme of the target compound 1-21 was as follows:

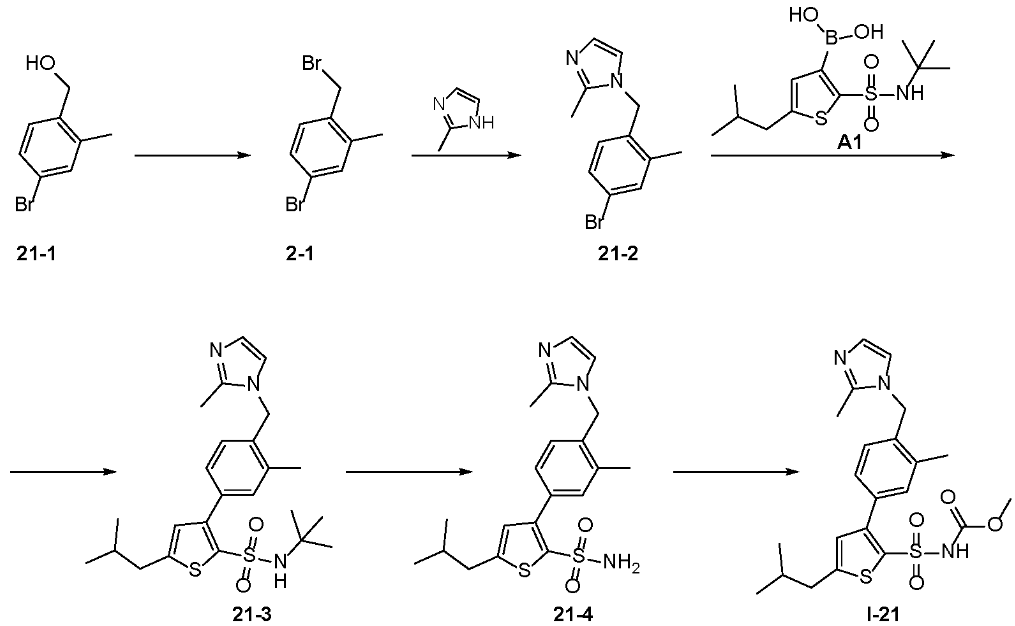


Step 1: Synthesis of 4-bromo-1-(bromomethyl)-2-methylbenzene

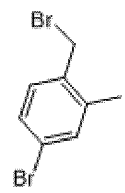

2-1

(4-bromo-2-methyl-phenyl)methanol (1 g, 4.97 mmol) was dissolved in dichloromethane (20 mL), carbon tetrabromide (1.98 g, 5.97 mmol) was added, and then triphenylphosphine (1.57 g, 5.97 mmol) was added. The reaction solution reacted at 20°C for 2 hours. The reaction solution was then poured into water (50 mL), and extracted with ethyl acetate (100 mL) for three times. The organic phases were collected, dried over anhydrous sodium sulfate, filtered, and concentrated. The crude product was separated and purified by a silica gel column (petroleum ether: ethyl acetate (V/V)= (50:1) to (20:1)) to obtain 4-bromo-1-(bromomethyl)-2-methylbenzene (1.1 g, yield 83.8%).

Step 2: Synthesis of 1-(4-bromo-2-methylbenzyl)-2-methyl-1H-imidazole

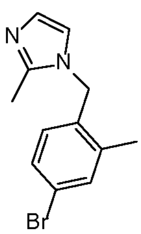

21-2

4-bromo-1-(bromomethyl)-2-methylbenzene (1.00 g, 3.79 mmol) and 2-methyl-1H-imidazole (311 mg, 3.79 mmol) were dissolved in N,N-dimethylformamide (10.0 mL), potassium carbonate (1.57 g, 11.4 mmol) was added, and the mixture reacted at 50°C for 2 hours. The reaction solution was poured into water (20 mL), and extracted with ethyl acetate (50 mL) for three times. The organic phases were collected, dried over anhydrous sodium sulfate, filtered, and

concentrated. The crude product was separated and purified by a silica gel column (petroleum ether: ethyl acetate (V/V)=(50:1) to (1:1)) to obtain 1-(4-bromo-2-methylbenzyl)-2-methyl-1H-imidazole (800 mg, yield 79.6%).
LC-MS, M/Z (ESI): 265.2 $[M+H]^+$.

Step 3: Synthesis of N-(tert-butyl)-5-isobutyl-3-(3-methyl-4-((2-methyl-1H-imidazol-1 -yl)methyl)phenyl)thiophene-2-sulfonamide

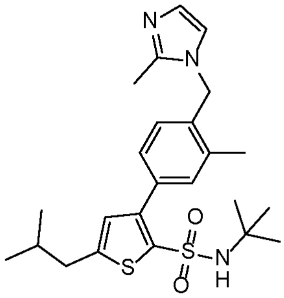

**21-3**

(2-(N-(tert-butyl)sulfamoyl)-5-isobutylthiophen-3-yl)boronic acid (260 mg, 814 μmol) was dissolved in tetrahydrofuran (5.00 mL) and water (5.00 mL). 1-(4-bromo-2-methylbenzyl)-2-methyl-1H-imidazole (216 mg, 814 μmol) was added, and then potassium phosphate (864 mg, 4.07 mmol) and Xphos Pd G4 (70.1 mg, 81.4 μmol) were added. The mixture was stirred and reacted at 60°C under nitrogen protection for 1 hour. The reaction solution was concentrated to obtain a crude product, and the crude product was separated and purified by a silica gel column (petroleum ether: ethyl acetate (V/V)=(50:1) to (1:1)) to obtain N-(tert-butyl)-5-isobutyl-3-(3-methyl-4-((2-methyl-1H-imidazol-1-yl)methyl)phenyl)thiophene-2-sulfonamide (300 mg, yield 80.1%).
LC-MS, M/Z (ESI): 460.4 $[M+H]^+$.

Step 4: Synthesis of 5-isobutyl-3-(3-methyl-4-((2-methyl-1H-imidazol-1-yl)methyl)phenyl)thiophene-2-sulfonamide

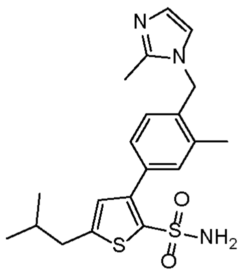

**21-4**

N-(tert-butyl)-5-isobutyl-3-(3-methyl-4-((2-methyl-1H-imidazol-1-yl)methyl)phenyl)thiophene-2-sulfonamide (300 mg, 653 μmol) was dissolved in trifluoroacetic acid (3 mL) and dichloromethane (3 mL), and the mixture reacted at 25°C for 1 hour. The reaction solution was poured into water (10 mL), and pH of the reaction solution was adjusted to 9 with saturated sodium bicarbonate. The reaction solution was then extracted with dichloromethane (20 mL). The organic phases were collected, dried over anhydrous sodium sulfate, filtered, and concentrated. The crude product was separated and purified by a silica gel column (petroleum ether: ethyl acetate (V/V)=(10:1) to (1:1)) to obtain 5-isobutyl-3-(3-methyl-4-((2-methyl-1H-imidazol-1-yl)methyl)phenyl)thiophene-2-sulfonamide (200 mg, yield 75.9%).
LC-MS, M/Z (ESI): 404.6 $[M+H]^+$.

Step 5: Synthesis of methyl ((5-isobutyl-3-(3-methyl-4-((2-methyl-1H-imidazol-1-yl)methyl)phenyl)thiophen-2-yl)sulfonyl)carbamate

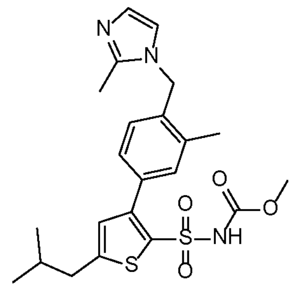

**I-21**

5-isobutyl-3-(3-methyl-4-((2-methyl-1H-imidazol-1-yl)methyl)phenyl)thiophene-2-sulfonamide (100 mg, 248 μmol) and N,N-diisopropylethylamine (96.1 mg, 743 μmol) were dissolved in dichloromethane (10.0 mL), and methyl chloroformate (141 mg, 1.49 mmol) was added. The mixture was stirred and reacted at 0°C for 1 hour. The reaction solution was concentrated, separated and purified by high performance liquid chromatography (column: 3_Phenomenex Luna C1875*30mm*3um; solvent: A=water + 0.05 volume of formic acid (99%), B=acetonitrile; gradient: 12% to 42%, 15 minutes), and concentrated and dried to obtain methyl ((5-isobutyl-3-(3-methyl-4-((2-methyl-1H-imidazol-1-yl)methyl)phenyl)thiophen-2-yl)sulfonyl)carbamate (100 mg, yield 87.4%).

$^1$H NMR ($CDCl_3$, 400 MHz) δ: 7.50 (d, 1H), 7.44 (s, 1H), 6.86 (s, 1H), 6.73-6.77 (m, 2H), 6.64 (s, 1H), 5.07 (s, 2H), 3.61 (s, 3H), 2.70 (d, 2H), 2.60 (s, 3H), 2.19 (s, 3H), 1.93-2.00 (m, 1H), 1.00 (d, 6H).
LC-MS, M/Z (ESI): 462.6 $[M+H]^+$.

**Example 19:** Preparation of target compound **I-22**

N-(ethylcarbamoyl)-5-isobutyl-3-(3-methyl-4-((2-methyl-1H-imidazol-1-yl)methyl)phenyl)thiophene-2-sulfonamide

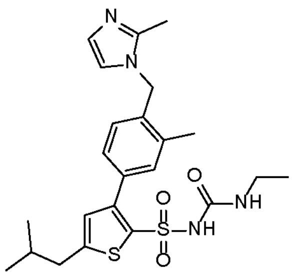

**I-22**

The synthetic scheme of the target compound **I-22** was as follows:

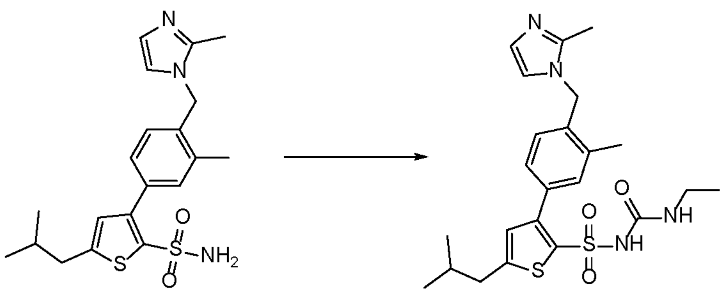

**21-4** **I-22**

Step 1: Synthesis of N-(ethylcarbamoyl)-5-isobutyl-3-(3-methyl-4-((2-methyl-1H-imidazol-1 -yl)methyl)phenyl)thiophene-2-sulfonamide

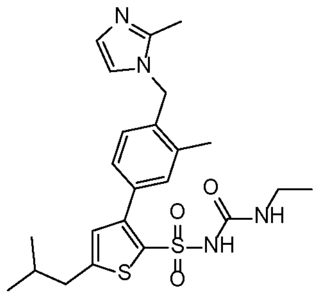

I-22

5-isobutyl-3-(3-methyl-4-((2-methyl-1H-imidazol-1-yl)methyl)phenyl)thiophene-2-sulfonamide (100 mg, 248 μmol) and triethylamine (50.2 mg, 496 μmol) were dissolved in dichloromethane (5 mL), ethyl isocyanate (17.6 mg, 248 μmol) was added, and the mixture was stirred and reacted at 0°C for 1 hour. The reaction solution was concentrated, separated and purified by high performance liquid chromatography (column: Waters Xbridge 150*25mm*5um; solvent: A=water + 0.05 volume of ammonia water (30%), B=acetonitrile; gradient: 3% to 33%, 9 minutes), and concentrated and dried to obtain N-(ethylcarbamoyl)-5-isobutyl-3-(3-methyl-4-((2-methyl-1H-imidazol-1-yl)methyl)phenyl)thiophene-2-sulfonamide (45.1 mg, yield 36.4%).

$^1$H NMR (400 MHz, DMSO_$d_6$) $\delta$ 7.41-7.43 (m, 2H), 7.10 (s, 1H), 6.83-6.84 (m, 2H), 6.49 (d, 1H), 5.17 (s, 2H), 2.89-2.92 (m, 2H), 2.66 (d, 2H), 2.30 (s, 3H), 2.23 (s, 3H), 1.84-1.86 (m, 1H), 1.14 (t, 3H), 0.93 (d, 6H).
LC-MS, M/Z (ESI): 475.2 $[M+H]^+$

**Example 20:** Preparation of target compound **I-23**

N-((2-hydroxyethyl)carbamoyl)-5-isobutyl-3-(3-methyl-4-((2-methyl-1H-imidazol-1 -yl)methyl)phenyl)thiophene-2-sulfonamide

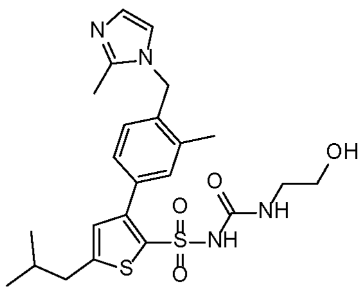

I-23

The synthetic scheme of the target compound 1-23 was as follows:

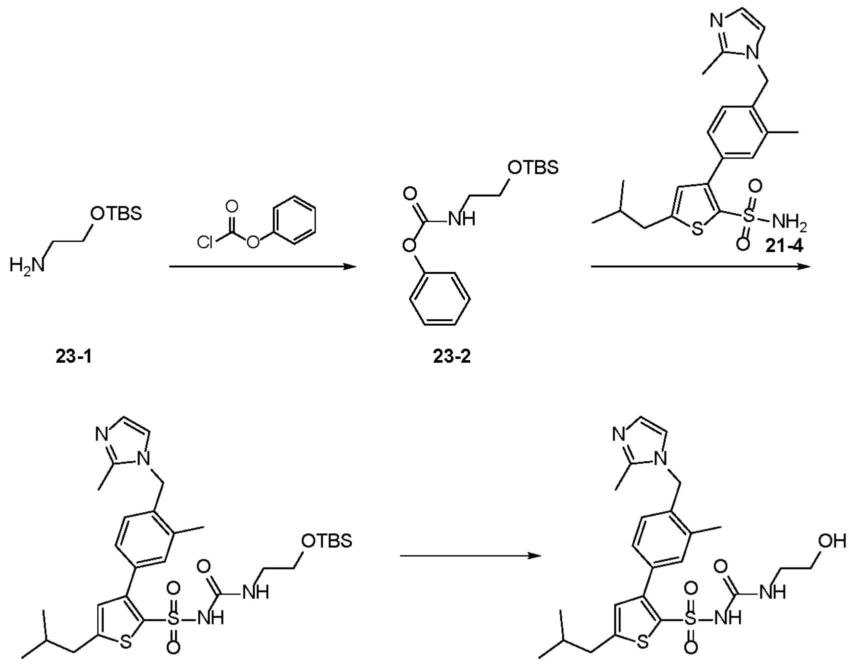

23-1 23-2 21-4 23-3 I-23

Step 1: Synthesis of phenyl (2-((tert-butyldimethylsilyl)oxy)ethyl)carbamate

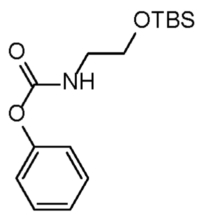

23-2

2-((tert-butyldimethylsilyl)oxy)ethane-1-amine (1.00 g, 5.70 mmol) was dissolved in dichloromethane (10 mL), and then triethylamine (1.15 g, 11.4 mmol) and phenyl chloroformate (1.07 g, 6.84 mmol) were added. The mixture reacted at 25°C for 1 hour. The reaction solution was poured into water (50 mL), and extracted with dichloromethane (50 mL) for three times. The organic phases were collected, dried over anhydrous sodium sulfate, filtered, and concentrated to obtain phenyl (2-((tert-butyldimethylsilyl)oxy)ethyl)carbamate (800 mg, yield 47.5%).
LC-MS, M/Z (ESI): 296.2 $[M+H]^+$

Step 2: Synthesis of N-((2-((tert-butyldimethylsilyl)oxy)ethyl)carbamoyl)-5-isobutyl-3-(3-methyl-4-((2-methyl-1H-imidazol-1-yl)methyl)phenyl)thiophene-2-sulfonamide

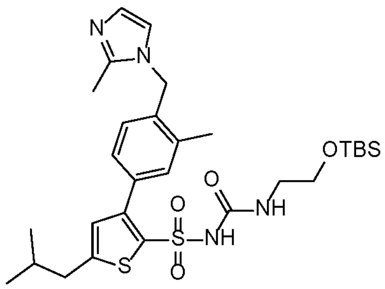

**23-3**

5-isobutyl-3-(3-methyl-4-((2-methyl-1H-imidazol-1-yl)methyl)phenyl)thiophene-2-sulfonamide (150 mg, 372 μmol) and 1,8-diazabicyclo[5.4.0]undec-7-ene (170 mg, 1.12 mmol) were dissolved in acetonitrile (5 mL), and phenyl (2-((tertbutyldimethylsilyl)oxy)ethyl)carbamate (132 mg, 446 μmol) was added. The mixture was stirred and reacted at 25°C for 1 hour. After the reaction was completed, water (20 mL) was added to quench the reaction, and then the reaction system was extracted with ethyl acetate (25 mL×2). The organic phases were combined, dried over anhydrous sodium sulfate, filtered, and concentrated. The crude product was separated and purified by high performance liquid chromatography (column: Waters Xbridge 150*25mm*5um; solvent: A=water + 0.05 volume of ammonia water (30%), B=acetonitrile; gradient: 15% to 45%, 8 minutes), and concentrated and dried to obtain N-((2-((tert-butyldimethylsilyl)oxy)ethyl)carbamoyl)-5-isobutyl-3-(3-methyl-4-((2-methyl-1H-imidazol-1-yl)methyl)phenyl)thiophene-2-sulfonamide (150 mg, yield 66.7%).
LC-MS, M/Z (ESI): 605.1 $[M+H]^+$

Step 3: Synthesis of N-((2-hydroxyethyl)carbamoyl)-5-isobutyl-3-(3-methyl-4-((2-methyl-1H-imidazol-1-yl)methyl)phenyl)thiophene-2-sulfonamide

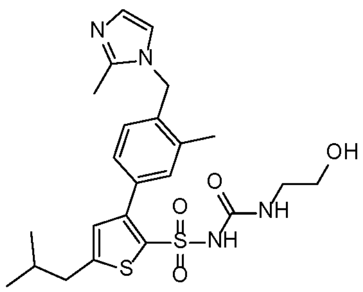

**I-23**

N-((2-((tert-butyldimethylsilyl)oxy)ethyl)carbamoyl)-5-isobutyl-3-(3-methyl-4-((2-methyl-1H-imidazol-1-yl)methyl)phenyl)thiophene-2-sulfonamide (100 mg, 165 μmol) was dissolved in ethyl acetate (2 mL), a solution of hydrochloric acid in ethyl acetate (4 M, 1 mL) was added, and the mixture was stirred and reacted at 25°C for 1 hour. The reaction solution was concentrated. The residue was beat to slurry with dichloromethane (2 mL) and filtered to obtain N-((2-hydroxyethyl)carbamoyl)-5-isobutyl-3-(3-methyl-4-((2-methyl-1H-imidazol-1-yl)methyl)phenyl)thiophene-2-sulfonamide (12.0 mg, yield 13.6%).

$^1$H NMR (400 MHz, DMSO_$d_6$) $\delta$ 7.64 (s, 1H), 7.52 (s, 1H), 7.36-7.39 (m, 2H), 6.90 (s, 1H), 6.83 (d, 1H), 6.37 (t, 1H), 5.43 (s, 2H), 4.73 (brs, 1H), 3.30-3.31 (m, 2H), 2.97-3.01 (m, 2H), 2.71 (d, 2H), 2.58 (s, 3H), 2.33 (s, 3H), 1.86-1.90 (m, 1H), 0.93 (d, 6H).
LC-MS, M/Z (ESI): 491.3 $[M+H]^+$

**Example 21:** Preparation of target compound **I-24**

Methyl ((3-(3-cyano-4-((2-methyl-1H-imidazol-1-yl)methyl)phenyl)-5-isobutylthiophen-2-yl)sulfonyl)carbamate

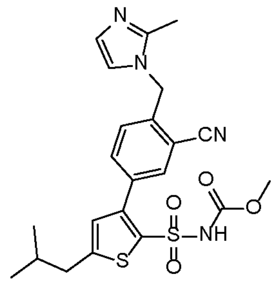

I-24

The synthetic scheme of the target compound **I-24** was as follows:

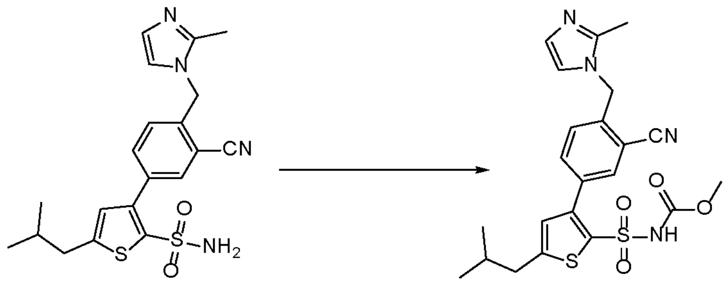

12-4 I-24

Step 1: Synthesis of methyl ((3-(3-cyano-4-((2-methyl-1H-imidazol-1-yl)methyl)phenyl)-5-isobutylthiophen-2-yl)sulfonyl)carbamate

3-(3-cyano-4-((2-methyl-1H-imidazol-1-yl)methyl)phenyl)-5-isobutylthiophene-2-sulfonamide (100 mg, 241 μmol) and N,N-diisopropylethylamine (93.5 mg, 724 μmol) were dissolved in dichloromethane (5 mL), and methyl chloroformate (137 mg, 248 μmol) was added. The mixture was stirred and reacted at 0°C for 1 hour. The reaction solution was concentrated, separated and purified by high performance liquid chromatography (column: Waters Xbridge 150*25mm*5um; solvent: A=water + 0.05 volume of aqueous ammonia (30%), B=acetonitrile; gradient: 5% to 35%, 8 minutes), and concentrated and dried to obtain methyl ((3-(3-cyano-4-((2-methyl-1H-imidazol-1-yl)methyl)phenyl)-5-isobutylthiophen-2-yl)sulfonyl)carbamate (93.3 mg, yield 81.8%).

$^1$H NMR (400 MHz, $CDCl_3$) $\delta$ 8.01 (d, 1H), 7.87 (dd, 1H), 7.28 (d, 1H), 6.80 (d, 1H), 6.66 (s, 1H), 6.30 (s, 1H), 5.12 (s, 2H), 3.52 (s, 3H), 2.72 (s, 3H), 2.63 (d, 2H), 1.85-1.90 (m, 1H), 0.92 (d, 6H).
LC-MS, M/Z (ESI): 473.2 $[M+H]^+$

**Example 22:** Preparation of target compound **I-25**

Methyl ((3-(4-((2-(tert-butyl)-1H-imidazol-1-yl)methyl)-2-methylphenyl)-5-isobutylthiophen-2-yl)sulfonyl)carbamate

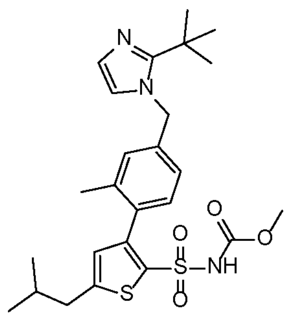

I-25

The synthetic scheme of the target compound 1-25 was as follows:

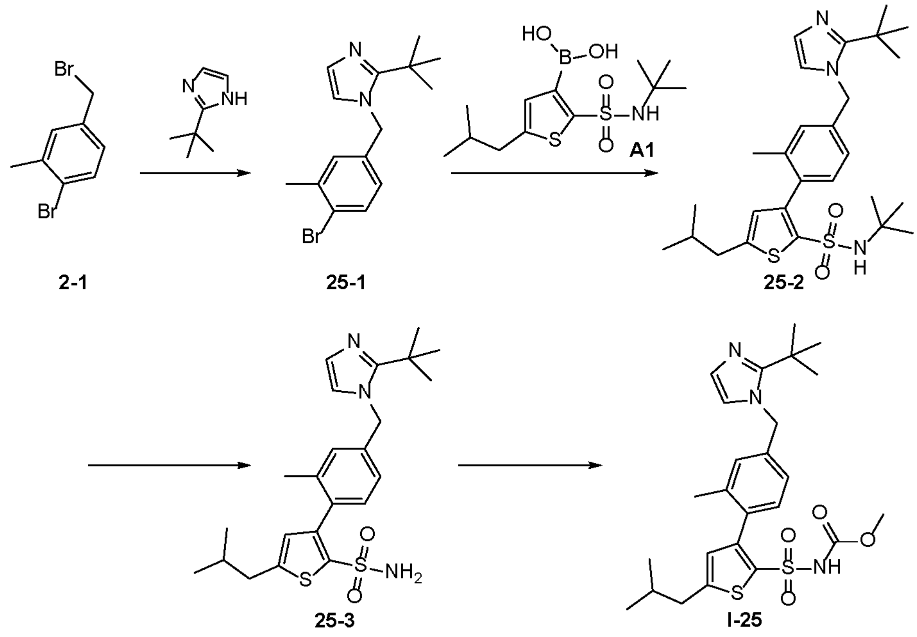

A1

2-1 25-1 25-2

25-3 I-25

Step 1: Synthesis of 1-(4-bromo-3-methylbenzyl)-2-(tert-butyl)-1H-imidazole

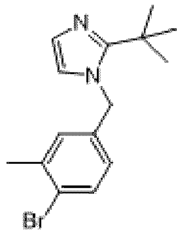

25-1

1-bromo-4-(bromomethyl)-2-methylbenzene (500 mg, 1.89 mmol) and 2-(tert-butyl)-1H-imidazole (235 mg, 1.89 mmol) were dissolved in N,N-dimethylformamide (10 mL), potassium carbonate (785 mg, 5.68 mmol) was added, and the mixture reacted at 50°C for 2 hours. The reaction solution was poured into water (25 mL), and extracted with ethyl acetate (50 mL) for three times. The organic phases were collected, dried over anhydrous sodium sulfate, filtered, and concentrated. The crude product was separated and purified by a silica gel column (petroleum ether: ethyl acetate (V/V)=(50:1) to (1:1)) to obtain 1-(4-bromo-3-methylbenzyl)-2-(tert-butyl)-1H-imidazole (400 mg, yield 68.7%).
LC-MS, M/Z (ESI): 309.2 $[M+H]^+$

Step 2: Synthesis of N-(tert-butyl)-3-(4-((2-(tert-butyl)-1H-imidazol-1-yl)methyl)-2-methylphenyl)-5-isobutylthiophene-2-sulfonamide

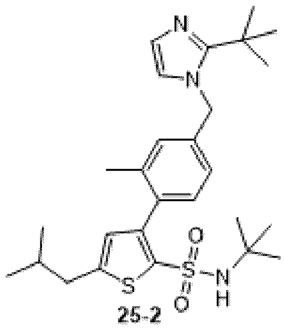

25-2

(2-(N-(tert-butyl)sulfamoyl)-5-isobutylthiophen-3-yl)boronic acid (250 mg, 783 μmol) was dissolved in tetrahydrofuran (3 mL) and water (3 mL). 1-(4-bromo-3-methylbenzyl)-2-(tert-butyl)-1H-imidazole (241 mg, 783 μmol) was added, and potassium phosphate (830 mg, 3.92 mmol) and XPhos Pd G4 (67.4 mg, 78.3 μmol) were added. The mixture was stirred and reacted at 60°C under nitrogen protection for 4 hours. The reaction solution was concentrated to obtain a crude product, and the crude product was separated and purified by a silica gel column (petroleum ether: ethyl acetate (V/V)=(50:1) to (0:1)) to obtain N-(tert-butyl)-3-(4-((2-(tert-butyl)-1H-imidazol-1-yl)methyl)-2-methylphenyl)-5-isobutylthiophene-2-sulfonamide (300 mg, yield 76.4%).
LC-MS, M/Z (ESI): 502.3 $[M+H]^+$

Step 3: Synthesis of 3-(4-((2-(tert-butyl)-1H-imidazol-1-yl)methyl)-2-methylphenyl)-5-isobutylthiophene-2-sulfonamide

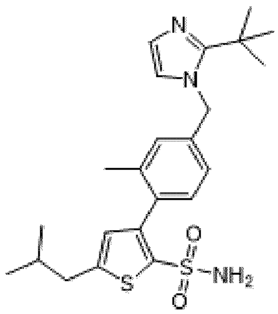

25-3

N-(tert-butyl)-3-(4-((2-(tert-butyl)-1H-imidazol-1-yl)methyl)-2-methylphenyl)-5-isobutylthiophene-2-sulfonamide (200 mg, 399 μmol) was dissolved in trifluoroacetic acid (2 mL) and dichloromethane (2 mL), and the mixture reacted at 25°C for 8 hours. The reaction solution was poured into water (10 mL), and then extracted with dichloromethane (50 mL) for three times. The organic phases were collected, dried over anhydrous sodium sulfate, filtered, and concentrated. The crude product was separated and purified by a silica gel column (petroleum ether: ethyl acetate (V/V)=(10:1) to (1:1)) to obtain 3-(4-((2-(tert-butyl)-1H-imidazol-1-yl)methyl)-2-methylphenyl)-5-isobutylthiophene-2-sulfonamide (150 mg, yield 84.4%).
LC-MS, M/Z (ESI): 446.2 $[M+H]^+$

Step 4: Synthesis of methyl ((3-(4-((2-(tert-butyl)-1H-imidazol-1-yl)methyl)-2-methylphenyl)-5-isobutylthiophen-2-yl)sulfonyl)carbamate

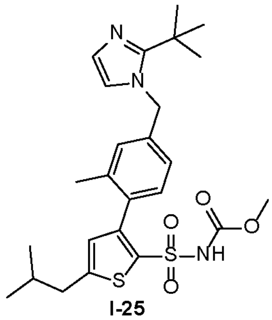

I-25

3-(4-((2-(tert-butyl)-1H-imidazol-1-yl)methyl)-2-methylphenyl)-5-isobutylthiophene-2-sulfonamide (100 mg, 224 μmol) and N,N-diisopropylethylamine (87.0 mg, 673 μmol) were dissolved in dichloromethane (2 mL), methyl chloroformate (137 mg, 1.45 mmol) was added, and the mixture was stirred and reacted at 0°C for 1 hour. The reaction solution was concentrated, separated and purified by high performance liquid chromatography (column: Waters Xbridge 150*25mm*5um; solvent: A=water + 0.05 volume of aqueous ammonia (30%), B=acetonitrile; gradient: 8% to 38%, 9 minutes), and concentrated and dried to obtain methyl ((3-(4-((2-(tert-butyl)-1H-imidazol-1-yl)methyl)-2-methylphenyl)-5-isobutylthiophen-2-yl)sulfonyl)carbamate (38.7 mg, yield 38.7%).

$^1$H NMR (400 MHz, DMSO $d_6$) $\delta$ 7.15 (d, 1H), 7.01 (s, 1H), 6.86-6.89 (m, 2H), 6.75-6.78 (m, 1H), 6.51 (s, 1H), 5.36 (s, 2H), 3.25 (s, 3H), 3.17 (s, 3H), 2.61 (d, 2H), 1.82-1.87 (m, 1H), 1.34 (s, 9H), 0.93 (d, 6H).
LC-MS, M/Z (ESI): 504.4 $[M+H]^+$

**Example 23:** Preparation of target compound **I-26**

Methyl ((3-(4-((2-(tert-butyl)-1H-imidazol-1-yl)methyl)phenyl)-5-isobutyl-4-methylthiophen-2-yl)sulfonyl)carbamate

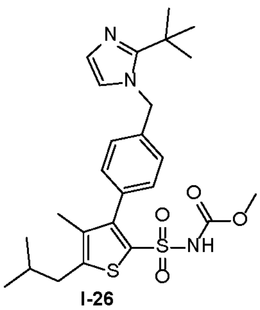

I-26

The synthetic scheme of the target compound 1-26 was as follows:

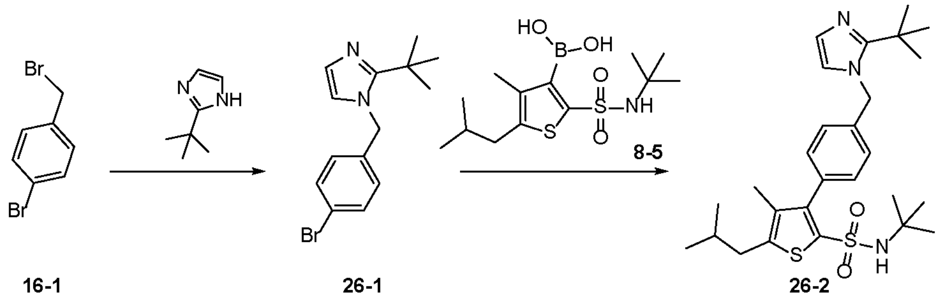

16-1 26-1 26-2

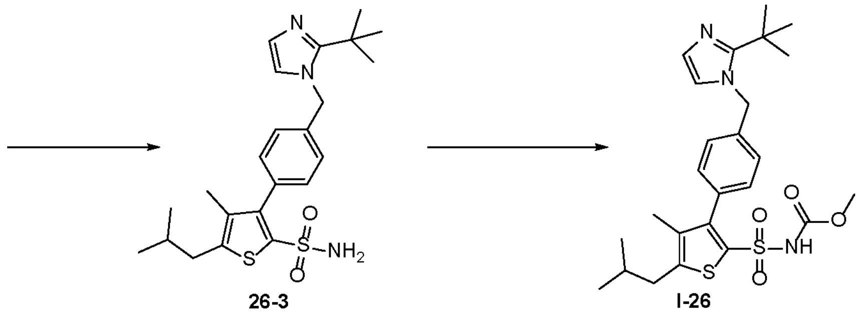

26-3 I-26

Step 1: Synthesis of 1-(4-bromobenzyl)-2-(tert-butyl)-1H-imidazole

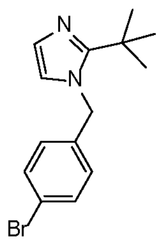

26-1

1-bromo-4-(bromomethyl)benzene (500 mg, 2.00 mmol) and 2-tert-butyl-1H-imidazole (248 mg, 2.00 mmol) were dissolved in N,N-dimethylformamide (5 mL), and then potassium carbonate (830 mg, 6.00 mmol) was added. The mixture reacted at 55°C for 1 hour. The reaction solution was poured into water (25 mL), and extracted with ethyl acetate (50 mL) for three times. The organic phases were collected, dried over anhydrous sodium sulfate, filtered, and concentrated. The crude product was separated and purified by a silica gel column (petroleum ether: ethyl acetate (V/V)=(50:1) to (1:1)) to obtain 1-(4-bromobenzyl)-2-(tert-butyl)-1H-imidazole (400 mg, yield 68.2%).
LC-MS, M/Z (ESI): 293.1 $[M+H]^+$

Step 2: Synthesis of N-(tert-butyl)-3-(4-((2-(tert-butyl)-1H-imidazol-1-yl)methyl)phenyl)-5-isobutyl-4-methylthiophene-2-sulfonamide

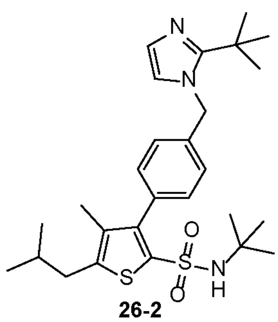

26-2

(2-(N-(tert-butyl)sulfamoyl)-5-isobutyl-4-methylthiophen-3-yl)boronic acid (250 mg, 750 μmol) was dissolved in

tetrahydrofuran (5 mL) and water (2 mL). 1-(4-bromobenzyl)-2-(tert-butyl)-1H-imidazole (220 mg, 750 μmol) was added, and potassium phosphate (796 mg, 3.75 mmol) and XPhos Pd G4 (64.6 mg, 75.0 μmol) were added. Then, the mixture was stirred and reacted at 60°C under nitrogen protection for 2 hours. The reaction solution was concentrated to obtain a crude product, and the crude product was separated and purified by a silica gel column (petroleum ether: ethyl acetate (V/V)=(50: 1) to (0:1)) to obtain N-(tert-butyl)-3-(4-((2-(tert-butyl)-1H-imidazol-1-yl)methyl)phenyl)-5-isobutyl-4-methylthiophene-2-sulfonamide (250 mg, yield 66.4%).
LC-MS, M/Z (ESI): 502.3 $[M+H]^+$

Step 3: Synthesis of 3-(4-((2-(tert-butyl)-1H-imidazol-1-yl)methyl)phenyl)-5-isobutyl-4-methylthiophene-2-sulfonamide

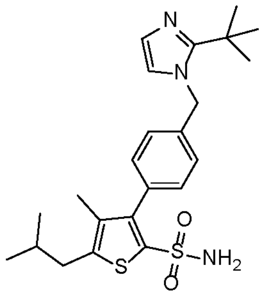

**26-3**

N-(tert-butyl)-3-(4-((2-(tert-butyl)-1H-imidazol-1-yl)methyl)phenyl)-5-isobutyl-4-methylthiophene-2-sulfonamide (200 mg, 399 μmol) was dissolved in trifluoroacetic acid (5 mL) and dichloromethane (4 mL), and the mixture reacted at 30°C for 2 hours. The reaction solution was poured into water (10 mL), and then extracted with dichloromethane (50 mL). The organic phases were collected, dried over anhydrous sodium sulfate, filtered, and concentrated. The crude product was separated and purified by a silica gel column (petroleum ether: ethyl acetate (V/V)=(10:1) to (1:1)) to obtain 3-(4-((2-(tert-butyl)-1H-imidazol-1-yl)methyl)phenyl)-5-isobutyl-4-methylthiophene-2-sulfonamide (150 mg, yield 84.4%).
LC-MS, M/Z (ESI): 446.2 $[M+H]^+$

Step 4: Synthesis of methyl ((3-(4-((2-(tert-butyl)-1H-imidazol-1-yl)methyl)phenyl)-5-isobutyl-4-methylthiophen-2-yl) sulfonyl)carbamate

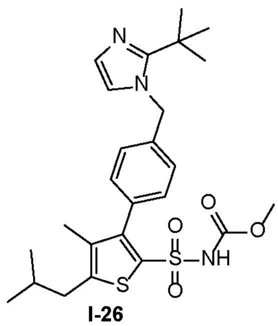

**I-26**

3-(4-((2-(tert-butyl)-1H-imidazol-1-yl)methyl)phenyl)-5-isobutyl-4-methylthiophene-2-sulfonamide (100 mg, 224 μmol) and N,N-diisopropylethylamine (87.0 mg, 673 μmol) were dissolved in dichloromethane (2.00 mL), methyl chloroformate (127 mg, 1.35 mmol) was added, and the mixture was stirred and reacted at 0°C for 1 hour. The reaction solution was concentrated, separated and purified by high performance liquid chromatography (column: Waters Xbridge 150*25mm*5um; solvent: A=water + 0.05 volume of formic acid (99%), B=acetonitrile; gradient: 20% to 50%, 15 minutes), and concentrated and dried to obtain methyl ((3-(4-((2-(tert-butyl)-1H-imidazol-1-yl)methyl)phenyl)-5-isobutyl-4-methylthiophen-2-yl)sulfonyl)carbamate (330 mg, yield 33.0%).

$^1$H NMR (400 MHz, $CDCl_3$) δ 7.24 (d, 2H), 7.13 (d, 2H), 6.95 (s, 1H), 6.78 (s, 1H), 5.38 (s, 2H), 3.68 (s, 3H), 2.68 (d, 2H),

1.92-1.97 (m, 1H), 1.85 (s, 3H), 1.45 (s, 9H), 1.01 (d, 6H).
LC-MS, M/Z (ESI): 504.2 [M+H]+

**Example 24:** Preparation of target compound **I-27**

Methyl ((3-(3-cyano-4-((2-isopropyl-1H-imidazol-1-yl)methyl)phenyl)-5-isobutylthiophen-2-yl)sulfonyl)carbamate

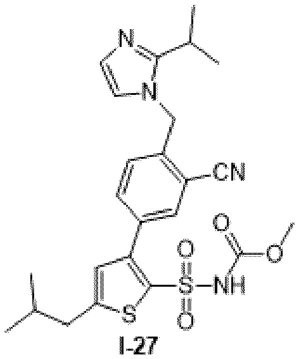

I-27

The synthetic scheme of the target compound **I-27** was as follows:

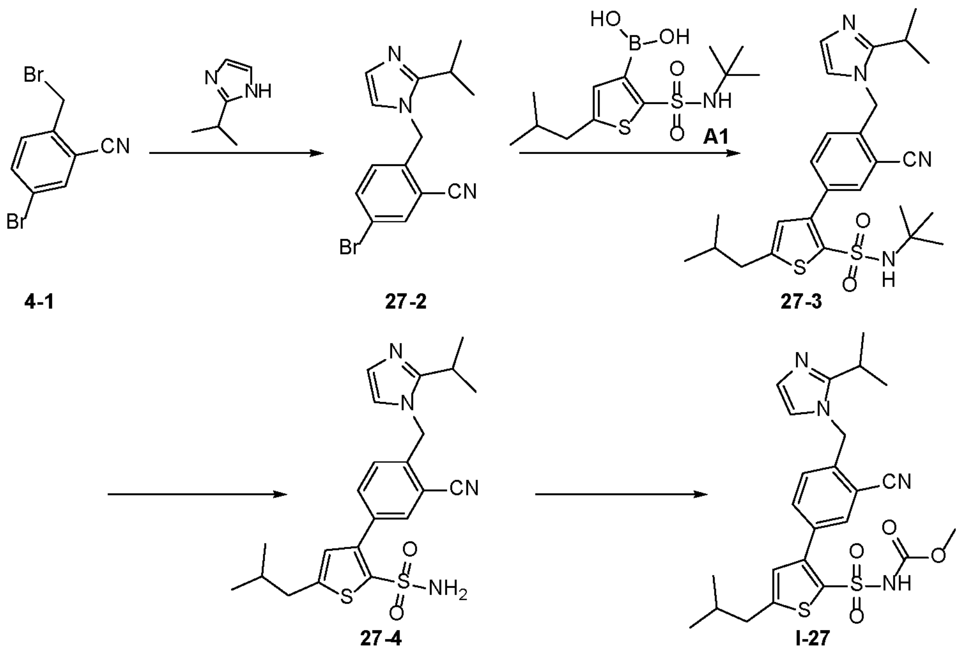


Step 1: Synthesis of 5-bromo-2-((2-isopropyl-1H-imidazol-1-yl)methyl)benzonitrile

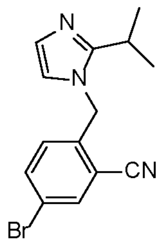

27-2

5-bromo-2-(bromomethyl)benzonitrile (200 mg, 727 μmol) and 2-isopropyl-1H-imidazole (80.1 mg, 727 μmol) were dissolved in N,N-dimethylformamide (10 mL), potassium carbonate (302 mg, 2.18 mmol) was added, and the mixture reacted at 50°C for 2 hours. The reaction solution was poured into water (25 mL), and extracted with ethyl acetate (50 mL). The organic phases were collected, dried over anhydrous sodium sulfate, filtered, and concentrated. The crude product was separated and purified by a silica gel column (petroleum ether: ethyl acetate (V/V)=(50:1) to (1:1)) to obtain 5-bromo-2-((2-isopropyl-1H-imidazol-1-yl)methyl)benzonitrile (150 mg, yield 67.8%).
LC-MS, M/Z (ESI): 304.0 $[M+H]^+$

Step 2: Synthesis of N-(tert-butyl)-3-(3-cyano-4-((2-isopropyl-1H-imidazol-1-yl)methyl)phenyl)-5-isobutylthiophene-2-sulfonamide

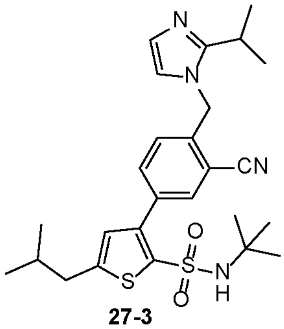

27-3

(2-(N-(tert-butyl)sulfamoyl)-5-isobutylthiophen-3-yl)boronic acid (150 mg, 470 μmol) was dissolved in tetrahydrofuran (10 mL) and water (2 mL). 5-bromo-2-((2-isopropyl-1H-imidazol-1-yl)methyl)benzonitrile (143 mg, 470 μmol) was added, and potassium phosphate (299 mg, 1.41 mmol) and XPhos Pd G4 (40.4 mg, 47.0 μmol) were added. Then, the mixture was stirred and reacted at 60°C under nitrogen protection for 2 hours. The reaction solution was concentrated to obtain a crude product, and the crude product was separated and purified by a silica gel column (petroleum ether: ethyl acetate (V/V)=(50:1) to (0:1)) to obtain N-(tert-butyl)-3-(3-cyano-4-((2-isopropyl-1H-imidazol-1-yl)methyl)phenyl)-5-isobutylthiophene-2-sulfonamide (200 mg, yield 85.4%).
LC-MS, M/Z (ESI): 499.2 $[M+H]^+$

Step 3: Synthesis of 3-(3-cyano-4-((2-isopropyl-1H-imidazol-1-yl)methyl)phenyl)-5-isobutylthiophene-2-sulfonamide

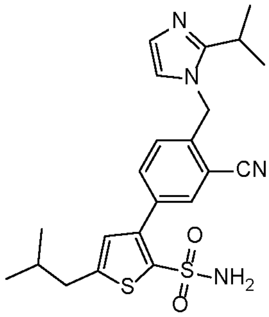

**27-4**

N-(tert-butyl)-3-(3-cyano-4-((2-isopropyl-1H-imidazol-1-yl)methyl)phenyl)-5-isobutylthiophene-2-sulfonamide (180 mg, 361 μmol) was dissolved in trifluoroacetic acid (5 mL) and dichloromethane (3 mL), and the mixture reacted at 30°C for 2 hours. The reaction solution was poured into water (10 mL), and then extracted with dichloromethane (50 mL). The organic phases were collected, dried over anhydrous sodium sulfate, filtered, and concentrated. The crude product was separated and purified by a silica gel column (petroleum ether: ethyl acetate (V/V)=(10:1) to (1:1)) to obtain 3-(3-cyano-4-((2-isopropyl-1H-imidazol-1-yl)methyl)phenyl)-5-isobutylthiophene-2-sulfonamide (150 mg, yield 93.9%).
LC-MS, M/Z (ESI): 443.2 $[M+H]^+$

Step 4: Synthesis of methyl ((3-(3-cyano-4-((2-isopropyl-1H-imidazol-1-yl)methyl)phenyl)-5-isobutylthiophen-2-yl)sulfonyl)carbamate

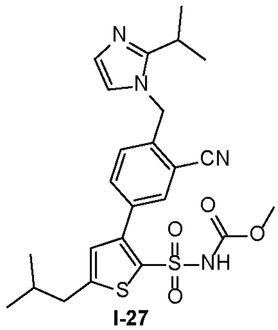

**I-27**

3-(3-cyano-4-((2-isopropyl-1H-imidazol-1-yl)methyl)phenyl)-5-isobutylthiophene-2-sulfonamide (5) (130 mg, 294 μmol) and N,N-diisopropylethylamine (114 mg, 881 μmol) were dissolved in dichloromethane (10.0 mL), methyl chloroformate (179 mg, 1.89 mmol) was added, and the mixture was stirred and reacted at 0°C for 1 hour. The reaction solution was concentrated, separated and purified by high performance liquid chromatography (column: Waters Xbridge 150*25mm*5um; solvent: A=water + 0.05 volume of aqueous ammonia (30%), B=acetonitrile; gradient: 8% to 38%, 9 minutes), and concentrated and dried to obtain methyl ((3-(3-cyano-4-((2-isopropyl-1H-imidazol-1-yl)methyl)phenyl)-5-isobutylthiophen-2-yl)sulfonyl)carbamate (120 mg, yield 81.6%).

$^1$H NMR (400 MHz, $CDCl_3$) $\delta$ 7.95 (s, 1H), 7.83 (d, 1H), 7.12 (s, 1H), 7.04 (d, 1H), 6.73 (s, 2H), 5.35 (s, 2H), 3.67 (s, 3H), 3.16-3.20 (m, 1H), 2.72 (d, 2H), 1.94-1.96 (m, 1H), 1.40 (d, 6H), 1.01 (d, 6H).
LC-MS, M/Z (ESI): 501.2 $[M+H]^+$

**Example 25:** Preparation of target compound **I-28**

Ethyl ((5-isobutyl-4-methyl-3-(4-((2-methyl-1H-imidazol-1-yl)methyl)phenyl)thiophen-2-yl)sulfonyl)carbamate

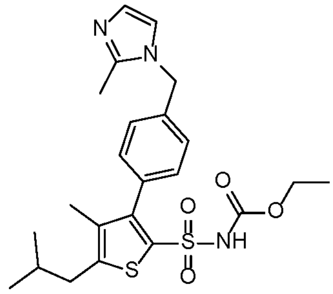

I-28

The synthetic scheme of the target compound 1-28 was as follows:

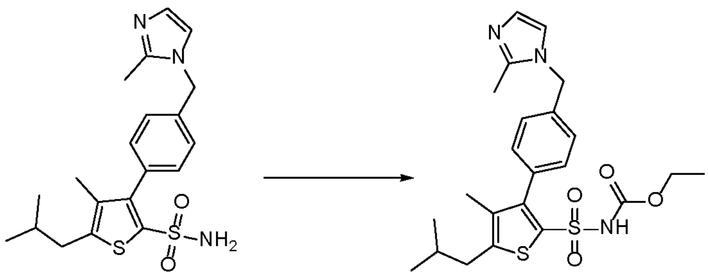

Step 1: Synthesis of ethyl ((5-isobutyl-4-methyl-3-(4-((2-methyl-1H-imidazol-1-yl)methyl)phenyl)thiophen-2-yl)sulfonyl) carbamate

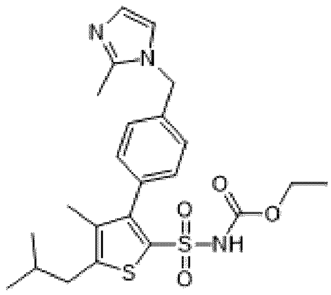

I-28

5-isobutyl-4-methyl-3-(4-((2-methyl-1H-imidazol-1-yl)methyl)phenyl)thiophene-2-sulfonamide (300 mg, 743 μmol) and N,N-diisopropylethylamine (288 mg, 2.23 mmol) were dissolved in dichloromethane (5 mL), ethyl chloroformate (242 mg, 2.23 mmol) was added, and the mixture was stirred and reacted at 0°C for 1 hour. The reaction solution was concentrated, separated and purified by high performance liquid chromatography (column: Waters Xbridge 150*25mm*5um; solvent: A=water + 0.05 volume formic acid (99%), B=acetonitrile; gradient: 15% to 45%, 15 minutes), and concentrated and dried to obtain ethyl ((5-isobutyl-4-methyl-3-(4-((2-methyl-1H-imidazol-1-yl)methyl)phenyl)thiophen-2-yl)sulfonyl)carbamate (300 mg, yield 84.9%).

$^1$H NMR (400 MHz, DMSO_d$_6$) $\delta$ 7.44 (s, 1H), 7.20-7.26 (m, 5H), 5.30 (s, 2H), 3.81 (q, 2H), 2.63 (d, 2H), 1.82-1.85 (m, 1H), 1.74 (s, 3H), 1.03 (t, 6H), 0.95 (d, 6H).
LC-MS, M/Z (ESI): 476.2 [M+H]$^+$

**Example 26:** Preparation of target compound **I-29**

Propyl ((5-isobutyl-4-methyl-3-(4-((2-methyl-1H-imidazol-1-yl)methyl)phenyl)thiophen-2-yl)sulfonyl)carbamate

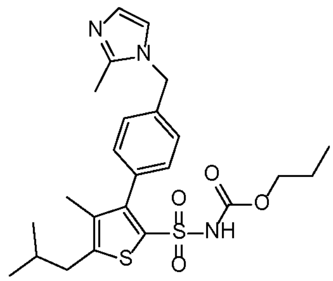

I-29

The synthetic scheme of the target compound 1-29 was as follows:

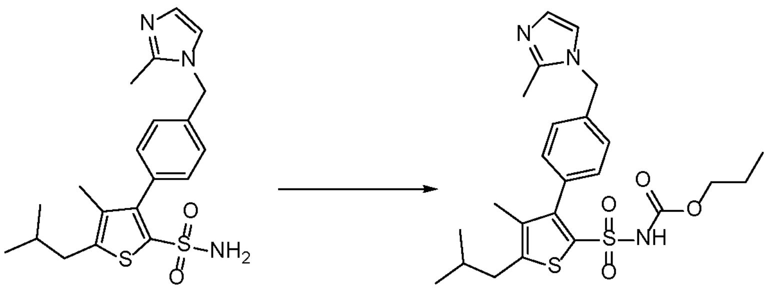

16-4 I-29

Step 1: Synthesis of propyl ((5-isobutyl-4-methyl-3-(4-((2-methyl-1H-imidazol-1-yl)methyl)phenyl)thiophen-2-yl)sulfonyl)carbamate

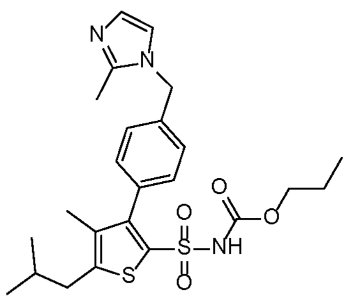

I-29

5-isobutyl-4-methyl-3-(4-((2-methyl-1H-imidazol-1-yl)methyl)phenyl)thiophene-2-sulfonamide (90.0 mg, 223 μmol) and N,N-diisopropylethylamine (86.5 mg, 669 μmol) were dissolved in dichloromethane (5.00 mL), propyl chloroformate (27.3 mg, 223 μmol) was added, and the mixture was stirred and reacted at 0°C for 1 hour. The reaction solution was concentrated, separated and purified by high performance liquid chromatography (column: Waters Xbridge 150*25mm*5um; solvent: A=water + 0.05 volume of aqueous ammonia (30%), B=acetonitrile; gradient: 13% to 43%, 35 minutes), and concentrated and dried to obtain propyl ((5-isobutyl-4-methyl-3-(4-((2-methyl-1H-imidazol-1-yl)methyl) phenyl)thiophen-2-yl)sulfonyl)carbamate (12.6 mg, yield 13.9%).

$^1$H NMR (400 MHz, DMSO $d_6$) $\delta$ 7.40 (s, 1H), 7.19-7.25 (m, 4H), 7.13 (s, 1H), 5.28 (s, 2H), 3.70 (t, 2H), 2.62 (d, 2H), 2.41 (s, 3H), 1.84-1.87 (m, 1H), 1.77 (s, 3H), 1.41-1.43 (m, 2H), 0.95 (d, 6H), 0.79 (t, 3H).
LC-MS, M/Z (ESI): 490.2 $[M+H]^+$

**Example** 27: Preparation of target compound **I-31**

Butyl ((5-isobutyl-4-methyl-3-(4-((2-methyl-1H-imidazol-1-yl)methyl)phenyl)thiophen-2-yl)sulfonyl)carbamate

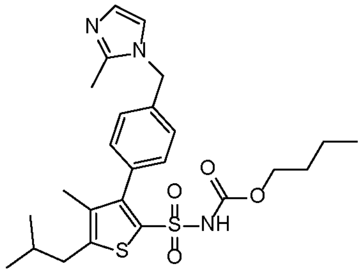

I-31

The synthetic scheme of the target compound **I-31** was as follows:

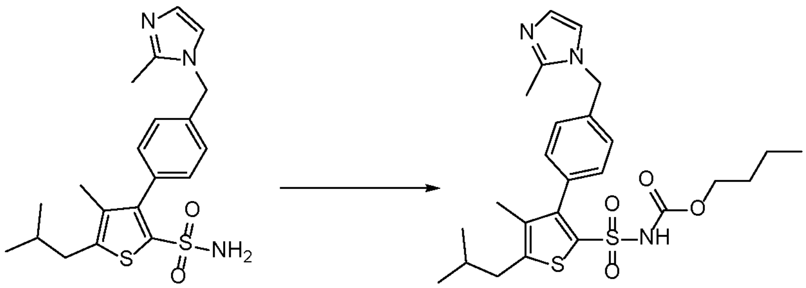

16-4 I-31

Step 1: Synthesis of butyl ((5-isobutyl-4-methyl-3-(4-((2-methyl-1H-imidazol-1-yl)methyl)phenyl)thiophen-2-yl)sulfonyl) carbamate

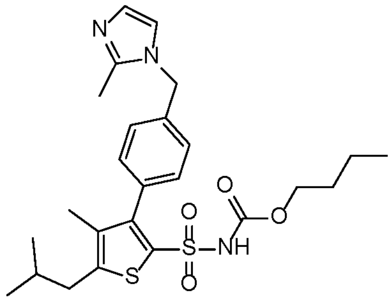

I-31

5-isobutyl-4-methyl-3-(4-((2-methyl-1H-imidazol-1-yl)methyl)phenyl)thiophene-2-sulfonamide (100 mg, 248 μmol) and N,N-diisopropylethylamine (64.1 mg, 496 μmol) were dissolved in dichloromethane (5 mL), butyl chloroformate (67.7 mg, 496 μmol) was added, and the mixture was stirred and reacted at 0°C for 0.5 hour. The reaction solution was concentrated, separated and purified by high performance liquid chromatography (column: Phenomenex Luna C18 150*25mm*10um; solvent: A=water + 0.05 volume of trifluoroacetic acid (99%), B=acetonitrile; gradient: 33% to 63%, 9 minutes), and concentrated and dried to obtain butyl ((5-isobutyl-4-methyl-3-(4-((2-methyl-1H-imidazol-1-yl)methyl) phenyl)thiophen-2-yl)sulfonyl)carbamate (42.4 mg, yield 34.0%).

$^1$H NMR (400 MHz, DMSO $d_6$): $\delta$ 11.65 (brs, 1H), 7.71 (d, 1H), 7.64 (d, 1H), 7.38 (d, 2H), 7.25 (d, 2H), 5.44 (s, 2H), 3.96 (t, 2H), 2.70 (d, 2H), 2.61 (s, 3H), 1.84-1.93 (m, 1H), 1.80 (s, 3H), 1.38-1.47 (m, 2H), 1.16-1.26 (m, 2H), 0.96 (d, 6H), 0.84 (t, 3H).
LC-MS, M/Z (ESI): 504.2 [M+H]$^+$

**Example 28:** Preparation of target compound **I-32**

Methyl ((3-(4-((2-ethyl-1H-imidazol-1-yl)methyl)phenyl)-5-isobutyl-4-methylthiophen-2-yl)sulfonyl)carbamate

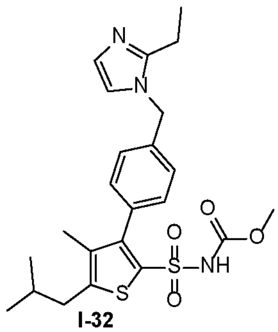

**I-32**

The synthetic scheme of the target compound **1-32** was as follows:

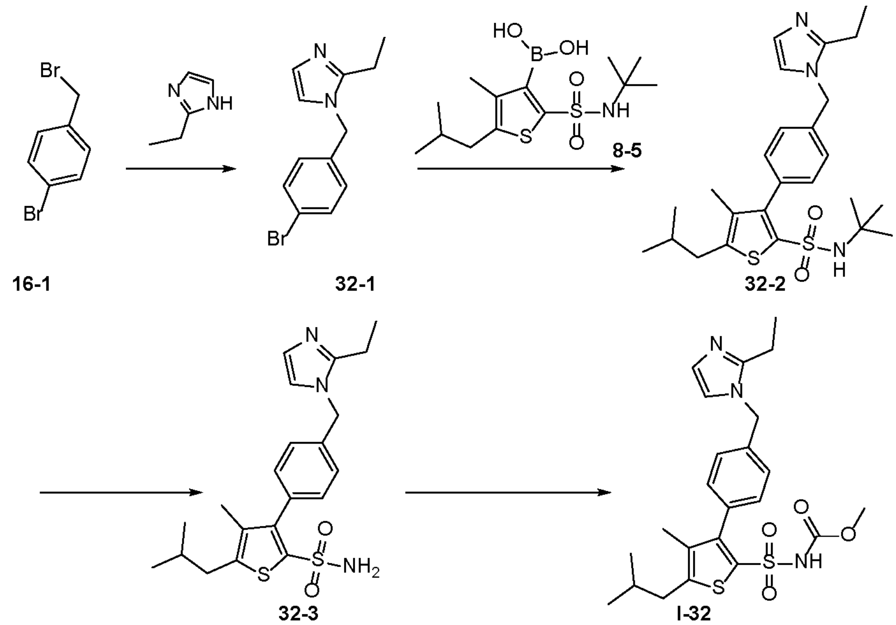

**16-1** **32-1** **8-5** **32-2**

**32-3** **I-32**

Step 1: Synthesis of 1-(4-bromobenzyl)-2-ethyl-1H-imidazole

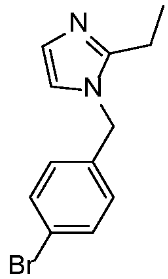

**32-1**

1-bromo-4-(bromomethyl)benzene (1.00 g, 4.00 mmol) and 2-ethyl-1H-imidazole (384 mg, 4.00 mmol) were

dissolved in N,N-dimethylformamide (30 mL), potassium carbonate (1.66 g, 12.0 mmol) was added, and the mixture reacted at 50°C for 2 hours. The reaction solution was poured into water (25 mL), and extracted with ethyl acetate (100 mL) for three times. The organic phases were collected, dried over anhydrous sodium sulfate, filtered, and concentrated. The crude product was separated and purified by a silica gel column (petroleum ether: ethyl acetate (V/V)=(50:1) to (1:1)) to obtain 1-(4-bromobenzyl)-2-ethyl-1H-imidazole (900 mg, yield 84.8%).
LC-MS, M/Z (ESI): 267.2 $[M+H]^+$

Step 2: Synthesis of N-(tert-butyl)-3-(4-((2-ethyl-1H-imidazol-1-yl)methyl)phenyl)-5-isobutyl-4-methylthiophene-2-sulfonamide

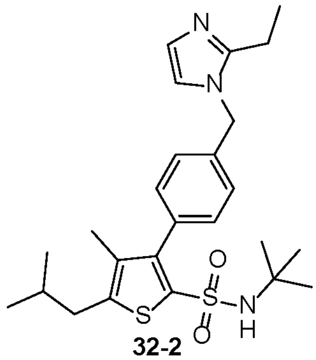

32-2

(2-(N-(tert-butyl)sulfamoyl)-5-isobutyl-4-methylthiophen-3-yl)boronic acid (400 mg, 1.20 mmol) was dissolved in tetrahydrofuran (5 mL) and water (5 mL). 1-(4-bromobenzyl)-2-ethyl-1H-imidazole (318 mg, 1.20 mmol) was added, and potassium phosphate (1.27 g, 6.00 mmol) and XPhos Pd G4 (103 mg, 120 μmol) were added. The mixture was stirred and reacted at 60°C under nitrogen protection for 4 hours. The reaction solution was concentrated to obtain a crude product, and the crude product was separated and purified by a silica gel column (petroleum ether: ethyl acetate (V/V)=(50:1) to (0:1)) to obtain N-(tert-butyl)-3-(4-((2-ethyl-1H-imidazol-1-yl)methyl)phenyl)-5-isobutyl-4-methylthiophene-2-sulfonamide (450 mg, yield 79.1%).
LC-MS, M/Z (ESI): 474.3 $[M+H]^+$

Step 3: Synthesis of 3-(4-((2-ethyl-1H-imidazol-1-yl)methyl)phenyl)-5-isobutyl-4-methylthiophene-2-sulfonamide

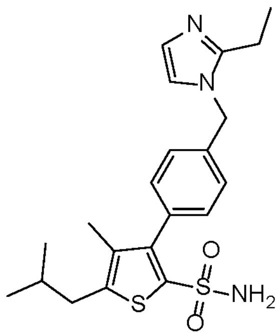

32-3

N-(tert-butyl)-3-(4-((2-ethyl-1H-imidazol-1-yl)methyl)phenyl)-5-isobutyl-4-methylthiophene-2-sulfonamide (400 mg, 844 μmol) was dissolved in trifluoroacetic acid (5 mL) and dichloromethane (5 mL), and the mixture reacted at 40°C for 8 hours. The reaction solution was poured into water (10 mL), and then extracted with dichloromethane (50 mL) for three times. The organic phases were collected, dried over anhydrous sodium sulfate, filtered, and concentrated. The crude product was separated and purified by a silica gel column (petroleum ether: ethyl acetate (V/V)=(10:1) to (1:1)) to obtain 3-(4-((2-ethyl-1H-imidazol-1-yl)methyl)phenyl)-5-isobutyl-4-methylthiophene-2-sulfonamide (320 mg, yield 90.6%).
LC-MS, M/Z (ESI): 418.4 $[M+H]^+$

Step 4: Synthesis of methyl ((3-(4-((2-ethyl-1H-imidazol-1-yl)methyl)phenyl)-5-isobutyl-4-methylthiophen-2-yl)sulfonyl) carbamate

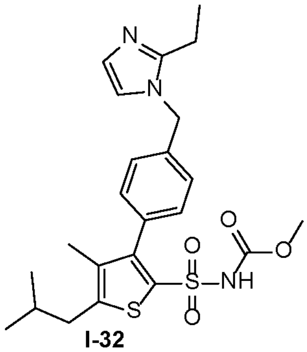

I-32

3-(4-((2-ethyl-1H-imidazol-1-yl)methyl)phenyl)-5-isobutyl-4-methylthiophene-2-sulfonamide (316 mg, 759 μmol) and N,N-diisopropylethylamine (294 mg, 2.28 mmol) were dissolved in dichloromethane (5 mL), methyl chloroformate (430 mg, 4.55 mmol) was added, and the mixture was stirred and reacted at 0°C for 1 hour. The reaction solution was concentrated, separated and purified by high performance liquid chromatography (column: Phenomenex Luna C18 150*25mm*10um; solvent: A=water + 0.05 volume of formic acid (99%), B=acetonitrile; gradient: 25% to 55%, 9 minutes), and concentrated and dried to obtain methyl ((3-(4-((2-ethyl-1H-imidazol-1-yl)methyl)phenyl)-5-isobutyl-4-methylthiophen-2-yl)sulfonyl)carbamate (241 mg, yield 80.3%).

$^1$H NMR (400 MHz, $CDCl_3$) δ: 7.33 (d, 2H), 7.17 (d, 2H), 6.70 (s, 2H), 5.08 (s, 2H), 3.65 (s, 3H), 2.80-2.85 (m, 2H), 2.67 (d, 2H), 1.92-2.01 (m, 1H), 1.84 (s, 3H), 1.28 (t, 3H), 1.01 (d, 6H).
LC-MS, M/Z (ESI): 476.4 $[M+H]^+$

Example 29: Preparation of target compound 1-33

Methyl ((5-isobutyl-3-(4-((2-isopropyl-1H-imidazol-1-yl)methyl)phenyl)-4-methylthiophen-2-yl)sulfonyl)carbamate

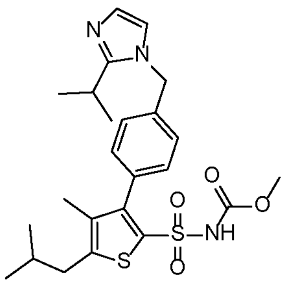

I-33

The synthetic scheme of the target compound 1-33 was as follows:

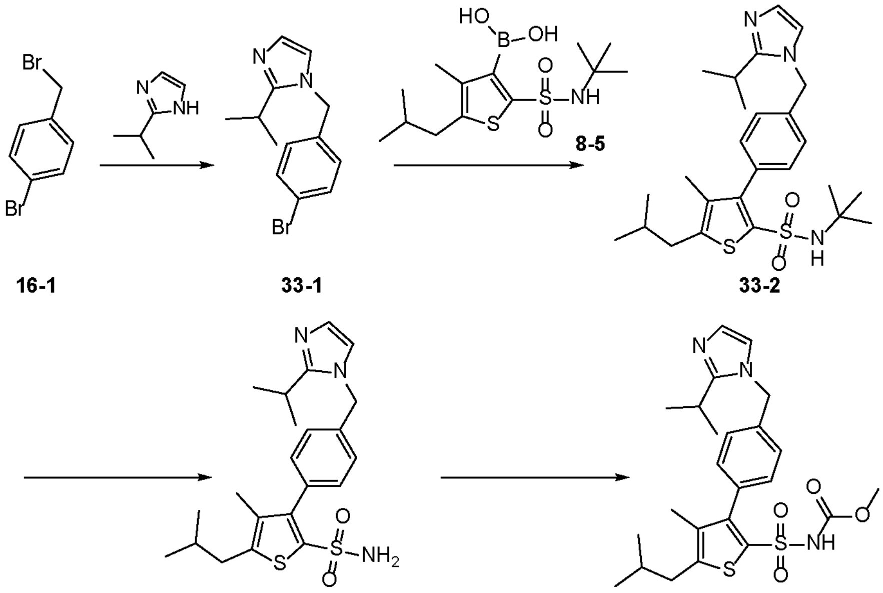

8-5

16-1 33-1 33-2

33-3 I-33

Step 1: Synthesis of 1-(4-bromobenzyl)-2-isopropyl-1H-imidazole

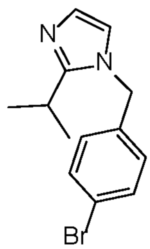

33-1

1-bromo-4-(bromomethyl)benzene (1.00 g, 4.00 mmol) and 2-isopropyl-1H-imidazole (441 mg, 4.00 mmol) were dissolved in N,N-dimethylformamide (15 mL), potassium carbonate (1.66 g, 12.0 mmol) was added, and the mixture reacted at 55°C for 1 hour. The reaction solution was poured into water (50 mL), and extracted with ethyl acetate (50 mL) for three times. The organic phases were collected, dried over anhydrous sodium sulfate, filtered, and concentrated. The crude product was separated and purified by a silica gel column (petroleum ether: ethyl acetate (V/V)=(50:1) to (1:1)) to obtain 1-(4-bromobenzyl)-2-isopropyl-1H-imidazole (900 mg, yield 80.6%).
LC-MS, M/Z (ESI): 279.0 $[M+H]^+$

Step 2: Synthesis of N-(tert-butyl)-5-isobutyl-3-(4-((2-isopropyl-1H-imidazol-1-yl)methyl)phenyl)-4-methylthiophene-2-sulfonamide

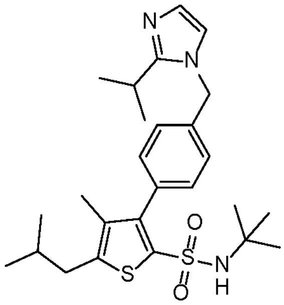

**33-2**

(2-(N-(tert-butyl)sulfamoyl)-5-isobutyl-4-methylthiophen-3-yl)boronic acid (400 mg, 1.20 mmol) was dissolved in tetrahydrofuran (6 mL) and water (2 mL). 1-(4-bromobenzyl)-2-isopropyl-1H-imidazole (302 mg, 1.08 mmol) was added, and potassium phosphate (1.27 g, 6.00 mmol) and XPhos Pd G4 (103 mg, 120 μmol) were added. Then, the mixture was stirred and reacted at 60°C under nitrogen protection for 2 hours. The reaction solution was concentrated to obtain a crude product, and the crude product was separated and purified by a silica gel column (petroleum ether: ethyl acetate (V/V)=(50:1) to (0:1)) to obtain N-(tert-butyl)-5-isobutyl-3-(4-((2-isopropyl-1H-imidazol-1-yl)methyl)phenyl)-4-methylthiophene-2-sulfonamide (430 mg, yield 73.5%).
LC-MS, M/Z (ESI): 488.2 $[M+H]^+$

Step 3: Synthesis of 5-isobutyl-3-(4-((2-isopropyl-1H-imidazol-1-yl)methyl)phenyl)-4-methylthiophene-2-sulfonamide

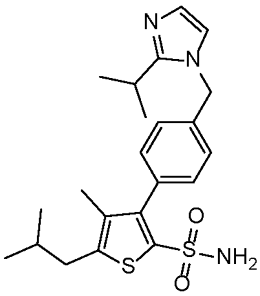

**33-3**

N-(tert-butyl)-5-isobutyl-3-(4-((2-isopropyl-1H-imidazol-1-yl)methyl)phenyl)-4-methylthiophene-2-sulfonamide (430 mg, 882 μmol) was dissolved in trifluoroacetic acid (15 mL) and dichloromethane (5 mL), and the mixture reacted at 40°C for 1 hour. The reaction solution was poured into water (10 mL), and then extracted with dichloromethane (20 mL) for three times. The organic phases were collected, dried over anhydrous sodium sulfate, filtered, and concentrated. The crude product was separated and purified by a silica gel column (petroleum ether: ethyl acetate (V/V)=(10:1) to (1:1)) to obtain 5-isobutyl-3-(4-((2-isopropyl-1H-imidazol-1-yl)methyl)phenyl)-4-methylthiophene-2-sulfonamide (350 mg, yield 92.0%).
LC-MS, M/Z (ESI): 432.2 $[M+H]^+$

Step 4: Synthesis of methyl ((5-isobutyl-3-(4-((2-isopropyl-1H-imidazol-1-yl)methyl)phenyl)-4-methylthiophen-2-yl)sulfonyl)carbamate

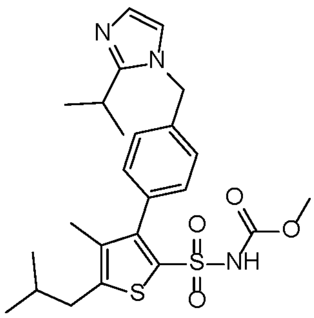

I-33

5-isobutyl-3-(4-((2-isopropyl-1H-imidazol-1-yl)methyl)phenyl)-4-methylthiophene-2-sulfonamide (300 mg, 695 μmol) and N,N-diisopropylethylamine (270 mg, 2.09 mmol) were dissolved in dichloromethane (5 mL), methyl chloroformate (394 mg, 4.17 mmol) was added, and the mixture was stirred and reacted at 0°C for 1 hour. The reaction solution was concentrated, separated and purified by high performance liquid chromatography (column: Phenomenex luna C18 150*25mm*10um; solvent: A=water + 0.05 volume of formic acid (99%), B=acetonitrile; gradient: 17% to 47%, 10 minutes), and concentrated and dried to obtain methyl ((5-isobutyl-3-(4-((2-isopropyl-1H-imidazol-1-yl)methyl)phenyl)-4-methylthiophen-2-yl)sulfonyl)carbamate (182 mg, yield 53.4%).

$^1$H NMR (400 MHz, $CDCl_3$): $\delta$ 7.26 (d, 2H), 7.15 (d, 2H), 6.90 (d, 1H), 6.78 (d, 1H), 5.15 (s, 2H), 3.67 (s, 3H), 3.02-3.09 (m, 1H), 2.68 (d, 2H), 1.93-1.97 (m, 1H), 1.83 (s, 3H), 1.31 (d, 6H), 1.01 (d, 6H).
LC-MS, M/Z (ESI): 490.2 $[M+H]^+$

**Example 30:** Preparation of target compound **I-34**

Methyl ((3-(4-((2-chloro-1H-imidazol-1-yl)methyl)phenyl)-5-isobutyl-4-methylthiophen-2-yl)sulfonyl)carbamate

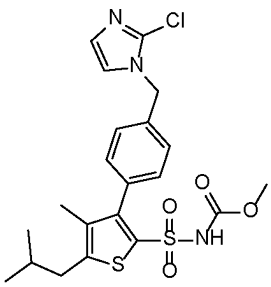

I-34

The synthetic scheme of the target compound **I-34** was as follows:

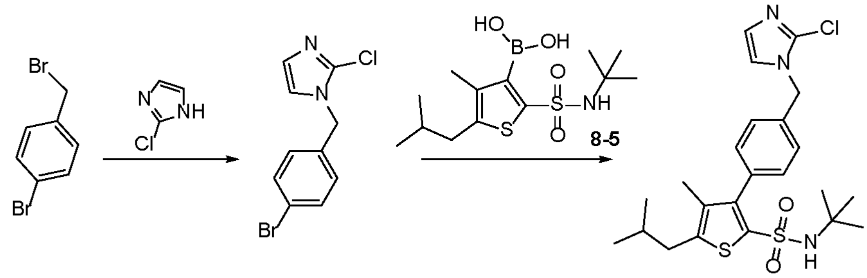


16-1 34-1 34-2

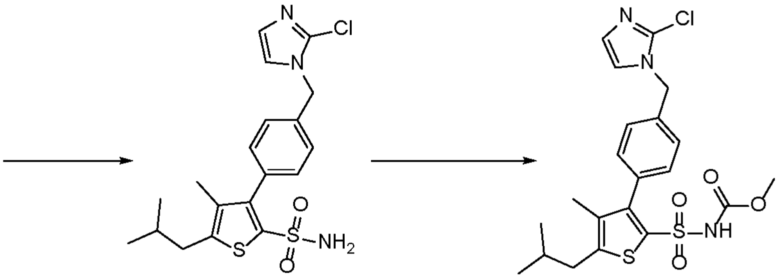

34-3 I-34

Step 1: Synthesis of 1-(4-bromobenzyl)-2-chloro-1H-imidazole

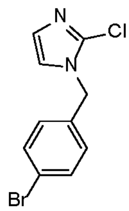

34-1

1-bromo-4-(bromomethyl)benzene (1.00 g, 4.00 mmol) and 2-chloro-1H-imidazole (410 mg, 4.00 mmol) were dissolved in N,N-dimethylformamide (20.0 mL), potassium carbonate (1.66 g, 12.0 mmol) was added, and the mixture was reacted at 55°C for 1 hour. The reaction solution was poured into water (50 mL), and extracted with ethyl acetate (50 mL) for three times. The organic phases were collected, dried over anhydrous sodium sulfate, filtered, and concentrated. The crude product was separated and purified by a silica gel column (petroleum ether: ethyl acetate (V/V)=(50:1) to (1:1)) to obtain 1-(4-bromobenzyl)-2-chloro-1H-imidazole (900 mg, yield 82.8%).
LC-MS, M/Z (ESI): 271.0 $[M+H]^+$

Step 2: Synthesis of N-(tert-butyl)-3-(4-((2-chloro-1H-imidazol-1-yl)methyl)phenyl)-5-isobutyl-4-methylthiophene-2-sulfonamide

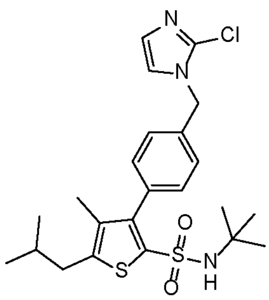

34-2

(2-(N-(tert-butyl)sulfamoyl)-5-isobutyl-4-methylthiophen-3-yl)boronic acid (400 mg, 1.20 mmol) was dissolved in tetrahydrofuran (6 mL) and water (2 mL). 1-(4-bromobenzyl)-2-chloro-1H-imidazole (293 mg, 1.08 mmol) was added, and potassium phosphate (1.27 g, 6.00 mmol) and XPhos Pd G4 (103 mg, 120 μmol) were added. Then, the mixture was stirred and reacted at 60°C under nitrogen protection for 2 hours. The reaction solution was concentrated to obtain a crude product, and the crude product was separated and purified by a silica gel column (petroleum ether: ethyl acetate (V/V)=(50:1) to (0:1)) to obtain N-(tert-butyl)-3-(4-((2-chloro-1H-imidazol-1-yl)methyl)phenyl)-5-isobutyl-4-methylthiophene-2-sulfonamide (510 mg, yield 88.5%).
LC-MS, M/Z (ESI): 480.2 $[M+H]^+$

Step 3: Synthesis of 3-(4-((2-chloro-1H-imidazol-1-yl)methyl)phenyl)-5-isobutyl-4-methylthiophene-2-sulfonamide

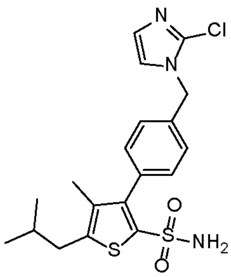

34-3

N-(tert-butyl)-3-(4-((2-chloro-1H-imidazol-1-yl)methyl)phenyl)-5-isobutyl-4-methylthiophene-2-sulfonamide (500 mg, 1.04 mmol) was dissolved in trifluoroacetic acid (15 mL) and dichloromethane (5 mL), and the mixture reacted at 40°C for 1 hour. The reaction solution was poured into water (10 mL), and then extracted with dichloromethane (20 mL) for three times. The organic phases were collected, dried over anhydrous sodium sulfate, filtered, and concentrated. The crude product was separated and purified by a silica gel column (petroleum ether: ethyl acetate (V/V)=(10:1) to (1:1)) to obtain a brown oily compound 3-(4-((2-chloro-1H-imidazol-1-yl)methyl)phenyl)-5-isobutyl-4-methylthiophene-2-sulfonamide (400 mg, yield 90.6%).
LC-MS, M/Z (ESI): 424.1 $[M+H]^+$

Step 4: Synthesis of methyl ((3-(4-((2-chloro-1H-imidazol-1-yl)methyl)phenyl)-5-isobutyl-4-methylthiophen-2-yl)sulfonyl)carbamate

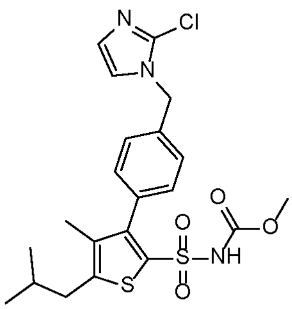

I-34

3-(4-((2-chloro-1H-imidazol-1-yl)methyl)phenyl)-5-isobutyl-4-methylthiophene-2-sulfonamide (350 mg, 826 μmol) and N,N-diisopropylethylamine (320 mg, 2.48 mmol) were dissolved in dichloromethane (5 mL), methyl chloroformate (468 mg, 4.95 mmol) was added, and the mixture was stirred and reacted at 0°C for 1 hour. The reaction solution was concentrated, separated and purified by high performance liquid chromatography (column: Phenomenex luna C18 150*25mm*10um; solvent: A=water + 0.05 volume of formic acid (99%), B=acetonitrile; gradient: 48% to 78%, 10 minutes), and concentrated and dried to obtain methyl ((3-(4-((2-chloro-1H-imidazol-1-yl)methyl)phenyl)-5-isobutyl-4-methylthiophen-2-yl)sulfonyl)carbamate (155 mg, yield 38.9%).

$^1H$ NMR (400 MHz, $CDCl_3$): $\delta$ 7.23-7.26 (m, 4H), 6.99-7.01 (d, 1H), 6.97 (d, 1H), 5.17 (s, 2H), 3.70 (s, 3H), 2.69 (d, 2H),

1.95-2.01 (m, 1H), 1.86 (s, 3H), 1.02 (d, 6H).
LC-MS, M/Z (ESI): 482.1 $[M+H]^+$

**Example 31:** Preparation of target compound **I-15**

Methyl ((5-isobutyl-3-(4-(1-(2-methyl-1H-imidazol-1-yl)ethyl)phenyl)thiophen-2-yl)sulfonyl)carbamate

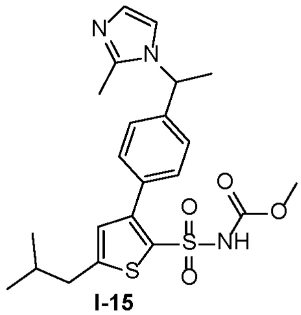

**I-15**

The synthetic scheme of the target compound **I-15** was as follows:

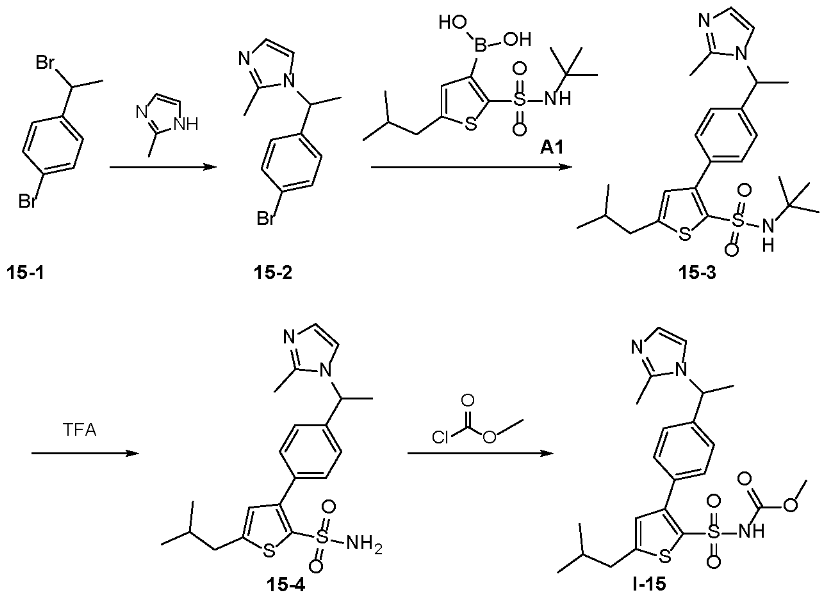


15-1 15-2 15-3

15-4 I-15

Step 1: Synthesis of 1-(1-(4-bromophenyl)ethyl)-2-methyl-1H-imidazole

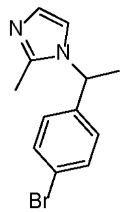

**15-2**

1-bromo-4-(1-bromoethyl)benzene (1.00 g, 3.79 mmol) and 2-methyl-1H-imidazole (933 mg, 11.3 mmol) were dissolved in N,N-dimethylformamide (20 mL), potassium carbonate (1.57 g, 11.3 mmol) was added, and the mixture

reacted at 50°C for 2 hours. The reaction solution was poured into water (50 mL), and extracted with ethyl acetate (100 mL) for three times. The organic phases were collected, dried over anhydrous sodium sulfate, filtered, and concentrated. The crude product was separated and purified by a silica gel column (petroleum ether: ethyl acetate (V/V)=(50:1) to (1:1)) to obtain 1-(1-(4-bromophenyl)ethyl)-2-methyl-1H-imidazole (900 mg, yield 89.6%).
LC-MS, M/Z (ESI): 267.0 $[M+H]^+$

Step 2: Synthesis of N-(tert-butyl)-5-isobutyl-3-(4-(1-(2-methyl-1H-imidazol-1-yl)ethyl)phenyl)thiophene-2-sulfonamide

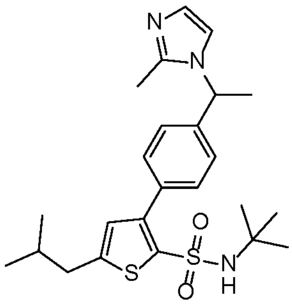

**15-3**

(2-(N-(tert-butyl)sulfamoyl)-5-isobutylthiophen-3-yl)boronic acid (500 mg, 1.57 mmol) was dissolved in tetrahydrofuran (10 mL) and water (5 mL). 1-(1-(4-bromophenyl)ethyl)-2-methyl-1H-imidazole (415 mg, 1.57 mmol) was added, and potassium phosphate (1.66 g, 7.83 mmol) and XPhos Pd G4 (134 mg, 156 μmol) were added. Then, the mixture was stirred and reacted at 60°C under nitrogen protection for 4 hours. The reaction solution was concentrated to obtain a crude product, and the crude product was separated and purified by a silica gel column (petroleum ether: ethyl acetate (V/V)=(50:1) to (0:1)) to obtain N-(tert-butyl)-5-isobutyl-3-(4-(1-(2-methyl-1H-imidazol-1-yl)ethyl)phenyl)thiophene-2-sulfonamide (610 mg, yield 84.7%).
LC-MS, M/Z (ESI): 460.1 $[M+H]^+$

Step 3: Synthesis of 5-isobutyl-3-(4-(1-(2-methyl-1H-imidazol-1-yl)ethyl)phenyl)thiophene-2-sulfonamide

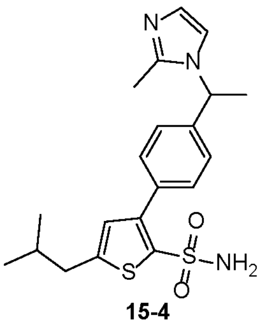

**15-4**

N-(tert-butyl)-5-isobutyl-3-(4-(1-(2-methyl-1H-imidazol-1-yl)ethyl)phenyl)thiophene-2-sulfonamide (500 mg, 1.09 mol) was dissolved in trifluoroacetic acid (5 mL) and dichloromethane (10 mL), and the mixture reacted at 20°C for 8 hours. The reaction solution was poured into water (10 mL), and then extracted with dichloromethane (50 mL) for three times. The organic phases were collected, dried over anhydrous sodium sulfate, filtered, and concentrated. The crude product was separated and purified by a silica gel column (petroleum ether: ethyl acetate (V/V)=(10:1) to (0:1)) to obtain 5-isobutyl-3-(4-(1-(2-methyl-1H-imidazol-1-yl)ethyl)phenyl)thiophene-2-sulfonamide (410 mg, yield 93.4%).
LC-MS, M/Z (ESI): 404.1 $[M+H]^+$

Step 4: Synthesis of methyl ((5-isobutyl-3-(4-(1-(2-methyl-1H-imidazol-1-yl)ethyl)phenyl)thiophen-2-yl)sulfonyl)carbamate

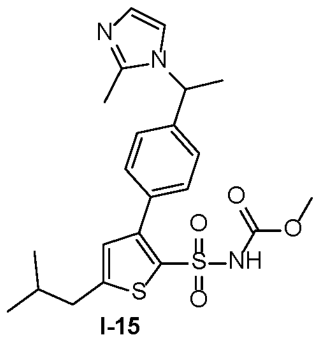

I-15

5-isobutyl-3-(4-(1-(2-methyl-1H-imidazol-1-yl)ethyl)phenyl)thiophene-2-sulfonamide (400 mg, 991 μmol) and N,N-diisopropylethylamine (384 mg, 2.91 mmol) were dissolved in dichloromethane (10 mL), methyl chloroformate (93.5 mg, 991 μmol) was added, and the mixture was stirred and reacted at 0°C for 0.5 hour. The reaction solution was concentrated, separated and purified by high performance liquid chromatography (column: Waters Xbridge Prep OBDC18 150*40mm*10um; solvent: A=water + 0.05 volume of ammonia water (30%), B=acetonitrile; gradient: 0% to 20%, 10 minutes), and concentrated and dried to obtain methyl ((5-isobutyl-3-(4-(1-(2-methyl-1H-imidazol-1-yl)ethyl)phenyl) thiophen-2-yl)sulfonyl)carbamate (220 mg, yield 52.2%).

$^1$H NMR (400 MHz, DMSO_d$_6$) $\delta$ 7.69-7.81 (m, 3H), 7.36 (s, 1H), 7.28 (d, 2H), 6.80 (s, 1H), 5.66-5.69 (m, 1H), 3.27 (s, 3H), 2.62 (d, 2H), 2.48 (s, 3H), 1.80-1.88 (m, 4H), 0.93 (d, 6H).
LC-MS, M/Z (ESI): 462.2 [M+H]$^+$

**Example 32:** Preparation of target compound **I-30**

Methyl ((5-isobutyl-4-methyl-3-(2-methyl-4-((2-methyl-1H-imidazol-1-yl)methyl)phenyl)thiophen-2-yl)sulfonyl)carbamate

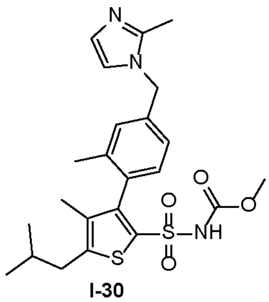

I-30

The synthetic scheme of the target compound **I-30** was as follows:

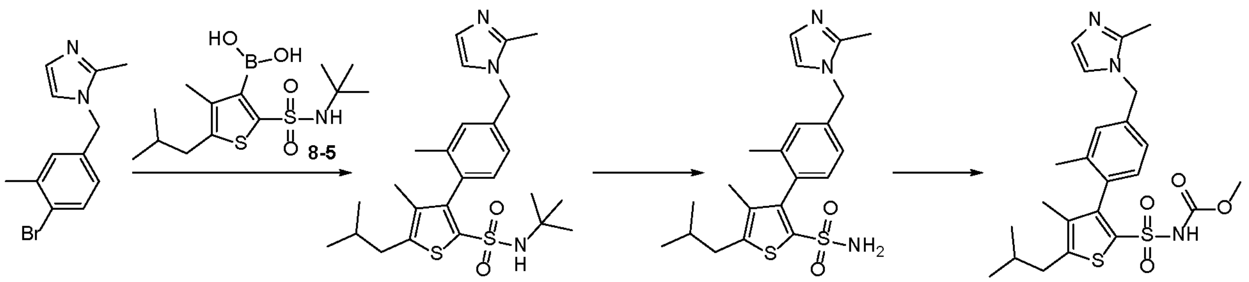


11-2 30-1 30-2 I-30

Step 1: Synthesis of N-(tert-butyl)-5-isobutyl-4-methyl-3-(2-methyl-4-((2-methyl-1H-imidazol-1-yl)methyl)phenyl)thiophene-2-sulfonamide

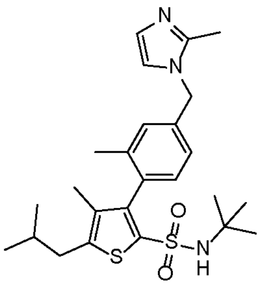

30-1

(2-(N-(tert-butyl)sulfamoyl)-5-isobutyl-4-methylthiophen-3-yl)boronic acid (400 mg, 1.20 mmol) was dissolved in tetrahydrofuran (10 mL) and water (5 mL). 1-(4-bromo-3-methylbenzyl)-2-methyl-1H-imidazole (286 mg, 1.08 mmol) was added, and potassium phosphate (1.27 g, 6.00 mmol) and XPhos Pd G4 (102 mg, 120 μmol) were added. Then, the mixture was stirred and reacted at 60°C under nitrogen protection for 2 hours. The reaction solution was concentrated to obtain a crude product, and the crude product was separated and purified by a silica gel column (petroleum ether: ethyl acetate (V/V)=(50:1) to (0:1)) to obtain N-(tert-butyl)-5-isobutyl-4-methyl-3-(2-methyl-4-((2-methyl-1H-imidazol-1-yl) methyl)phenyl)thiophene-2-sulfonamide (300 mg, crude product).
LC-MS, M/Z (ESI): 474.2 $[M+H]^+$

Step 3: Synthesis of 5-isobutyl-4-methyl-3-(2-methyl-4-((2-methyl-1H-imidazol-1-yl)methyl)phenyl)thiophene-2-sulfonamide

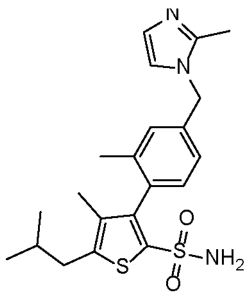

30-2

N-(tert-butyl)-5-isobutyl-4-methyl-3-(2-methyl-4-((2-methyl-1H-imidazol-1-yl)methyl)phenyl)thiophene-2-sulfonamide (300 mg, 633 μmol) was dissolved in trifluoroacetic acid (2 mL) and dichloromethane (2 mL), and the mixture reacted at 40°C for 2 hours. The reaction solution was poured into water (10 mL), and then extracted with dichloromethane (50 mL) for three times. The organic phases were collected, dried over anhydrous sodium sulfate, filtered, and concentrated to obtain 5-isobutyl-4-methyl-3-(2-methyl-4-((2-methyl-1H-imidazol-1-yl)methyl)phenyl)thiophene-2-sulfonamide (200 mg, crude).
LC-MS, M/Z (ESI): 418.2 $[M+H]^+$

Step 4: Synthesis of methyl ((5-isobutyl-4-methyl-3-(2-methyl-4-((2-methyl-1H-imidazol-1-yl)methyl)phenyl)thiophen-2-yl)sulfonyl)carbamate

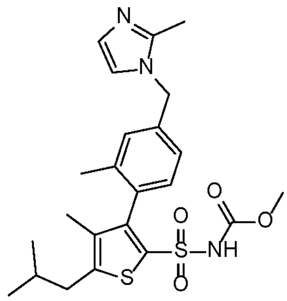

I-30

5-isobutyl-4-methyl-3-(2-methyl-4-((2-methyl-1H-imidazol-1-yl)methyl)phenyl)thiophene-2-sulfonamide (200 mg, 479 μmol) and methyl chloroformate (90.5 mg, 958 μmol) were dissolved in dichloromethane (5 mL), N,N-diisopropylethylamine (186 mg, 1.44 mmol) was added, and the mixture was stirred and reacted at 0°C for 1 hour. The reaction solution was concentrated, separated and purified by high performance liquid chromatography (column: Phenomenex luna C18 150*25mm*10um; solvent: A=water + 0.05 volume of formic acid (99%), B=acetonitrile; gradient: 25% to 55%, 7 minutes), and concentrated and dried to obtain methyl ((5-isobutyl-4-methyl-3-(2-methyl-4-((2-methyl-1H-imidazol-1-yl)methyl)phenyl)thiophen-2-yl)sulfonyl)carbamate (10.6 mg, yield 5.30%).

$^1$H NMR (400 MHz, $CDCl_3$) $\delta$ 7.18 (d, 1H), 7.06 (s, 1H), 6.99 (d, 1H), 6.66 (d, 1H), 6.49 (d, 1H), 4.97 (s, 2H), 3.68 (s, 3H), 2.68 (d, 2H), 2.41 (s, 3H), 2.05 (s, 3H), 1.94-2.01 (m, 1H), 1.72 (s, 3H), 1.00 (d, 6H).
LC-MS, M/Z (ESI): 476.1 $[M+H]^+$

Example 33: Preparation of target compound **I-35**

Methyl ((5-isobutyl-4-methyl-3-(3-methyl-4-((2-methyl-1H-imidazol-1-yl)methyl)phenyl)thiophen-2-yl)sulfonyl)carbamate

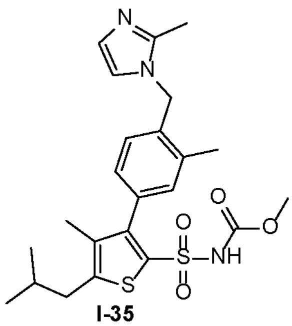

I-35

The synthetic scheme of the target compound **I-35** was as follows:

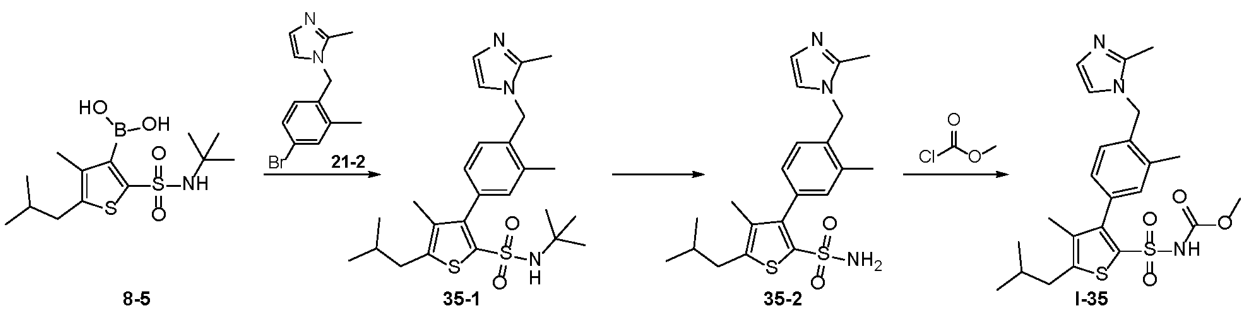


8-5 35-1 35-2 I-35

Step 1: Synthesis of N-(tert-butyl)-5-isobutyl-4-methyl-3-(3-methyl-4-((2-methyl-1H-imidazol-1-yl)methyl)phenyl)thiophene-2-sulfonamide

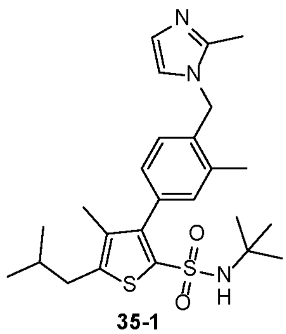

**35-1**

(2-(N-(tert-butyl)sulfamoyl)-5-isobutyl-4-methylthiophen-3-yl)boronic acid (400 mg, 1.20 mmol) was dissolved in tetrahydrofuran (5 mL) and water (5 mL). 1-(4-bromo-2-methylbenzyl)-2-methyl-1H-imidazole (254 mg, 960 μmol) was added, and potassium phosphate (1.27 g, 6.00 mmol) and XPhos Pd G4 (103 mg, 120 μmol) were added. Then, the mixture was stirred and reacted at 60°C under nitrogen protection for 4 hours. The reaction solution was concentrated to obtain a crude product, and the crude product was separated and purified by a silica gel column (petroleum ether: ethyl acetate (V/V)=(50:1) to (0:1)) to obtain N-(tert-butyl)-5-isobutyl-4-methyl-3-(3-methyl-4-((2-methyl-1H-imidazol-1-yl)methyl)phenyl)thiophene-2-sulfonamide (450 mg, yield 79.1%).
LC-MS, M/Z (ESI): 474.3 $[M+H]^+$

Step 2: Synthesis of 5-isobutyl-4-methyl-3-(3-methyl-4-((2-methyl-1H-imidazol-1-yl)methyl)phenyl)thiophene-2-sulfonamide

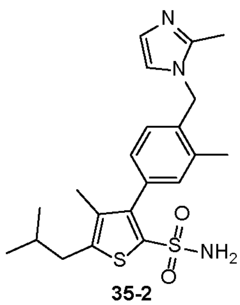

**35-2**

N-(tert-butyl)-5-isobutyl-4-methyl-3-(3-methyl-4-((2-methyl-1H-imidazol-1-yl)methyl)phenyl)thiophene-2-sulfonamide (400 mg, 844 μmol) was dissolved in trifluoroacetic acid (5 mL) and dichloromethane (5 mL), and the mixture reacted at 40°C for 8 hours. The reaction solution was poured into water (10 mL), and then extracted with dichloromethane (50 mL) for three times. The organic phases were collected, dried over anhydrous sodium sulfate, filtered, and concentrated. The crude product was separated and purified by a silica gel column (petroleum ether: ethyl acetate (V/V)=(10:1) to (1:1)) to obtain 5-isobutyl-4-methyl-3-(3-methyl-4-((2-methyl-1H-imidazol-1-yl)methyl)phenyl)thiophene-2-sulfonamide (320 mg, yield 90.7%).
LC-MS, M/Z (ESI): 418.4 $[M+H]^+$

Step 3: Synthesis of methyl ((5-isobutyl-4-methyl-3-(3-methyl-4-((2-methyl-1H-imidazol-1-yl)methyl)phenyl)thiophen-2-yl)sulfonyl)carbamate

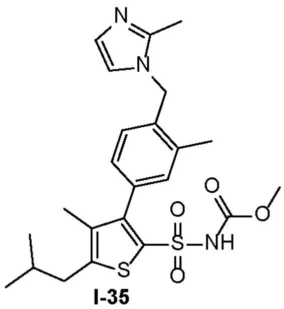

I-35

5-isobutyl-4-methyl-3-(3-methyl-4-((2-methyl-1H-imidazol-1-yl)methyl)phenyl)thiophene-2-sulfonamide (300 mg, 718 μmol) and N,N-diisopropylethylamine (278 mg, 2.16 mmol) were dissolved in dichloromethane (5 mL), methyl chloroformate (339 mg, 3.59 mmol) was added, and the mixture was stirred and reacted at 0°C for 1 hour. The reaction solution was concentrated, separated and purified by high performance liquid chromatography (column: Phenomenex luna C18 150*25mm*10um; solvent: A=water + 0.05 volume of formic acid (99%), B=acetonitrile; gradient: 22% to 52%, 9 minutes), and concentrated and dried to obtain methyl ((5-isobutyl-4-methyl-3-(3-methyl-4-((2-methyl-1H-imidazol-1-yl)methyl)phenyl)thiophen-2-yl)sulfonyl)carbamate (153 mg, yield 51.0%).

$^1$H NMR (400 MHz, $CDCl_3$) $\delta$ 7.16-7.23 (m, 2H), 6.96 (d, 1H), 6.66 (d, 1H), 6.54 (d, 1H), 4.99 (s, 2H), 3.66 (s, 3H), 2.67 (d, 2H), 2.52 (s, 3H), 2.19 (s, 3H), 1.94-1.97 (m, 1H), 1.86 (s, 3H), 1.02 (d, 6H).
LC-MS, M/Z (ESI): 476.4 $[M+H]^+$

**Example 34:** Preparation of target compound **I-36**

Butyl ((5-isobutyl-3-(2-methyl-4-((2-methyl-1H-imidazol-1-yl)methyl)phenyl)thiophen-2-yl)sulfonyl)carbamate

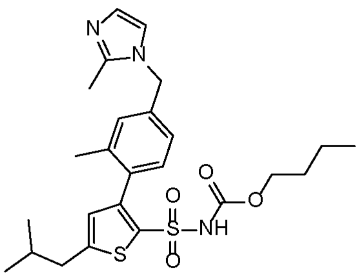

I-36

The synthetic scheme of the target compound **I-36** was as follows:

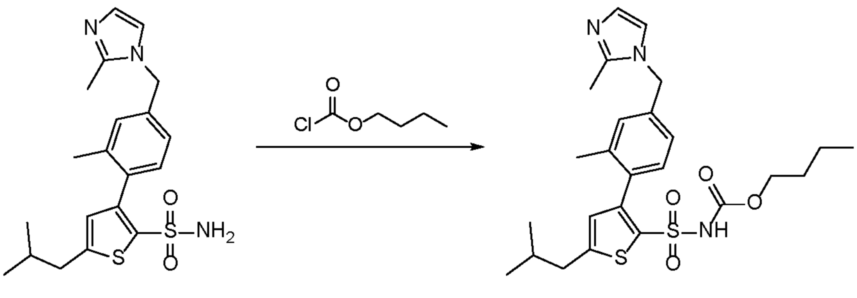

11-4 I-36

Step 1: Synthesis of butyl ((5-isobutyl-3-(2-methyl-4-((2-methyl-1H-imidazol-1-yl)methyl)phenyl)thiophen-2-yl)sulfonyl)carbamate

5-isobutyl-3-(2-methyl-4-((2-methyl-1H-imidazol-1-yl)methyl)phenyl)thiophene-2-sulfonamide (400 mg, 991

μmol) and butyl chloroformate (406 mg, 2.97 mmol) were dissolved in dichloromethane (6 mL), N,N-diisopropylethylamine (384 mg, 2.97 mmol) was added, and the mixture was stirred and reacted at 0°C for 0.5 hour. After the reaction was completed, water (50 mL) was added to quench the reaction, and then the reaction system was extracted with dichloromethane (25 mL×4). The organic phases were combined, dried over anhydrous sodium sulfate, filtered, and concentrated. The crude product was separated and purified by high performance liquid chromatography (column: Phenomenex luna C18 150*40 mm; solvent: A=water + 0.05 volume of formic acid (99%), B=acetonitrile; gradient: 22% to 52%, 15 minutes), and concentrated and dried to obtain butyl ((5-isobutyl-3-(2-methyl-4-((2-methyl-1H-imidazol-1-yl) methyl)phenyl)thiophen-2-yl)sulfonyl)carbamate (96.0 mg, yield 18.3%).

$^1$H NMR (400 MHz, $CDCl_3$) $\delta$ 7.31 (d, 1H), 7.03 (s, 1H), 6.93 (d, 1H), 6.68 (s, 1H), 6.58 (s, 1H), 6.55 (s, 1H), 4.97 (s, 2H), 4.08 (t, 2H), 2.71 (d, 2H), 2.43 (s, 3H), 2.15 (s, 3H), 1.91-2.00 (m, 1H), 1.53-1.61 (m, 2H), 1.26-1.35 (m, 2H), 1.00 (d, 6H), 0.90 (t, 3H).
LC-MS, M/Z (ESI): 504.2 $[M+H]^+$

**Example** 35: Preparation of the following compounds can refer to the preparation of the above-mentioned comoounds.

| No. | Structural formula | Name | LC-MS, M/Z (ESI) |
|---|---|---|---|
| I-9 | 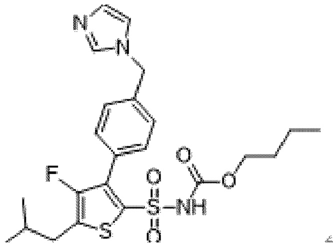 | Butyl (3-(4-((1H-imidazol-1-yl)methyl)phenyl)-4-fluoro-5-isobutylthiophen-2-yl)sulfonylcarbamate | 494.2 |
| I-20 | 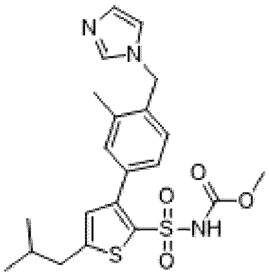 | Methyl (3-(4-((1H-imidazol-1-yl)methyl)-3-methylphenyl)-5-isobutylthiophen-2-yl)sulfonylcarbamate | 448.1 |

In the test examples of the present disclosure, a control compound I was prepared with the method referring to patent WO2021229244A1. The control compound I has the following structure:

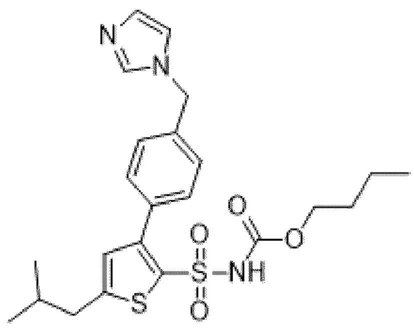

Control Compound I.

**Bioassays**

The following test methods can be adopted.

**Test Example 1: Binding assay of compounds to AT2R**

The assay was performed in accordance with the instruction manual of Angiotensin AT2 Receptor Ligand Binding Assay Kit (#C1TT1AT2, Cisbio). First, a compound stock solution (10 mM) was diluted in a gradient at a 5× dilution ratio (including 10 concentrations, each with two replicates). 160 nL of compounds at different concentrations was added to a 384-well plate. 40 μL of 1× TLB was added to each well, and the mixture was shaken at room temperature for 15 minutes. 15 mL centrifuge tube added with 5 mL of 1×TLB was prepared in advance. The frozen labeled cells were thawed in a water bath at 37°C (for 1 to 2 minutes), and the thawed cells were quickly transferred to the above 15 mL centrifuge tube. The mixture was mixed well and centrifuged at 1,000 g at room temperature for 5 minutes. The supernatant was removed, and the cells were resuspended by adding 2.7 mL 1× TLB. A new 384-well plate was taken, and 10 μL of mixed cells were added into the corresponding wells according to the assay design. 5 μL of 4× compound solution and 5 μL of 4× Tag-lite red fluorescent labeled ligand were added to each well. After incubation at room temperature for 1 hour, data were read using EnVision with an HTRF mode. The excitation light intensities at 665nM and 615nM in each well were read respectively, and the ratio was calculated (Ratio=A665nM/B615nM). The IC50 value was calculated by means of GraphPad Prism8 software, where X represents logarithmic value of compound concentration; and Y represents ratio of A665nM/B615nM.

[Table 1] Binding activity of compounds to AT1R and AT2R

| Compound No. | AT2R IC50 (nM) |
|---|---|
| Control Compound I | 4.46 |
| I-1 | 4.28 |
| I-2 | 3.32 |
| I-3 | 3.95 |
| I-4 | 3.54 |
| I-6 | 11.44 |
| I-7 | 3.80 |
| I-8 | 4.40 |
| I-10 | 2.15 |
| I-11 | 1.51 |
| I-12 | 1.35 |
| I-13 | 1.72 |
| I-14 | 1.68 |
| I-16 | 2.68 |
| I-17 | 2.09 |
| I-18 | 1.63 |
| I-19 | 1.00 |
| I-21 | 2.73 |
| I-22 | 7.28 |
| I-23 | 11.3 |
| I-24 | 1.51 |
| I-25 | 5.65 |
| I-26 | 3.76 |
| I-27 | 3.35 |

(continued)

| Compound No. | AT2R IC50 (nM) |
|---|---|
| Control Compound I | 4.46 |
| I-28 | 4.06 |
| I-29 | 4.50 |
| I-31 | 4.86 |
| I-32 | 1.32 |
| I-33 | 1.61 |
| I-34 | 4.32 |
| I-35 | 3.40 |
| I-36 | 2.39 |

The compounds of the present disclosure have a strong binding activity with AT2R, and the compounds tested in the present disclosure have a very weak binding activity with AT1R, indicating that the compounds have high selectivity.

**Test Example 2: CYP inhibition assay of some compounds**

The inhibitory effects of the compounds on seven enzymes, i.e., CYP1A2, CYP2C9, CYP2C19, CYP2D6, CYP3A4 (midazolam), CYP2B6, and CYP2C8, were evaluated at a concentration of10 μM. Mixed human liver microsomes, which were previously stored in a - 80°C refrigerator before use, were taken out and thawed in a water bath at 37°C before use, and placed on ice for further operation. 100 μL of microsome working solution was taken, and added with 2 μL of test product or positive inhibitor working solution. 2 μL of solvent was added to the solvent control. The mixture was preincubated in a water bath at 37°C for 10 minutes. After preincubation, 98 μL of NADPH regeneration solution was added to all samples to initiate the reaction. Then, the samples were put back into the water bath and incubated for a period of time. The reaction was quenched with 200 μL of stop solution. The plate was centrifuged at 3,220 g for 10 minutes, 100 μL of supernatant was transferred, and 100 μL of water was added and mixed well to the assay plate for LC/MS/MS analysis.

[Table 2] CYP450 inhibition results of some compounds

| | **Inhibition rate %** | | | | | | |
|---|---|---|---|---|---|---|---|
| Compound No. | CYP1A2 | CYP2C9 | CYP2C19 | CYP2D6 | CYP3A4 (midazolam) | CYP2B6 | CYP2C8 |
| Control Compound I | 95.38 | 100.00 | 100.00 | 86.46 | 92.64 | 63.19 | 97.48 |
| I-11 | 0 | 28.34 | 11.03 | 1.97 | 13.39 | 27.61 | 12.61 |
| I-12 | 4.18 | 45.75 | 30.24 | 10.43 | 21.13 | 31.55 | 39.49 |
| I-13 | 0.54 | 20.24 | 11.70 | 6.50 | 0 | 18.33 | 82.65 |
| I-14 | 0 | 32.79 | 21.70 | 11.42 | 0 | 16.94 | 50.64 |
| I-16 | 25.04 | 46.82 | 35.32 | 9.54 | 6.78 | 11.06 | 33.64 |
| I-34 | 2.82 | 77.30 | 36.48 | 12.64 | 3.80 | 13.65 | 71.75 |

Compared with the control compound, the compounds of the present disclosure have a relatively weaker inhibitory effect on CYP enzymes and they would not affect the normal metabolism of the body, indicating a lower probability of drug-drug interaction in the human body and higher safety.

**Test Example 3: Free plasma protein binding (PPB) assay of some compounds**

The dried dialysis membrane was soaked in ultrapure water for 1 hour, then soaked in 20% ethanol for 20 minutes, and then rinsed with ultrapure water for 2 to 3 times. Finally, the dried dialysis membrane was soaked in ultrapure water for 20 minutes for later use. The frozen plasma was thawed in a water bath at 37°C for about 20 minutes. pH value of

the plasma was measured after thawing was complete, and was adjusted to 7.4 with 1% phosphoric acid solution or 0.1M sodium hydroxide solution. The test compound was diluted into plasma preheated to 37°C to a final concentration of 1 μM, and the control compound warfarin in plasma had a final concentration of 2 μM. The pretreated dialysis membrane was assembled into the dialysis plate according to product instructions, and 100 μL of receiving solution (100 mM phosphate buffer solution plus 0.002% Tween 80) was added to one side of the permeable membrane in each dialysis well. 20 μL of each of the final solution of the test compound and the final solution of the control compound were taken in a 96-well sample plate, two duplicate samples were made, and $T_0$ sample was obtained, which was stored in a -20°C refrigerator. Another 100 μL of each of the above-mentioned final solutions were taken to the other side of the membrane in the dialysis device, two duplicate samples were made, and incubated at 37°C with constant shaking for 6 hours. After 6 hours of incubation, 20 μL of each of the dialyzed receiving solution and the administered plasma were taken, two duplicate samples were made, and sample A and sample B were obtained. Corresponding blank plasma or receiving solution of corresponding volume were added to sample B and sample A, respectively, allowing the volume ratio of plasma to buffer in each sample well to be 1:1. 300 μL of sample-containing internal standard acetonitrile solution was added to all the sample wells. The mixture was mixed well, and centrifuged at 5,500×g for 10 minutes. 150 μL of corresponding ultrapure water was added to the corresponding sample wells of the 96-well sample plate. 150 μL of the supernatant was taken into the sample wells, and mixed well. Then, LC/MS/MS analysis was performed. The calculation formula of $f_u$ is:

$$f_u = \frac{C_R}{C_D} \times 100\%,$$

where:

$C_R$: peak area ratio measured in the receiving chamber well
$C_D$: peak area ratio measured in the supply chamber well.

[Table 3] Plasma protein binding rate of some compounds

| | fu % | | | |
|---|---|---|---|---|
| Compound No. | Human | Rat | Mouse | Canine |
| Control Compound I | 0.06 | 0.05 | 0.09 | 0.23 |
| I-11 | 0.39 | 0.97 | 1.21 | 1.85 |
| I-13 | - | 0.43 | 2.11 | 1.16 |
| I-14 | - | - | 4.27 | - |
| I-16 | 1.26 | 1.09 | - | 3.49 |
| I-34 | 0.80 | 2.89 | 0.93 | 1.41 |

The degree of freeness of a compound in plasma is related to its efficacy in the body. Compared with the control compound, the compound in the present disclosure has a higher degree of freeness, which is beneficial for the compound to reach the target organ.

**Test Example 4: Liver microsome stability assay of some compounds**

The human liver microsome stability assay was performed by co-incubating the compound with human liver microsomes *in vitro*. The test compounds were first prepared as 10 mM stock solutions in DMSO solvent, and then diluted to 0.5 mM with acetonitrile. Human liver microsomes (Corning) were diluted with PBS to prepare a microsome/buffer solution, and this solution was used to dilute 0.5 mM of the compound to prepare a working solution. In the working solution, a concentration of the compound was 1.5 μM and a concentration of the human liver microsome was 0.75 mg/ml. A deep-well plate was taken, 30 μL of working solution was added to each well of the plate, and then 15 μL of pre-heated NADPH solution (6 mM) was added to start the reaction. The mixture was incubated at 37°C. 135 μL of acetonitrile was added to the corresponding wells to stop the reaction at 0 minute, 5 minutes, 15 minutes, 30 minute, and 45 minutes of incubation. Subsequent to the stop of the reaction by means of acetonitrile at the last time point of 45 minute, the plate was vortexed for 10 minutes (600 rpm/min) and then centrifuged for 15 minutes. After centrifugation, the supernatant was taken, and

purified water was added in a 1:1 ratio for LC-MS/MS detection. The ratio of a peak area of the compound to a peak area of the internal standard at individual time points was obtained. The peak area ratios of the compound at 5 minutes, 15 minutes, 30 minutes, and 45 minutes were compared with the peak area ratio at 0 minute, and the remaining percentage of the compound at each time point was calculated. $T_{1/2}$ was calculated using Graphpad8 software.

[Table 4] Liver microsomal stability of some compounds

| | $T_{1/2}$ (min) | | | |
|---|---|---|---|---|
| Compound No. | Human | Rat | Mouse | Canine |
| Control Compound I | 9.13 | 22.57 | 49.15 | 223.55 |
| I-16 | 238.97 | 83.49 | 1732.50 | 577.50 |
| I-34 | 45.00 | - | 61.88 | 121.58 |

The stability of a compound in liver microsomes reflects its risk of being metabolized and eliminated *in vivo.* The test compound has better stability in humans compared to the control compound.

**Test Example 5: Bidirectional permeability study of compounds in CACO-2 cells**

Caco-2 cells were inoculated onto polyethylene membrane (PET) in 96-well inserted plates at a rate of $1\times10^5$ cells/$cm^2$, and the medium was changed every 4 to 5 days until day 21 to 28, to form a fused cell monolayer. The transport buffer in the assay was HBSS and 10.0 mM HEPES, with a pH of 7.40±0.05. The test compounds were subj ected to a bidirectional test at 2.00 μM in the presence and absence of GF120918 (10.0 μM). Digoxin was subjected to a bidirectional test at 10.0 μM in the presence and absence of GF120918 (10.0 μM), while Nadolol and Metoprolol were subjected to a test from direction A to direction B at 2.00 μM in the absence of GF120918 (10.0 μM). The above tests were performed in duplicate. The final concentration of DMSO was adjusted to be lower than 1%. The plate was incubated in a $CO_2$ incubator at 37°C±1°C for 2 hours, and 5% $CO_2$ was added under saturated humidity without shaking. All the samples, after being mixed with acetonitrile containing internal standard, were centrifuged at 3,200 g for 10 minutes. For Nadolol and Metoprolol, 200 μL of supernatant was diluted with 600 μL of distilled water for LC-MS/MS analysis. For Digoxin and test compounds, 200 μL of supernatant was diluted with 200 μL of distilled water for LC-MS/MS analysis. The concentrations of the test compounds and control compounds in the starting solution, the donor solution, and the acceptor solution were quantified by LC-MS/MS based on a peak area ratio of the analyte to the internal standard.

After the transport assay, the integrity of the Caco-2 cell monolayer was determined using Lucifer Yellow Exclusion Assay.

[Table 5] Bidirectional permeability of compounds in CACO-2 cells

| Compound No. | $P_{app}$ mean value ($10^{-6}$ cm/s) |
|---|---|
| | A to B |
| Control Compound I | 0.381 |
| I-34 | 3.13 |

Compared with the control compound, the permeability of the test compound was improved, which was beneficial to the absorption of the compound.

In the specification, the description of the reference terms such as "one embodiment", "some embodiments", "example", "specific example", or "some example" means that the specific features, structures, materials, or characteristics described with reference to the embodiment or example are included in at least an embodiment or example of the present disclosure. In this specification, exemplary descriptions of the foregoing terms do not necessarily refer to the same embodiment or example. Moreover, the specific features, structures, materials, or characteristics described may be combined in any one or more embodiments or examples in a suitable manner. Furthermore, those skilled in the art may combine different embodiments or examples and features of different embodiments or examples described in this specification, unless they are contradictory to each other.

Although embodiments of the present disclosure are illustrated and described above, it can be understood that the above embodiments are illustrative and should not be construed as limitations of the present disclosure. Those skilled in the art can make changes, modifications, substitutions, and variations to the above embodiments within the scope of the present disclosure.

## Claims

1. A compound represented by Formula I, or a tautomer, stereoisomer, hydrate, solvate, pharmaceutically acceptable salt, or prodrug thereof:

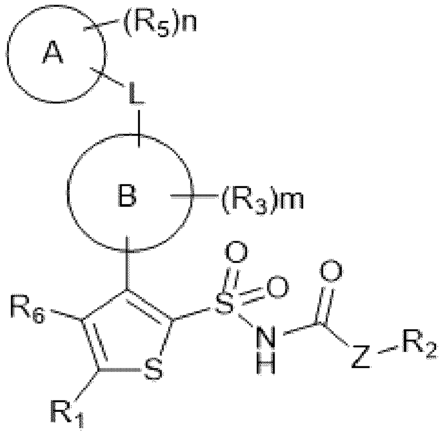

Formula I,

wherein:

ring B is selected from a benzene ring or a 5- to 6-membered heteroaromatic ring, wherein:

when ring B is the benzene ring, when $R_6$ is hydrogen, and when m is selected from 1, 2, 3, or 4, L is unsubstituted $C_1$ to $C_6$ alkyl or $C_1$ to $C_6$ alkyl substituted with one or more substituents, each of the one or more substituents being independently selected from halogen, $C_1$ to $C_6$ alkyl, halogenated $C_1$ to $C_6$ alkyl, or oxo (=O), and when a plurality of substituents are present, the plurality of substituents are the same or different; or
when ring B is the benzene ring, when $R_6$ is hydrogen, and when m is selected from 0, L is $C_1$ to $C_6$ alkyl substituted with one or more substituents, each of the one or more substituents being independently selected from halogen, $C_1$ to $C_6$ alkyl, halogenated $C_1$ to $C_6$ alkyl, or oxo (=O), and when a plurality of substituents are present, the plurality of substituents are the same or different; or
when ring B is the benzene ring, and when $R_6$ is selected from halogen, hydroxyl, cyano, amino, $C_1$ to $C_6$ alkyl, $C_3$ to $C_7$ cycloalkyl, $C_1$ to $C_6$ alkoxy, or -O-$C_3$-$C_7$ cycloalkyl, m is selected from 0, 1, 2, 3, or 4, and L is unsubstituted $C_1$ to $C_6$ alkyl or $C_1$ to $C_6$ alkyl substituted with one or more substituents, each of the one or more substituents being independently selected from halogen, $C_1$ to $C_6$ alkyl, halogenated $C_1$ to $C_6$ alkyl, or oxo (=O), wherein $R_6$ is independently substituted with 0, 1, 2, 3, or 4 substituents selected from halogen, hydroxyl, amino, cyano, $C_1$ to $C_6$ alkyl, or $C_3$ to $C_7$ cycloalkyl, when a plurality of substituents are present, the plurality of substituents are the same or different; or
when ring B is the 5- to 6-membered heteroaromatic ring, m is selected from 0, 1, 2, 3, or 4, L is unsubstituted $C_1$ to $C_6$ alkyl or $C_1$ to $C_6$ alkyl substituted with one or more substituents, each of the one or more substituents being independently selected from halogen, $C_1$ to $C_6$ alkyl, halogenated $C_1$ to $C_6$ alkyl, or oxo (=O), and $R_6$ is hydrogen or selected from halogen, hydroxyl, cyano, amino, $C_1$ to $C_6$ alkyl, $C_3$ to $C_7$ cycloalkyl, $C_1$ to $C_6$ alkoxy, or -O-$C_3$-$C_7$ cycloalkyl, $R_6$ is independently substituted with 0, 1, 2, 3, or 4 substituents selected from halogen, hydroxyl, amino, cyano, $C_1$ to $C_6$ alkyl, or $C_3$ to $C_7$ cycloalkyl, and when a plurality of substituents are present, the plurality of substituents are the same or different;

$R_1$ and $R_3$ are each independently selected from hydroxyl, cyano, amino, oxo (=O), $C_1$ to $C_6$ alkyl, $C_3$ to $C_7$ cycloalkyl, $C_1$ to $C_6$ alkoxy, or -O-$C_3$-$C_7$ cycloalkyl;
$R_1$ and $R_3$ are each independently substituted with 0, 1, 2, 3, or 4 substituents selected from halogen, hydroxyl, amino, cyano, $C_1$ to $C_6$ alkyl, or $C_3$ to $C_7$ cycloalkyl, when a plurality of substituents are present, the plurality of substituents are the same or different;
$R_2$ is selected from $C_1$ to $C_6$ alkyl, halogenated $C_1$ to $C_6$ alkyl, $C_3$ to $C_7$ cycloalkyl, $C_1$ to $C_6$ alkoxy, or $C_3$ to $C_7$ cycloalkyl-$C_1$ to $C_6$ alkyl;

$R_2$ is substituted with 0, 1, 2, 3, or 4 substituents selected from halogen, hydroxyl, amino, cyano, $C_1$ to $C_6$ alkyl, $C_3$ to $C_7$ cycloalkyl, 5- to 6-membered aryl, or 5- to 6-membered heteroaryl, when a plurality of substituents are present, the plurality of substituents are the same or different;
Z is selected from -O- or -$NR_4$-;
$R_4$ is $C_1$ to $C_3$ alkyl or H;
ring A is a 5- to 6-membered heteroaryl;
n is selected from 1, 2, 3, 4, or 5, when a plurality of substituents $R_5$ are present, the plurality of substituents $R_5$ are the same or different; and
$R_5$ is selected from H, halogen, -CN, $C_1$ to $C_6$ alkyl, $C_1$ to $C_6$ alkoxy, $C_3$ to $C_7$ cycloalkyl, or -O-$C_3$-$C_7$ cycloalkyl, when $R_5$ is $C_1$ to $C_6$ alkyl, $C_1$ to $C_6$ alkoxy, $C_3$ to $C_7$ cycloalkyl, or -O-$C_3$-$C_7$ cycloalkyl, the $C_1$ to $C_6$ alkyl, $C_1$ to $C_6$ alkoxy, $C_3$ to $C_7$ cycloalkyl, or -O-$C_3$-$C_7$ cycloalkyl is independently substituted with 0, 1, 2, 3, or 4 substituents selected from halogen, hydroxyl, $C_1$ to $C_6$ alkyl, or $C_3$ to $C_7$ cycloalkyl, and when a plurality of substituents are present, the plurality of substituents are the same or different.

**2.** The compound represented by Formula I, or the tautomer, stereoisomer, hydrate, solvate, pharmaceutically acceptable salt, or prodrug thereof according to claim 1, wherein the compound has the following structure:

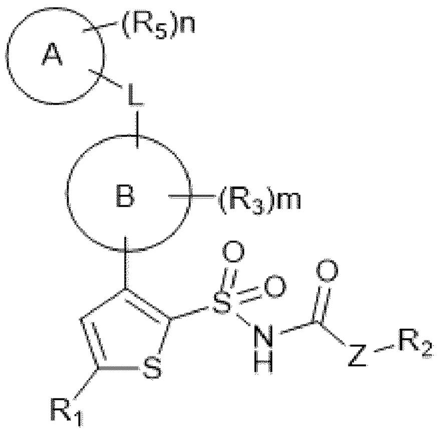

II,

wherein:

L is unsubstituted $C_1$ to $C_6$ alkyl or $C_1$ to $C_6$ alkyl substituted with one or more substituents, each of the one or more substituents being independently selected from halogen, $C_1$ to $C_6$ alkyl, halogenated $C_1$ to $C_6$ alkyl, or oxo (=O);
ring B is selected from a benzene ring or a 6 membered heteroaromatic ring, when ring B is the benzene ring, m is selected from 1, 2, 3, or 4, and when ring B is the 6 membered heteroaromatic ring, m is selected from 0, 1, 2, 3, or 4;
$R_1$ and $R_3$ are each independently selected from hydroxyl, cyano, amino, oxo (=O), $C_1$ to $C_6$ alkyl, $C_3$ to $C_7$ cycloalkyl, $C_1$ to $C_6$ alkoxy, or -O-$C_3$-$C_7$ cycloalkyl;
$R_1$ and $R_3$ are each independently substituted with 0, 1, 2, 3, or 4 substituents selected from halogen, hydroxyl, amino, cyano, $C_1$ to $C_6$ alkyl, or $C_3$ to $C_7$ cycloalkyl, when a plurality of substituents are present, the plurality of substituents are the same or different;
$R_2$ is selected from $C_1$ to $C_6$ alkyl, halogenated $C_1$ to $C_6$ alkyl, $C_3$ to $C_7$ cycloalkyl, $C_1$ to $C_6$ alkoxy, or $C_3$ to $C_7$ cycloalkyl-$C_1$ to $C_6$ alkyl;
$R_2$ is substituted with 0, 1, 2, 3, or 4 substituents selected from halogen, hydroxyl, amino, cyano, $C_1$ to $C_6$ alkyl, $C_3$ to $C_7$ cycloalkyl, 5- to 6-membered aryl, or 5- to 6-membered heteroaryl, when a plurality of substituents are present, the plurality of substituents are the same or different;
Z is selected from -O- or -$NR_4$-;
$R_4$ is $C_1$ to $C_3$ alkyl or H;
ring A is 5- to 6-membered heteroaryl;
n is selected from 1, 2, 3, 4, or 5, when a plurality of substituents $R_5$ are present, the plurality of substituents $R_5$ are the same or different; and
$R_5$ is selected from H, halogen, -CN, $C_1$ to $C_6$ alkyl, $C_1$ to $C_6$ alkoxy, $C_3$ to $C_7$ cycloalkyl, or -O-$C_3$-$C_7$ cycloalkyl,

when $R_5$ is $C_1$ to $C_6$ alkyl, $C_1$ to $C_6$ alkoxy, $C_3$ to $C_7$ cycloalkyl, or -O-$C_3$-$C_7$ cycloalkyl, the $C_1$ to $C_6$ alkyl, $C_1$ to $C_6$ alkoxy, $C_3$ to $C_7$ cycloalkyl, or -O-$C_3$-$C_7$ cycloalkyl is independently substituted with 0, 1, 2, 3, or 4 substituents selected from halogen, hydroxyl, $C_1$ to $C_6$ alkyl, or $C_3$ to $C_7$ cycloalkyl, and when a plurality of substituents are present, the plurality of substituents are the same or different.

**3.** The compound represented by Formula I, or the tautomer, stereoisomer, hydrate, solvate, pharmaceutically acceptable salt, or prodrug thereof according to claim 1, wherein the compound has the following structure:

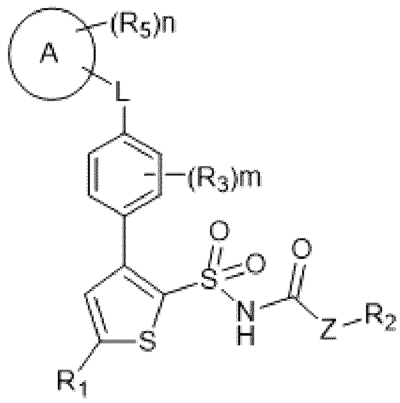

III,

wherein:

L is $C_1$ to $C_6$ alkyl substituted with one or more substituents, each of the one or more substituents being independently selected from halogen, $C_1$ to $C_6$ alkyl, halogenated $C_1$ to $C_6$ alkyl, or oxo (=O); and
m is selected from 0, 1, 2, 3, or 4.

**4.** The compound represented by Formula I, or the tautomer, stereoisomer, hydrate, solvate, pharmaceutically acceptable salt, or prodrug thereof according to claim 1, wherein the compound has the following structure:

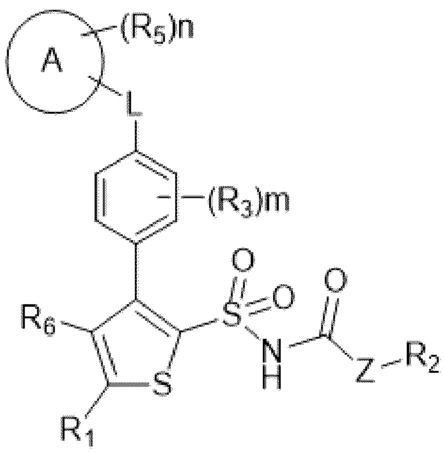

IV,

wherein:

when $R_6$ is selected from halogen, hydroxyl, cyano, amino, $C_1$ to $C_6$ alkyl, $C_3$ to $C_7$ cycloalkyl, $C_1$ to $C_6$ alkoxy, or -O-$C_3$-$C_7$ cycloalkyl, $R_6$ is independently substituted with 0, 1, 2, 3, or 4 substituents selected from halogen, hydroxyl, amino, cyano, $C_1$ to $C_6$ alkyl, or $C_3$ to $C_7$ cycloalkyl, when a plurality of substituents are present, the plurality of substituents are the same or different;
m is selected from 0, 1, 2, 3, or 4; and

L is unsubstituted $C_1$ to $C_6$ alkyl or $C_1$ to $C_6$ alkyl substituted with one or more substituents, each of the one or more substituents being independently selected from halogen, $C_1$ to $C_6$ alkyl, halogenated $C_1$ to $C_6$ alkyl, or oxo (=O).

**5.** The compound represented by Formula I, or the tautomer, stereoisomer, hydrate, solvate, pharmaceutically acceptable salt, or prodrug thereof according to claim 1, wherein the compound has the following structure:

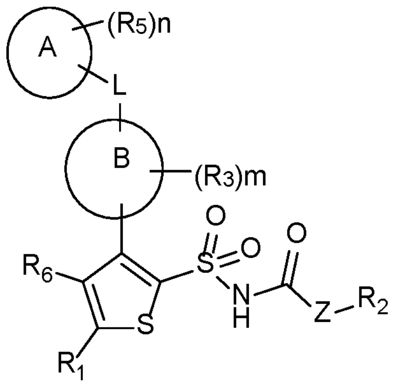

V,

wherein:

ring B is selected from a 5- to 6-membered heteroaryl;
$R_5$ is selected from H;
when $R_6$ is hydrogen or selected from halogen, hydroxyl, cyano, amino, $C_1$ to $C_6$ alkyl, $C_3$ to $C_7$ cycloalkyl, $C_1$ to $C_6$ alkoxy, or -O-$C_3$-$C_7$ cycloalkyl, $R_6$ is independently substituted with 0, 1, 2, 3, or 4 substituents selected from halogen, hydroxyl, amino, cyano, $C_1$ to $C_6$ alkyl, or $C_3$ to $C_7$ cycloalkyl, when a plurality of substituents are present, the plurality of substituents are the same or different;
m is selected from 0, 1, 2, 3, or 4;
L is unsubstituted $C_1$ to $C_6$ alkyl or $C_1$ to $C_6$ alkyl substituted with one or more substituents, each of the one or more substituents being independently selected from halogen, $C_1$ to $C_6$ alkyl, halogenated $C_1$ to $C_6$ alkyl, or oxo (=O);
preferably, ring B is the 5- to 6-membered heteroaryl, $R_5$ and $R_6$ are both hydrogen; and m is 0;

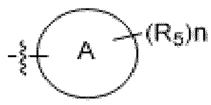

has a structure of

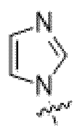

,

and $R_1$ is selected from isobutyl;
Z is selected from -O-, and $R_2$ is selected from $C_1$ to $C_6$ alkyl; and
preferably, the 5- to 6-membered heteroaryl is selected from a pyridine ring.

**6.** The compound, or the tautomer, stereoisomer, hydrate, solvate, pharmaceutically acceptable salt, or prodrug thereof according to any one of claims 1 to 5, wherein:

L is $C_1$ to $C_3$ alkyl;
L is optionally substituted with halogen, $C_1$ to $C_6$ alkyl, halogenated $C_1$ to $C_6$ alkyl, or oxo (=O);
preferably, L is $C_1$ to $C_3$ alkyl or $C_1$ to $C_3$ alkyl substituted with $C_1$ to $C_6$ alkyl; and
more preferably, L is $-CH_2-$ or $-CH(CH_3)-$.

**7.** The compound, or the tautomer, stereoisomer, hydrate, solvate, pharmaceutically acceptable salt, or prodrug thereof according to any one of claims 1 to 5, wherein:

$R_3$ is selected from hydroxyl, cyano, amino, oxo (=O), $C_1$ to $C_3$ alkyl, $C_3$ to $C_7$ cycloalkyl, $C_1$ to $C_3$ alkoxy, or -O-$C_3$-$C_7$ cycloalkyl;
$R_3$ is independently substituted with 0, 1, 2, 3, or 4 substituents selected from halogen, hydroxyl, amino, cyano, $C_1$ to $C_6$ alkyl, or $C_3$ to $C_7$ cycloalkyl, when a plurality of substituents are present, the plurality of substituents are the same or different;
preferably, $R_3$ is selected from cyano, methyl, methoxy, halogenated methyl, or cyclopropyl; and
more preferably, $R_3$ is selected from cyano or methyl.

**8.** The compound, or the tautomer, stereoisomer, hydrate, solvate, pharmaceutically acceptable salt, or prodrug thereof according to claim 1, wherein:

ring B is a 5- to 6-membered heteroaromatic ring having 1 or 2 heteroatoms of N;
preferably, ring B is selected from a pyridine ring, a pyrimidine ring, a pyrazine ring, or a pyridazine ring;
$R_3$ is selected from hydroxy, cyano, amino, oxo (=O), $C_1$ to $C_6$ alkyl, $C_3$ to $C_7$ cycloalkyl, $C_1$ to $C_6$ alkoxy, or -O-$C_3$-$C_7$ cycloalkyl;
$R_3$ is independently substituted with 0, 1, 2, 3, or 4 substituents selected from halogen, hydroxyl, amino, cyano, $C_1$ to $C_6$ alkyl, or $C_3$ to $C_7$ cycloalkyl, when a plurality of substituents are present, the plurality of substituents are the same or different;
preferably, $R_3$ is selected from cyano, $C_1$ to $C_3$ alkyl, $C_3$ to $C_6$ cycloalkyl, halogenated $C_1$ to $C_3$ alkyl, or $C_1$ to $C_3$ alkoxy;
more preferably, $R_3$ is selected from cyano, methyl, methoxy, halogenated methyl, or cyclopropyl; and
preferably, m is 0.

**9.** The compound, or the tautomer, stereoisomer, hydrate, solvate, pharmaceutically acceptable salt, or prodrug thereof according to any one of claims 1 to 4, wherein:

ring B is a benzene ring substituted with 1, 2, 3, or 4 of substituents $R_3$, and $R_3$ is selected from hydroxyl, cyano, amino, oxo (=O), $C_1$ to $C_6$ alkyl, $C_3$ to $C_7$ cycloalkyl, $C_1$ to $C_6$ alkoxy, or -O-$C_3$-$C_7$ cycloalkyl;
$R_3$ is independently substituted with 0, 1, 2, 3, or 4 substituents selected from halogen, hydroxyl, amino, cyano, $C_1$ to $C_6$ alkyl, or $C_3$ to $C_7$ cycloalkyl, when a plurality of substituents are present, the plurality of substituents are the same or different;
preferably, $R_3$ is selected from cyano, $C_1$ to $C_3$ alkyl, $C_3$ to $C_6$ cycloalkyl, halogenated $C_1$ to $C_3$ alkyl, or $C_1$ to $C_3$ alkoxy;
more preferably, $R_3$ is selected from cyano, methyl, methoxy, halogenated methyl, or cyclopropyl; and
more preferably, $R_3$ is selected from cyano or methyl.

**10.** The compound, or the tautomer, stereoisomer, hydrate, solvate, pharmaceutically acceptable salt, or prodrug thereof according to any one of claims 1 to 5, wherein:

$R_1$ is selected from $C_1$ to $C_6$ alkyl, halogenated $C_1$ to $C_6$ alkyl, $C_3$ to $C_7$ cycloalkyl, or $C_3$ to $C_7$ cycloalkyl-$C_1$ to $C_6$ alkyl;
preferably, $R_1$ is selected from $C_1$ to $C_6$ alkyl; and
more preferably, $R_1$ is isobutyl.

**11.** The compound, or the tautomer, stereoisomer, hydrate, solvate, pharmaceutically acceptable salt, or prodrug thereof according to any one of claims 1 to 5, wherein:

$R_2$ is selected from $C_1$ to $C_6$ alkyl or halogenated $C_1$ to $C_6$ alkyl;
$R_2$ is substituted with 0, 1, 2, 3, or 4 substituents selected from halogen, hydroxyl, amino, cyano, $C_1$ to $C_6$ alkyl, $C_3$ to $C_7$ cycloalkyl, 5- to 6-membered aryl, or 5- to 6-membered heteroaryl, when a plurality of substituents are present, the plurality of substituents are the same or different;
preferably, $R_2$ is selected from methyl, ethyl, propyl, isopropyl, trifluoropropyl, butyl, isobutyl, or $C_1$ to $C_3$ alkyl-6 membered heteroaryl;
preferably, $R_2$ is selected from methyl, ethyl, propyl, isopropyl, butyl, or isobutyl;
more preferably, $R_2$ is methyl, trifluoropropyl, butyl, or pyridylmethyl; and

more preferably, $R_2$ is butyl.

**12.** The compound, or the tautomer, stereoisomer, hydrate, solvate, pharmaceutically acceptable salt, or prodrug thereof according to any one of claims 1 to 5, wherein:

Z is selected from -O-, -NH-, or -N-$C_1$ to $C_3$ alkyl-;
preferably, Z is -O- or -NH-; and
preferably, Z is -NH-.

**13.** The compound, or the tautomer, stereoisomer, hydrate, solvate, pharmaceutically acceptable salt, or prodrug thereof according to any one of claims 1 to 5, wherein:

ring A is 5- to 6-membered heteroaryl containing 1, 2, and 3 heteroatoms;
n is 1, 2, 3, 4, or 5;
preferably, the heteroatom is selected from N, O, or S; and more preferably, the heteroatom is N;
preferably, ring A is selected from pyrrole, pyrazole, imidazole, triazole, pyridine, pyrimidine, pyridazine, pyrazine, or triazine;
more preferably,

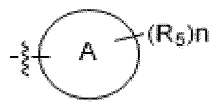

has a structure of

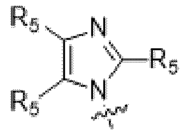,
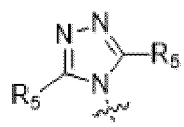,

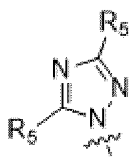,
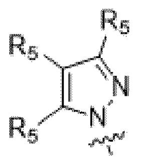, or
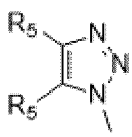;

$R_5$ is selected from H, halogen, -CN, $C_1$ to $C_6$ alkyl, $C_1$ to $C_6$ alkoxy, $C_3$ to $C_7$ cycloalkyl, or -O-$C_3$-$C_7$ cycloalkyl, when a plurality of substituents $R_5$ are present, the plurality of substituents $R_5$ are the same or different;
preferably, $R_5$ is selected from H, halogen, methyl, ethyl, tert-butyl, cyclopropyl, or halogenated methyl;
preferably, $R_5$ is selected from H, halogen, methyl, cyclopropyl, or halogenated methyl; and
more preferably,

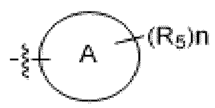

has a structure of

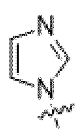,
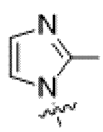,
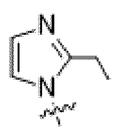,

or

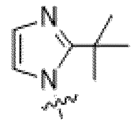

.

**14.** The compound, or the tautomer, stereoisomer, hydrate, solvate, pharmaceutically acceptable salt, or prodrug thereof according to any one of claims 1 to 5, wherein:

$R_6$ is selected from H, halogen, hydroxyl, cyano, amino, $C_1$ to $C_3$ alkyl, $C_1$ to $C_3$ alkoxy, $C_3$ to $C_7$ cycloalkyl, or -O-$C_3$-$C_7$ cycloalkyl;
$R_6$ is independently substituted with 0, 1, 2, 3, or 4 substituents selected from halogen, hydroxyl, amino, cyano, $C_1$ to $C_6$ alkyl, or $C_3$ to $C_7$ cycloalkyl, when a plurality of substituents are present, the plurality of substituents are the same or different;
preferably, $R_6$ is selected from H, halogen, hydroxyl, cyano, amino, $C_1$ to $C_3$ alkyl, $C_1$ to $C_3$ alkoxy, or $C_3$ to $C_7$ cycloalkyl;
preferably, $R_6$ is selected from H, halogen, or $C_1$ to $C_3$ alkyl; and
more preferably, $R_6$ is selected from H, fluorine, or methyl.

**15.** The compound, or the tautomer, stereoisomer, hydrate, solvate, pharmaceutically acceptable salt, or prodrug thereof according to claim 1, wherein:

when ring B is the benzene ring, $R_6$ is H, and m is selected from 1, 2, 3, or 4, L is $C_1$ to $C_3$ alkyl or $C_1$ to $C_3$ alkyl substituted with $C_1$ to $C_6$ alkyl;

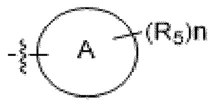

has a structure of

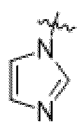 , 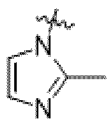 , 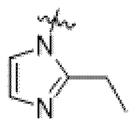 , 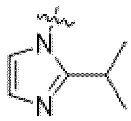 ,

or

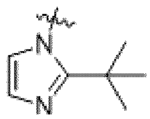 ;

$R_1$ is selected from isobutyl;
$R_3$ is selected from cyano or methyl;
Z is selected from -O- or -NH-;
$R_2$ is selected from $C_1$ to $C_6$ alkyl;
$R_2$ is substituted with 0, 1, 2, or 3 substituents selected from halogen, hydroxyl, or 5- to 6-membered heteroaryl; and
preferably, L is $-CH_2-$ or $-CH(CH_3)-$.

**16.** The compound, or the tautomer, stereoisomer, hydrate, solvate, pharmaceutically acceptable salt, or prodrug thereof according to claim 1, wherein:

when ring B is the benzene ring, $R_6$ is H, and m is 0, L is $C_1$ to $C_3$ alkyl or $C_1$ to $C_3$ alkyl substituted with $C_1$ to $C_6$ alkyl;

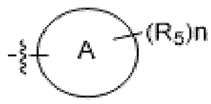

has a structure of

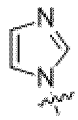 or 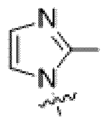 ;

$R_1$ is selected from isobutyl;
$R_3$ is selected from cyano or methyl;
Z is selected from -O-;
$R_2$ is selected from $C_1$ to $C_6$ alkyl; and
preferably, L is $-CH_2-$ or $-CH(CH_3)-$.

**17.** The compound, or the tautomer, stereoisomer, hydrate, solvate, pharmaceutically acceptable salt, or prodrug thereof according to claim 1, wherein:

when ring B is the benzene ring, $R_6$ is not hydrogen and is selected from halogen or $C_1$ to $C_3$ alkyl, and m is 0 or 1, L is $C_1$ to $C_3$ alkyl or $C_1$ to $C_3$ alkyl substituted with $C_1$ to $C_6$ alkyl;

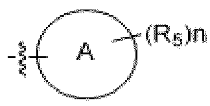

has a structure of

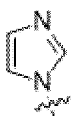 , 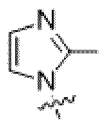 , 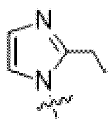 , 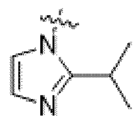 ,

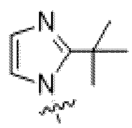 , or 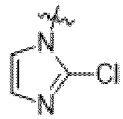 ;

$R_1$ is selected from isobutyl;
$R_3$ is selected from methyl or halogen;
Z is selected from -O-;
$R_2$ is selected from $C_1$ to $C_6$ alkyl; and
preferably, $R_6$ is selected from fluorine or methyl.

**18.** The compound, or the tautomer, stereoisomer, hydrate, solvate, pharmaceutically acceptable salt, or prodrug thereof according to any one of claims 1 to 5, wherein the compound comprises:

| | | |
|---|---|---|
| 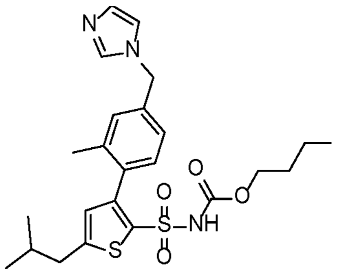 | 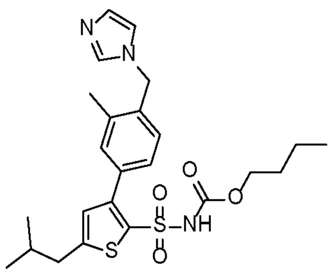 | 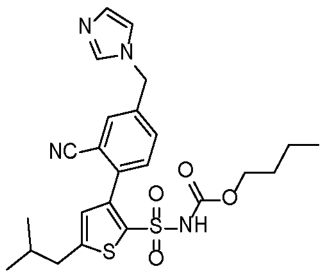 |
| I-1 | I-2 | I-3 |
| 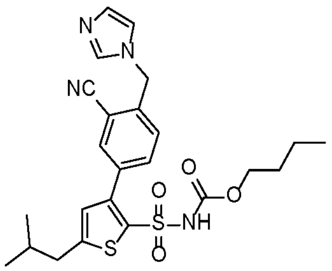 | 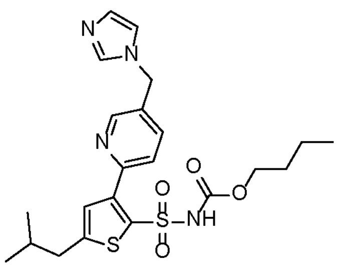 | 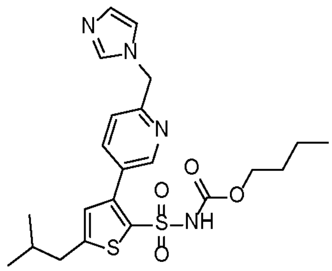 |
| I-4 | I-5 | I-6 |
| 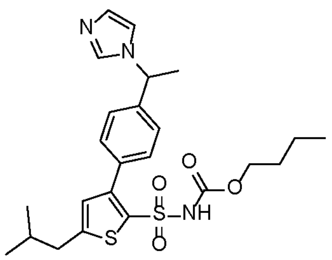 | 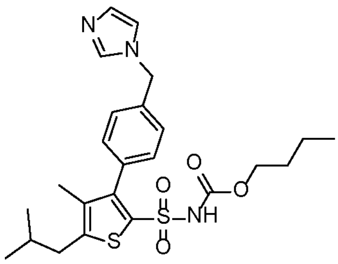 | 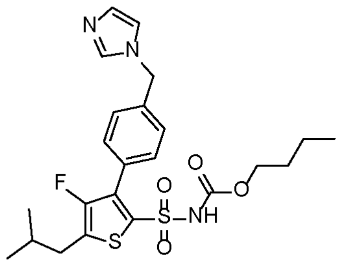 |
| I-7 | I-8 | I-9 |
| 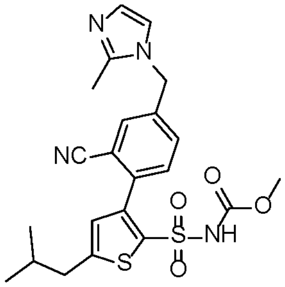 | 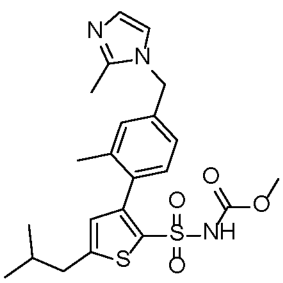 | 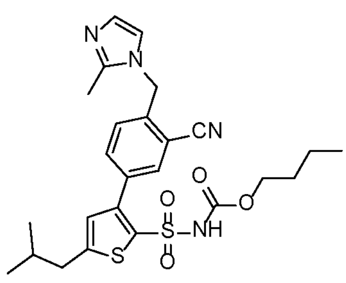 |
| I-10 | I-11 | I-12 |
| 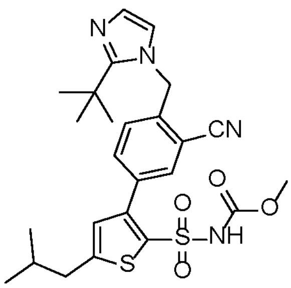 | 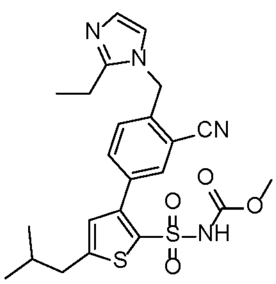 | 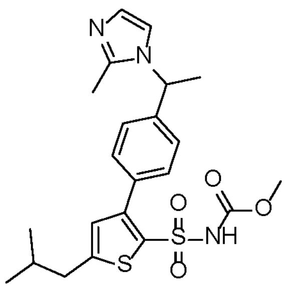 |
| I-13 | I-14 | I-15 |

(continued)

| | | |
|---|---|---|
| 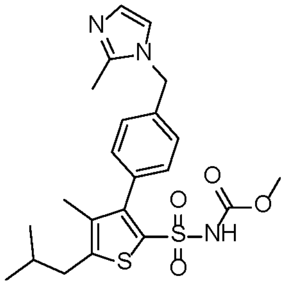 | 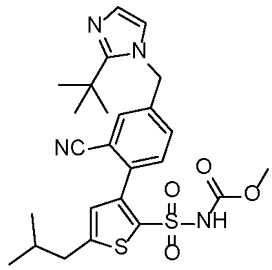 | 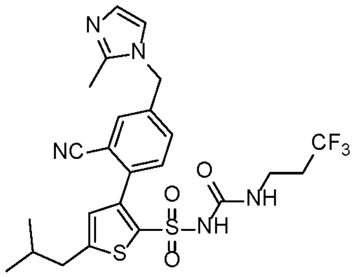 |
| I-16 | I-17 | I-18 |
| 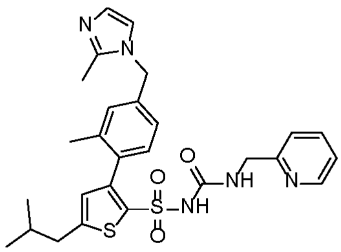 | 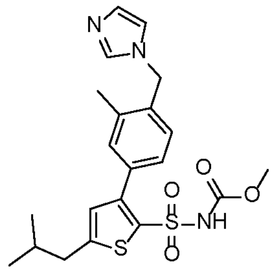 | 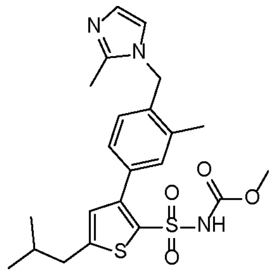 |
| I-19 | I-20 | I-21 |
| 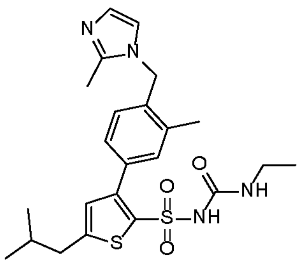 | 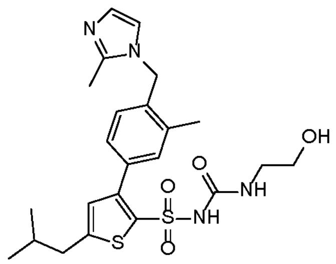 | 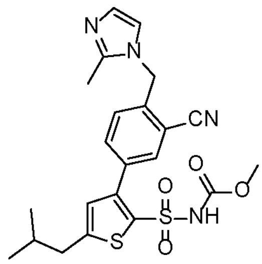 |
| I-22 | I-23 | I-24 |
| 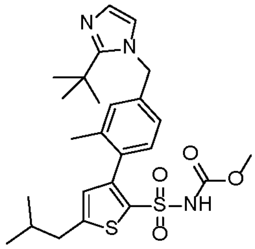 | 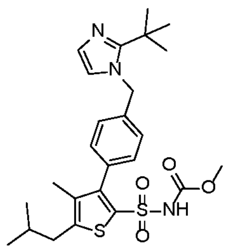 | 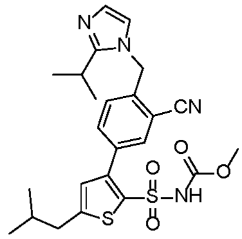 |
| I-25 | I-26 | I-27 |
| 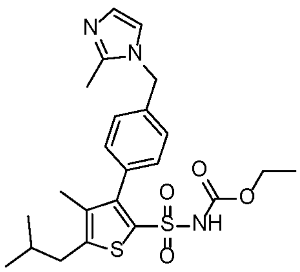 | 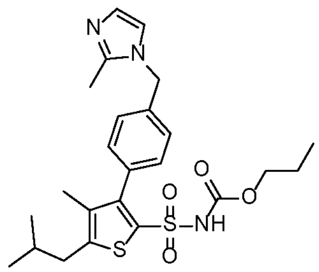 | 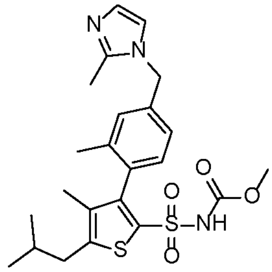 |
| I-28 | I-29 | I-30 |

(continued)

| | | |
|---|---|---|
| 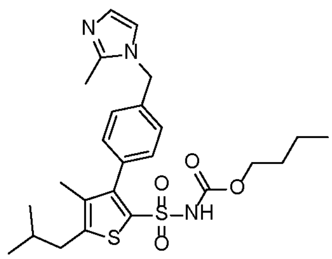 | 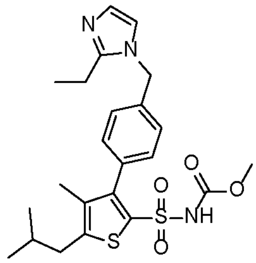 | 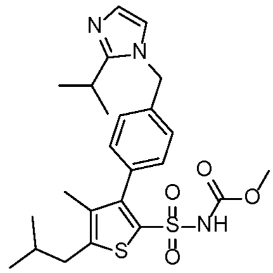 |
| I-31 | I-32 | I-33 |
| 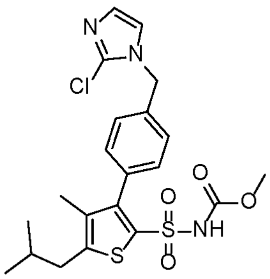 | 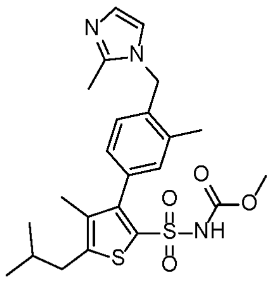 | 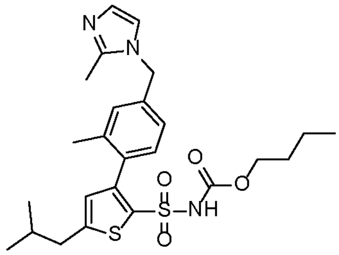 |
| I-34 | I-35 | I-36. |

**19.** A pharmaceutical composition, comprising:

the compound, or the tautomer, stereoisomer, hydrate, solvate, pharmaceutically acceptable salt, or prodrug thereof according to any one of claims 1 to 18; and
a pharmaceutically acceptable carrier.

**20.** Use of the compound, or the tautomer, stereoisomer, hydrate, solvate, pharmaceutically acceptable salt, or prodrug thereof according to any one of claims 1 to 18, or the pharmaceutical composition according to claim 19, the use comprising:

acting as an AT2 receptor agonist; and/or
preventing and/or treating a disease of insufficient endogenous generation of Ang II; and/or
preventing and/or treating a disease in which an increased effect of Ang II is desired or required; and/or
preparing a medicament, pharmaceutical composition, or formulation as an AT2 receptor agonist, and/or a medicament, pharmaceutical composition, or formulation for preventing and/or treating a disease in which the AT2 receptor is expressed and a stimulation of the AT2 receptor is desired or necessary.

**21.** The use according to claim 20, wherein the disease is a disease of gastrointestinal tract, cardiovascular system, respiratory tract, kidney, eyes, female reproductive system, or central nervous system.

**22.** The use according to claim 21, wherein the disease is esophagitis, Barrett's esophagus, gastric ulcer, duodenal ulcer, dyspepsia, gastroesophageal reflux, allergic bowel syndrome, inflammatory enteritis, pancreatitis, liver disease, gallbladder disease, multiple organ failure, sepsis, xerostomia, gastritis, gastric stasis, hyperacidity, biliary tract disease, abdominal disease, segmental ileitis, ulcerative colitis, diarrhea, constipation, acute colic, dysphagia, nausea, vomiting, Sjögren's syndrome, inflammatory disease, asthma, obstructive pulmonary disease, pneumonia, pulmonary hypertension, adult respiratory distress syndrome, idiopathic pulmonary fibrosis, renal failure, nephritis, renal hypertension, diabetic retinopathy, retinopathy of prematurity, retinal microvascularization, ovulatory mechanism disorder, hypertension, myocardial hypertrophy, heart failure, atherosclerosis, arterial thrombosis, venous thrombosis, endothelial dysfunction, endothelial damage, stenosis after balloon dilation, angiogenesis, diabetic complications, microvascular dysfunction, angina pectoris, arrhythmia, intermittent claudication, pre-eclampsia, myocardial infarction, reinfarction, ischemic damage, erectile dysfunction, neointimal hyperplasia, cognitive dysfunction, feeding dysfunction, thirst, stroke, cerebral hemorrhage, cerebral embolism, cerebral infarction, hypertrophy, prostatic hyperplasia, autoimmune diseases, psoriasis, obesity, nerve regeneration, ulcers, inhibition of adipose tissue hypertrophy, stem cell differentiation and proliferation, cancer, apoptosis, tumors, proliferative diabetes, nerve

damage, or organ rejection.

**23.** The use according to claim 22, wherein the disease is asthma, obstructive pulmonary disease, pneumonia, pulmonary hypertension, adult respiratory distress syndrome, or idiopathic pulmonary fibrosis.

**24.** A method for preventing and/or treating a disease of insufficient endogenous generation of Ang II, and/or a disease in which an increased effect of Ang II is desired or required, and/or a disease in which an AT2 receptor is expressed and a stimulation of the AT2 receptor is desired or necessary, wherein the method comprises:
administering a therapeutically effective amount of the compound, and tautomer, stereoisomer, hydrate, solvate, pharmaceutically acceptable salt, or prodrug thereof according to any one of claims 1 to 18, or the pharmaceutical composition according to claim 19 to a person suffering from or susceptible to the disease.

**25.** The method according to claim 24, wherein the disease is a disease of gastrointestinal tract, cardiovascular system, respiratory tract, kidney, eye, female reproductive system, or central nervous system.

**26.** The method according to claim 25, wherein the disease is esophagitis, Barrett's esophagus, gastric ulcer, duodenal ulcer, dyspepsia, gastroesophageal reflux, allergic bowel syndrome, inflammatory enteritis, pancreatitis, liver disease, gallbladder disease, multiple organ failure, sepsis, xerostomia, gastritis, gastric stasis, hyperacidity, biliary tract disease, abdominal disease, segmental ileitis, ulcerative colitis, diarrhea, constipation, acute colic, dysphagia, nausea, vomiting, Sjögren's syndrome, inflammatory disease, asthma, obstructive pulmonary disease, pneumonia, pulmonary hypertension, adult respiratory distress syndrome, idiopathic pulmonary fibrosis, renal failure, nephritis, renal hypertension, diabetic retinopathy, retinopathy of prematurity, retinal microvascularization, ovulatory mechanism disorder, hypertension, myocardial hypertrophy, heart failure, atherosclerosis, arterial thrombosis, venous thrombosis, endothelial dysfunction, endothelial damage, stenosis after balloon dilation, angiogenesis, diabetic complications, microvascular dysfunction, angina pectoris, arrhythmia, intermittent claudication, pre-eclampsia, myocardial infarction, reinfarction, ischemic damage, erectile dysfunction, neointimal hyperplasia, cognitive dysfunction, feeding dysfunction, thirst, stroke, cerebral hemorrhage, cerebral embolism, cerebral infarction, hypertrophy, prostatic hyperplasia, autoimmune diseases, psoriasis, obesity, nerve regeneration, ulcers, inhibition of adipose tissue hypertrophy, stem cell differentiation and proliferation, cancer, apoptosis, tumors, proliferative diabetes, nerve damage, or organ rejection.

**27.** The method according to claim 26, wherein the disease is asthma, obstructive pulmonary disease, pneumonia, pulmonary hypertension, adult respiratory distress syndrome, or idiopathic pulmonary fibrosis.

**INTERNATIONAL SEARCH REPORT**

International application No.
**PCT/CN2023/086425**

**A. CLASSIFICATION OF SUBJECT MATTER**

C07D409/10(2006.01)i； C07D409/14(2006.01)i； C07D401/14(2006.01)i； A61K31/4439(2006.01)i； A61K31/506(2006.01)i； A61P9/00(2006.01)i； A61P11/00(2006.01)i； A61P13/12(2006.01)i

According to International Patent Classification (IPC) or to both national classification and IPC

**B. FIELDS SEARCHED**

Minimum documentation searched (classification system followed by classification symbols)

IPC:C07D A61P A61K

Documentation searched other than minimum documentation to the extent that such documents are included in the fields searched

Electronic data base consulted during the international search (name of data base and, where practicable, search terms used)

CNABS; DWPI; VEN; ENTXT; ENTXTC; CJFD; CNKI; CNTXT; USTXT; WOTXT; EPTXT; STN-REG; STN-CAPLUS; ISI-Web of Science; 万方, Wanfang; 超星读秀, Chaoxing Duxiu: 武汉人福创新药物研发中心, 张学军, 臧杨, 丁肖华, 高振兴, 钱丽娜, 李群, 杨辉, 李莉娥, 杨俊, 维科尔, 噻吩, 磺酰胺, AT2, AngII, thiophene, sulfonamide, 结构式I~V, structural formulas I~V

**C. DOCUMENTS CONSIDERED TO BE RELEVANT**

| Category* | Citation of document, with indication, where appropriate, of the relevant passages | Relevant to claim No. |
|---|---|---|
| PX | GB 2605148 A (Vicore Pharma AB) 28 September 2022 (2022-09-28)<br>claims 1-21 | 1, 8, 10-14, 20-27 |
| X | WO 2021053344 A1 (Vicore Pharma AB) 25 March 2021 (2021-03-25)<br>claims 1-20, description page 1 lines 5-10, page 43 lines 1-9, 28-36, page 44 lines 1-7 | 1-27 |
| X | MAHALINGAM, A.K. et al. "Selective Angiotensin II AT2 Receptor Agonists with Reduced CYP 450 Inhibition"<br>*Bioorganic & Medicinal Chemistry,*<br>Vol. 18, No. (12), 09 April 2010 (2010-04-09), 4570-4590<br>ISSN: 0968-0896,<br>page 4570 left-hand column paragraph 1, page 4575 table 3 | 1-27 |
| X | WO 2004046141 A1 (Vicore Pharma AB) 03 June 2004 (2004-06-03)<br>claims 1-39, description page 45 lines 10, 11, page 48 lines 5-7, pages 51, 56, 59 lines 1-3 | 1-27 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **19 June 2023** | **22 July 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/086425**

**C. DOCUMENTS CONSIDERED TO BE RELEVANT**

| Category* | Citation of document, with indication, where appropriate, of the relevant passages | Relevant to claim No. |
|---|---|---|
| X | WANNBERG, J. et al. "N-(Methyloxycarbonyl)Thiophene Sulfonamides as High Affinity AT2 Receptor Ligands" *Bioorganic & Medicinal Chemistry,* Vol. vol. 29, 07 November 2020 (2020-11-07), p. 115859 ISSN: 0968-0896, page 3 flowchart 2, abstract | 1-27 |
| X | CN 101193886 A (VICORE PHARMA AB) 04 June 2008 (2008-06-04) description pages 51, 53 lines 1, 2, claims 1-37 | 1-27 |
| X | MURUGAIAH, A.M.S. et al. "From the First Selective Non-Peptide AT2 Receptor Agonist to Structurally Related Antagonists" *J. Med. Chem.,* Vol. 55, No. (5), 16 January 2012 (2012-01-16), pp. 2265-2278 ISSN: 0022-2623, page 2268 flowchart 4, abstract | 1-27 |

Form PCT/ISA/210 (second sheet) (July 2022)

**INTERNATIONAL SEARCH REPORT**

International application No. **PCT/CN2023/086425**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **20-27**
because they relate to subject matter not required to be searched by this Authority, namely:

Claims 20-23 relate to the use as an AT2 receptor agonist, for prevention and/or treatment of diseases caused by insufficient endogenous production of AngII, and for prevention and/or treatment of diseases in which AngII action is expected or required to be increased, and claims 24-27 relate to a method for prevention and/or treatment of diseases caused by insufficient endogenous production of AngII, and/or diseases in which AngII action is expected or necessary to be increased, and/or diseases in which an AT2 receptor is expressed and stimulation production is desired or necessary, which are methods for treating diseases that are practiced on a living human or animal body for the direct purpose of disease treatment, fall within methods for treatment of the human or animal body by therapy, and fall within the cases set out in PCT Rule 39.1(iv) for which no international search is required.

The search for claims 20-27 is performed on the basis of a reasonable expectation that said claims are amended as follows:

The subject matter of claims 20-23 is amended to "the use of the compound of any one of claims 1-18, a tautomer, stereoisomer, hydrate, solvate, pharmaceutically acceptable salt or prodrug thereof or the pharmaceutical composition of claim 19, the use comprising preparation of a drug for preventing and/or treating diseases caused by insufficient endogenous production of AngII, and/or preparation of a drug for preventing and/or treating diseases in which AngII action is expected or required to be increased, and/or preparation of a drug, pharmaceutical composition or preparation as an AT2 receptor agonist and for preventing and/or treating diseases in which an AT2 receptor is expressed and is expected or necessary to be stimulated". The subject matter of claims 24-27 is amended to "the use of the compound of any one of claims 1-18, a tautomer, stereoisomer, hydrate, solvate, pharmaceutically acceptable salt, or prodrug thereof or the pharmaceutical composition of claim 19 in the preparation of a drug, the drug being used for prevention and/or treatment of diseases caused by insufficient endogenous production of AngII, and/or diseases in which AngII action is expected or necessary to be increased, and/or diseases in which an AT2 receptor is expressed and stimulation production is desired or necessary".

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/086425**

| Patent document cited in search report | Publication date (day/month/year) | Patent family member(s) | Publication date (day/month/year) |
|---|---|---|---|
| GB 2605148 A | 28 September 2022 | None | |
| WO 2021053344 A1 | 25 March 2021 | CA 3152218 A1 | 25 March 2021 |
| | | AU 2020351301 A1 | 17 March 2022 |
| | | IL 291172 A | 01 May 2022 |
| | | BR 112022003130 A2 | 17 May 2022 |
| | | IN 202237016128 A | 27 May 2022 |
| | | KR 20220101071 A | 19 July 2022 |
| | | CN 114786774 A | 22 July 2022 |
| | | EP 4031240 A2 | 27 July 2022 |
| | | HK 40070216 A0 | 21 October 2022 |
| | | JP 2022549086 W | 24 November 2022 |
| | | US 2022388994 A1 | 08 December 2022 |
| WO 2004046141 A1 | 03 June 2004 | AU 2003302106 A1 | 15 June 2004 |
| CN 101193886 A | 04 June 2008 | WO 2006109048 A1 | 19 October 2006 |
| | | AU 2006235698 A1 | 25 October 2007 |
| | | IN 200707722 P1 | 09 November 2007 |
| | | EP 1869023 A1 | 26 December 2007 |
| | | MX 2007012634 A | 01 January 2008 |
| | | KR 20080009275 A | 28 January 2008 |
| | | JP 2008535898 A | 04 September 2008 |
| | | US 2009042931 A1 | 12 February 2009 |
| | | MX 291483 B | 28 October 2011 |
| | | US 8080571 B2 | 20 December 2011 |
| | | EP 1869023 B1 | 11 January 2012 |
| | | AU 2006235698 B2 | 29 March 2012 |
| | | ES 2395194 T3 | 11 February 2013 |
| | | JP 2013040208 A | 28 February 2013 |
| | | CA 2604797 C | 28 May 2013 |
| | | JP 5202301 B2 | 05 June 2013 |
| | | KR 101429484 B1 | 13 August 2014 |
| | | CN 101193886 B | 27 August 2014 |
| | | JP 5718301 B2 | 13 May 2015 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202210359646
- CN 202210715417
- CN 202211281172
- CN 202310325262
- WO 9943339 A
- WO 2021229244 A1


### Non-patent literature cited in the description


- **CAREY** ; **SUNDBERG**. ADVANCED ORGANIC CHEMISTRY 4THED. Plenum Press, 2000, vol. A,B
- **MAEHR**. *J. Chem. Ed.*, 1985, vol. 62, 114-120